# EUROPEAN PATENT SPECIFICATION

(11) **EP 2 545 150 B1**
(45) Date of publication and mention of the grant of the patent: **25.07.2018**
(21) Application number: 11709190.0
(22) Date of filing: 23.02.2011
(51) Int. Cl.: C11B 3/00, A23D 9/04, C11B 3/04, C11B 3/06, C11B 3/14, C12N 9/18

(54) **PROCESS**
VERFAHREN
PROCÉDÉ

(30) Priority: 12.03.2010 US 313194 P; 12.03.2010 EP 10156412
(43) Date of publication of application: 16.01.2013
(73) Proprietor: DuPont Nutrition Biosciences ApS, 1411 Copenhagen K (DK)
(72) Inventor: MIKKELSEN, Rene, 8660 Skandeborg (DK); JØRGENSEN, Tina, 8600 Silkeborg (DK); SØE, Jørn Borch, 8381 Tilst (DK)
(74) Representative: D Young & Co LLP
(86) International application number: PCT/IB2011/050742
(87) International publication number: WO 2011/110967

(56) References cited:
- EP-A1- 0 671 461
- WO-A2-2006/009676
- WO-A2-2010/143149
- GB-A- 701 633
- GB-A- 1 321 299
- US-A1- 2003 050 492
- LEVADOUX W L ET AL: "Pigment removal from canola oil using chlorophyllase.", JOURNAL OF THE AMERICAN OIL CHEMISTS' SOCIETY, vol. 64, no. 1, 1 January 1987 (1987-01-01), pages 139-144, XP002616845, DOI: 10.1007/BF02546269
- BITAR MARIANNE ET AL: "Chlorophyllase biocatalysis in an aqueous/miscible organic solvent medium containing canola oil", JOURNAL OF THE AMERICAN OIL CHEMISTS' SOCIETY, vol. 81, no. 10, 1 October 2004 (2004-10-01), pages 927-932, XP002543901, SPRINGER, BERLIN, DE ISSN: 0003-021X, DOI: 10.1007/S11746-004-1003-7

## Description

### FIELD

The present invention relates to the industrial processing of plant-derived food and feed products, especially vegetable oils. The invention may be employed to reduce or eliminate contamination by chlorophyll and chlorophyll derivatives.

### BACKGROUND

Chlorophyll is a green-coloured pigment widely found throughout the plant kingdom. Chlorophyll is essential for photosynthesis and is one of the most abundant organic metal compounds found on earth. Thus many products derived from plants, including foods and feeds, contain significant amounts of chlorophyll.

For example, vegetable oils derived from oilseeds such as soybean, palm or rape seed (canola), cotton seed and peanut oil typically contain some chlorophyll. However the presence of high levels of chlorophyll pigments in vegetable oils is generally undesirable. This is because chlorophyll imparts an undesirable green colour and can induce oxidation of oil during storage, leading to a deterioration of the oil.

Various methods have been employed in order to remove chlorophyll from vegetable oils. Chlorophyll may be removed during many stages of the oil production process, including the seed crushing, oil extraction, degumming, caustic treatment and bleaching steps. However the bleaching step is usually the most significant for reducing chlorophyll residues to an acceptable level. During bleaching the oil is heated and passed through an adsorbent to remove chlorophyll and other colour-bearing compounds that impact the appearance and/or stability of the finished oil. The adsorbent used in the bleaching step is typically clay.

In the edible oil processing industry, the use of such steps typically reduces chlorophyll levels in processed oil to between 0.02 to 0.05 ppm. However the bleaching step increases processing cost and reduces oil yield due to entrainment in the bleaching clay. The use of clay may remove many desirable compounds such as carotenoids and tocopherol from the oil. Also the use of clay is expensive, this is particularly due to the treatment of the used clay (i.e. the waste) which can be difficult, dangerous (prone to self-ignition) and thus costly to handle.

Thus attempts have been made to remove chlorophyll from oil by other means, for instance using the enzyme chlorophyllase.

In plants, chlorophyllase (chlase) is thought to be involved in chlorophyll degradation and catalyzes the hydrolysis of an ester bond in chlorophyll to yield chlorophyllide and phytol. WO 2006009676 describes an industrial process in which chlorophyll contamination can be reduced in a composition such as a plant oil by treatment with chlorophyllase. The watersoluble chlorophyllide which is produced in this process is also green in colour but can be removed by an aqueous extraction or silica treatment.

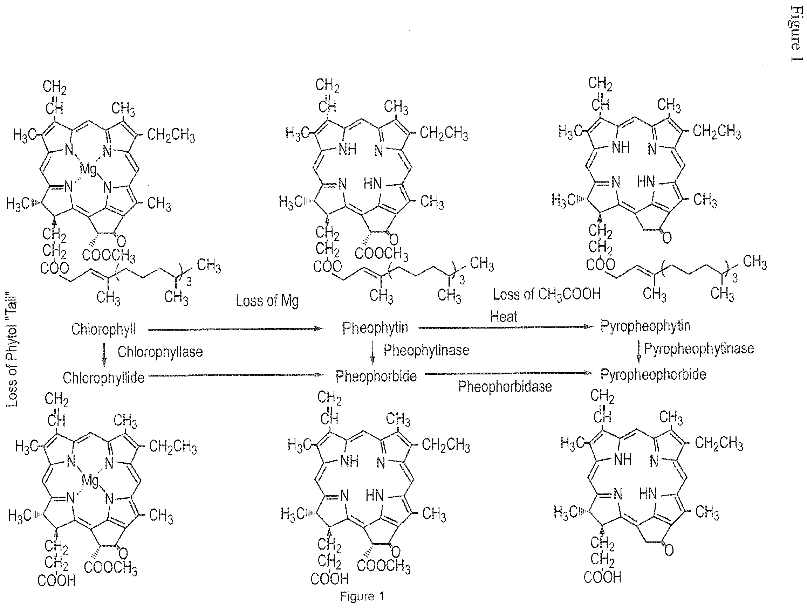

Chlorophyll is often partly degraded in the seeds used for oil production as well as during extraction of the oil from the seeds. One common modification is the loss of the magnesium ion from the porphyrin (chlorin) ring to form the derivative known as pheophytin (see Figure 1). The loss of the highly polar magnesium ion from the porphyrin ring results in significantly different physico-chemical properties of pheophytin compared to chlorophyll. Typically pheophytin is more abundant in the oil during processing than chlorophyll. Pheophytin has a greenish colour and may be removed from the oil by an analogous process to that used for chlorophyll, for instance as described in WO 2006009676 by an esterase reaction catalyzed by an enzyme having a pheophytinase activity. Under certain conditions, some chlorophyllases are capable of hydrolyzing pheophytin as well as chlorophyll, and so are suitable for removing both of these contaminants. The products of pheophytin hydrolysis are the red/brown-colored pheophorbide and phytol. Pheophorbide can also be produced by the loss of a magnesium ion from chlorophyllide, i.e. following hydrolysis of chlorophyll (see Figure 1). WO 2006009676 teaches removal of pheophorbide by an analogous method to chlorophyllide, e.g. by aqueous extraction or silica adsorption.

US 2003/0050492 **[D2]** described methods for refining vegetable oils and byproducts thereof. More particularly, US 2003/0050492 relates to processes for producing vegetable oils having reduced content of impurities such as free fatty acids and phosphatides.

EP 0671461 **[D3]** relates to a process for producing a high carotene content oil comprises blowing steam through an oil containing carotene in an amount of at least 500 ppm under a reduced pressure of not higher than 10 torr whilst maintaining the temperature at 110 to 150°C.

WO 2010/143149 **[D4]** relates to a method, use, apparatus and related products for treating a composition comprising pyropheophytin.

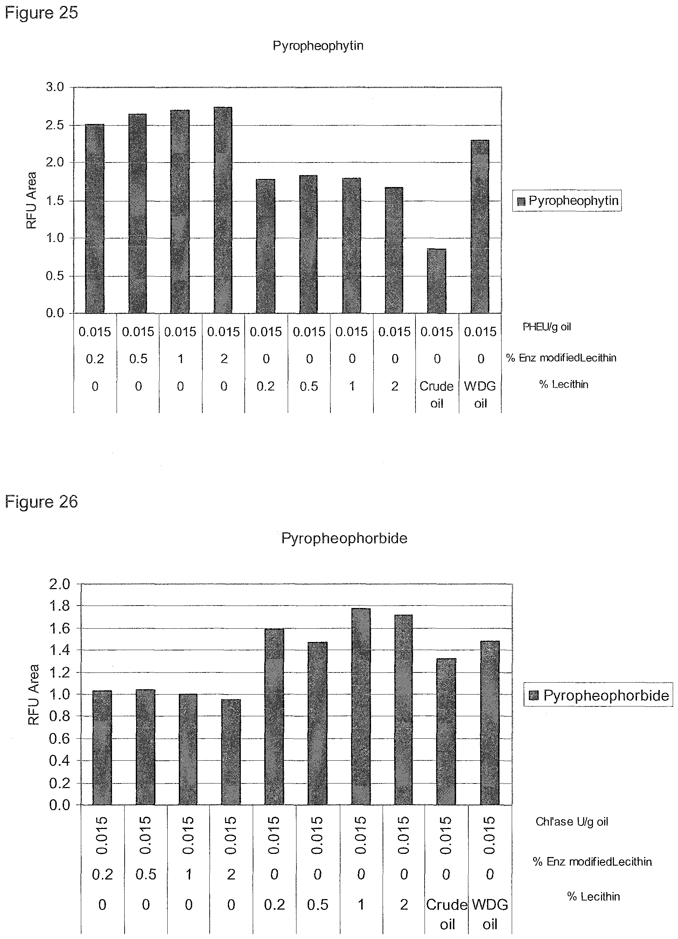

Pheophytin may be further degraded to pyropheophytin, both by the activity of plant enzymes during harvest and storage of oil seeds or by processing conditions (e.g. heat) during oil refining (see "Behaviour of Chlorophyll Derivatives in Canola Oil Processing", JAOCS, Vol, no. 9 (Sept. 1993) pages 837-841). One possible mechanism is the enzymatic hydrolysis of the methyl ester bond of the isocyclic ring of pheophytin followed by the non-enzymatic conversion of the unstable intermediate to pyropheophytin. A 28-29 kDa enzyme from *Chenopodium album* named pheophorbidase is reportedly capable of catalyzing an analogous reaction on pheophorbide, to produce the phytol-free derivative of pyropheophytin known as pyropheophorbide (see Figure 26). Pyropheophorbide is less polar than pheophorbide resulting in the pyropheophoribe having a decreased water solubility and an increased oil solubility compared with pheophorbide.
Depending on the processing conditions, pyropheophytin can be more abundant than both pheophytin and chlorophyll in vegetable oils during processing (see Table 9 in volume 2.2. of Bailey's Industrial Oil and Fat Products (2005), 6th edition, Ed. by Fereidoon Shahidi, John Wiley & Sons). This is partly because of the loss of magnesium from chlorophyll during harvest and storage of the plant material. If an extended heat treatment at 90°C or above is used, the amount of pyropheophytin in the oil is likely to increase and could be higher than the amount of pheophytin. Chlorophyll levels are also reduced by heating of oil seeds before pressing and extraction as well as the oil degumming and alkali treatment during the refining process. It has also been observed that phospholipids in the oil can complex with magnesium and thus reduce the amount of chlorophyll. Thus chlorophyll is a relatively minor contaminant compared to pyropheophytin (and pheophytin) in many plant oils.
There is a still a need for an improved process for removing chlorophyll and chlorophyll derivatives such as pheophytin and pyropheophytin from plant oils. In particular, there is a need for a process in which chlorophyll and chlorophyll derivatives are removed with enhanced efficiency, whilst reducing the loss of other desirable compounds from the oil.

### SUMMARY

In one aspect the present invention provides a process for refining a plant oil, comprising a step of contacting the oil with an enzyme which is capable of hydrolysing chlorophyll or a chlorophyll derivative, wherein the enzyme is contacted with the oil in the presence of at least 1% by weight phospholipid. The enzyme is contacted with the oil in the presence of less than 0.2% by weight lysophosholipid, for example less than 0.15%, less than 0.1% or less than 0.05% by weight, based on the total weight of oil. The enzyme is contacted with the oil before or during a step of degumming of the oil. The degumming step may comprise, for example, water degumming, acid degumming, enzymatic degumming, and/or total degumming/neutralisation (e.g. addition of an acid to the oil followed by neutralisation with an alkali).

Where the enzyme is contacted with the oil before degumming, in particular embodiments an enzymatic degumming step may comprise contacting the oil with a phospholipase (e.g. phospholipase A1, phospholipase A2 or phospholipase C) or an acyltransferase. The acyltransferase may comprise, for example, the amino acid sequence of SEQ ID NO:23 or a sequence having at least 80% sequence identity thereto.
In another embodiment, the process comprises contacting the oil with the enzyme and a phospholipase which does not produce lysopholipids (e.g. phospholipase C) in a single step. For example, the enzyme may be contacted with the oil during an enzymatic degumming step using phospholipase C, i.e. the enzyme and phospholipase C are used simultaneously. The enzyme is contacted with the oil in the presence of at least 1%, at least 1.5% or at least 2% by weight phospholipid.
In further embodiments, the enzyme is contacted with the oil at a temperature of less than 80°C, preferably less than 70°C, preferably 55°C to 65°C, preferably 58° to 62°C, e.g. about 60°C. Preferably the enzyme is contacted with the oil in the presence of 1 to 5% by weight water, e.g. about 1% or about 2% by weight water. In one embodiment, the enzyme is contacted with the oil at a pH of 6.0 to 6.8, e.g. 6.3 to 6.5.
Preferably the process does not comprise a step of clay treatment. The process preferably further comprises performing a deodorisation step to produce a deodorized oil and a distillate (e.g. an aqueous distillate or a nitrogenous distillate). Typically the process produces a level of carotenoids and/or tocopherol in the refined (deodorized or non-deodorized) oil and/or distillate which is elevated compared to a process comprising a clay treatment step.
In one embodiment the enzyme comprises a chlorophyllase, pheophytinase, pyropheophytinase or pheophytin pheophorbide hydrolase. For example, the enzyme may comprise a polypeptide sequence as defined in any one of SEQ ID NOs: 1, 2, 4, 6 or 8 to 15, or a functional fragment or variant thereof. Preferably the enzyme comprises a polypeptide sequence having at least 75% sequence identity to any one of SEQ ID NOs: 1, 2, 4, 6 or 8 to 15, e.g. over at least 50 amino acid residues. In particularly preferred embodiments, the enzyme comprises the sequence of SEQ ID NO:2 or SEQ ID NO:4 or a sequence having at least 90% sequence identity thereto. Also disclosed is a refined plant oil obtainable by a process as defined above. Furhter disclosed is a distillate (e.g. an aqueous or nitrogenous distillate) obtainable by the process as defined above, i.e. a process as described herein comprising a deodorization step.
In a further aspect, the invention provides a process as defined above, to increase a level of carotenoids and/or tocopherol in a refined oil and/or a distillate obtained by deodorization of the oil.
As described herein, the activity of chlorophyllases and related enzymes in oil has been found to be dependent on the phospholipid content of the oil. Therefore the present invention provides an improved oil refining process in which chlorophyllase or a related enzyme is used in the presence of a minimum level of phospholipid. Moreover, based on the demonstration that elevated lysophospholipid levels are associated with reduced activity of chlorophyllases, the invention provides an improved process in which the enzyme is used in the presence of a low level of lysophospholipid. By enhancing the activity of the enzyme, the process of the present invention advantageously facilitates the removal of chlorophyll and chlorophyll derivatives typically without the need for a clay treatment step. This may increase the level of useful compounds such as tocopherol and carotenoids in the oil, which can be recovered in a deodorization step or retained in the finished product.

### BRIEF DESCRIPTION OF DRAWINGS

Figure 1 shows the reactions involving chlorophyll and derivatives and enzymes used in the present invention.
Figure 2 shows the amino acid sequence of *Arabidopsis thaliana* chlorophyllase (SEQ ID NO:1).
Figure 3 shows the amino acid sequence of *Triticum aestivum* chlorophyllase (SEQ ID NO:2).
Figure 4 shows a nucleotide sequence encoding *Triticum aestivum* chlorophyllase (SEQ ID NO:3).
Figure 5 shows the amino acid sequence of *Chlamydomonas reinhardtii* chlorophyllase (SEQ ID NO:4).
Figure 6 shows a nucleotide sequence encoding *Chlamydomonas reinhardtii* chlorophyllase (SEQ ID NO:5).
Figure 7 shows the amino acid sequence of a pheophytin pheophorbide hydrolase (PPH) from *Arabidopsis thaliana* (SEQ ID NO:6). A chloroplast transit peptide is shown in bold.
Figure 8 shows the nucleotide sequence of a cDNA from *Arabidopsis thaliana* encoding pheophytin pheophorbide hydrolase (SEQ ID NO:7). The PPH of SEQ ID NO:6 is encoded by residues 173 to 1627 of SEQ ID NO:7.
Figure 9 shows the polypeptide sequence of *Populus trichocarpa* PPH (SEQ ID NO:8).
Figure 10 shows the polypeptide sequence of *Vitis vinifera* PPH (SEQ ID NO:9).
Figure 11 shows the polypeptide sequence of *Ricinus communis* PPH (SEQ ID NO:10).
Figure 12 shows the polypeptide sequence of *Oryza sativa* (japonica cultivar-group) PPH (SEQ ID NO:11).
Figure 13 shows the polypeptide sequence of *Zea mays* PPH (SEQ ID NO:12).
Figure 14 shows the polypeptide sequence of *Nicotiana tabacum* PPH (SEQ ID NO:13).
Figure 15 shows the polypeptide sequence of *Oryza sativa* Japonica Group PPH (SEQ ID NO:14).
Figure 16 shows (a) the polypeptide sequence of *Physcomitrella patens* subsp. patens PPH (SEQ ID NO:15)
Figure 17 shows schematically the fusion of the wheat (*Triticum aestivum*) chlorophyllase gene to the aprE signal sequence.
Figure 18 shows schematically the plasmid pBN-TRI_CHL containing the wheat (*Triticum aestivum*) chlorophyllase gene.
Figure 19 shows schematically the fusion of the *Chlamydomonas reinhardtii* chlorophyllase gene to the aprE signal sequence.
Figure 20 shows schematically the plasmid pBN-CHL CHL containing the *Chlamydomonas reinhardtii* chlorophyllase gene.
Figure 21 shows samples of refined rapeseed oil treated with chlorophyllase in the presence of different surfactants, including soya lecithin, sorbitan monooleate and sorbitan trioleate, as described in Example 3.
Figure 22 shows samples of refined oil or a mixture of refined oil and crude soya oil, with or without treatment with chlorophyllase and chlorophyll addition, as described in Example 4.

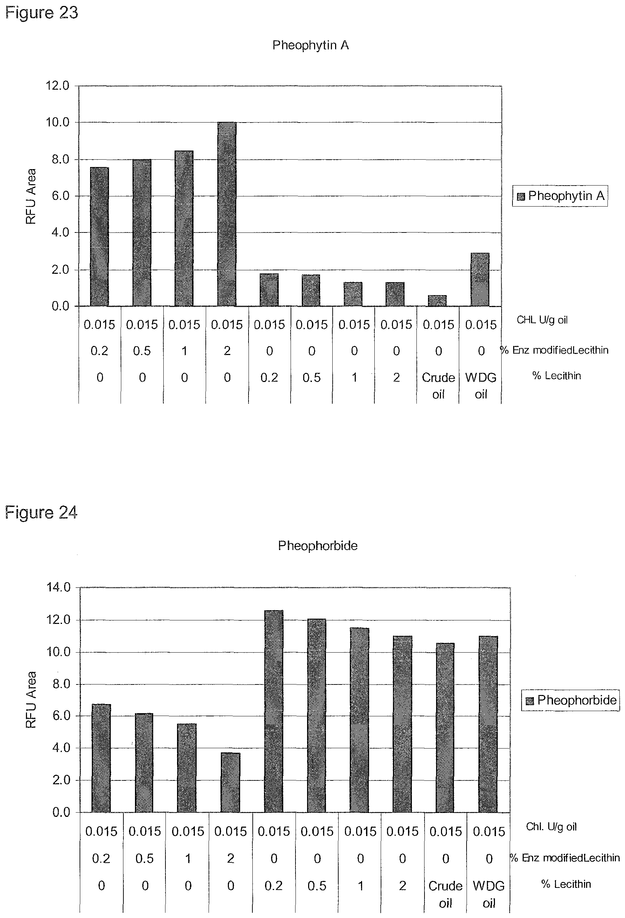

Figure 23 shows relative fluorescence values from HPLC analysis indicative of pheophytin levels in rapeseed oil samples following chlorophyllase treatment in the presence of varying levels of lecithin and modified lecithin (modified by an acyltransferase), as described in Example 5.
Figure 24 shows relative fluorescence values from HPLC analysis indicative of pheophorbide levels in rapeseed oil samples following chlorophyllase treatment in the presence of varying levels of lecithin and modified lecithin (modified by an acyltransferase), as described in Example 5.
Figure 25 shows relative fluorescence values from HPLC analysis indicative of pyropheophytin levels in rapeseed oil samples following chlorophyllase treatment in the presence of varying levels of lecithin and modified lecithin (modified by an acyltransferase), as described in Example 5.
Figure 26 shows relative fluorescence values from HPLC analysis indicative of pyropheophorbide levels in rapeseed oil samples following chlorophyllase treatment in the presence of varying levels of lecithin and modified lecithin (modified by an acyltransferase), as described in Example 5.
Figure 27 shows relative fluorescence values from HPLC analysis indicative of pheophytin levels in rapeseed oil samples following treatment with *Triticum aestivum* or *Chlamydomonas reinhardtii* chlorophyllase in the presence of an acyltransferase, phospholipase C or phospholipase A1, as described in Example 6.

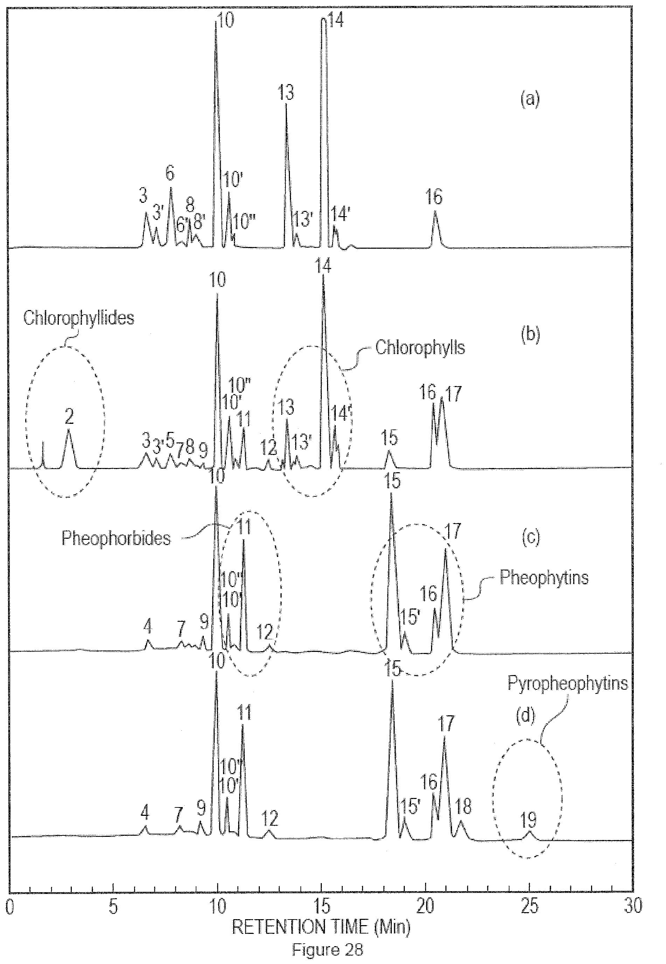

Figure 28 shows an HPLC chromatogram using absorbance detection (430 nm) indicating numbered peaks associated with: 1 = chlorophyllide b; 2 = chlorophyllide a; 3 = neoxanthin; 3' = neoxanthin isomer; 4 = neochrome; 5 = violaxanthin; 6 = luteoxanthin; 7 = auroxanthin; 8 = anteraxanthin; 8' = anteraxanthin isomer; 9 = mutatoxanthin; 10 = lutein; 10' = lutein isomer; 10" = lutein isomer; 11 = pheophorbide b; 12 = pheophorbide a; 13 = chlorophyll b; 13' = chlorophyll b'; 14 = chlorophyll a; 14' = chlorophyll a'; 15 = pheopytin b; 15' = pheophytin b'; 16 = β-carotene; 17 = pheophytin a; 17' = pheophytin a'; 18 = pyropheophytin b; 19 = pyropheophytin a.
Figure 29 shows pheophytin a and pyropheophytin levels in oil at various stages of a standard refining process using bleaching with clay and an enzymatic refining processing using chlorophyllase without clay treatment, as described in Example 8.
Figure 30 shows pheophorbide a and pyropheophorbide levels in oil at various stages of a standard refining process using bleaching with clay and an enzymatic refining processing using chlorophyllase without clay treatment, as described in Example 8.
Figure 31 is a diagrammatic representation of an oil refining process according to the present invention.
Figure 32 shows the amino acid sequence of a mutant *Aeromonas salmonicida* mature lipid acyltransferase (GCAT) with a mutation of Asn80Asp after undergoing post-translational modification (SEQ ID No. 23).
Figure 33 shows the effect of chlorophyllase on degradation of pheophytin a in oil.
Figure 34 shows the effect of pH and % water on chlorophyllase activity
Figure 35 shows epimer forms of pheophytin and their rearrangement.
Figure 36 shows the relative ratio of pheophytin a epimers at different pH after 4 hr reaction time.
Figure 37 shows the effect of temperature on pheophytin hydrolysis by chlorophyllase treatment of oil.
Figure 38 shows the effect of temperature on pyropheophytin levels.
Figure 39 shows the effect of temperature on total levels of chlorophyll and pheophytin and pyropheophytin.
Figure 40 shows the effect of different mixing conditions on pheophytin hydrolysis.
Figure 41 shows pheophytin as a function of time and pH in oil treated with chlorophyllase.
Figure 42 shows pheophytin a isomer ratio as a function of time and pH in oil treated with chlorophyllase.
Figure 43 shows pyropheophytin as a function of time and pH in oil treated with chlorophyllase.
Figure 44 shows enzymatic pyropheophytin hydrolysis as a function of time and pH in oil treated with chlorophyllase.
Figure 45 shows the effect of pH on chlorophyllase degradation of pheophytin.
Figure 46 shows the effect of pH on the amount of pheophytin *a* and *a'* epimers.
Figure 47 shows the effect of pH on yropheophytin levels.
Figure 48 shows the effect of pH on total levels of pheophytin plus pyropheophytin.
Figure 49 shows the effect of pH on pheophorbide levels.
Figure 50 shows the effect of pH on pheophytin in rapeseed oil after 2 hr by chlorohyllase treatment in water degumming process(WDG) and total degumming process(TDG).
Figure 51 shows the effect of water content and pH on pheophytin in chlorophyllase treated oil.
Figure 52 shows the effect of water content and pH on pyropheophytin in chlorophyllase treated oil.
Figure 53 shows the effect of water content and pH on pheophytin epimers in chlorophyllase treated oil.
Figure 54 shows the effect of temperature, pH adjustment and reaction time on pheophytin levels by different dosages of chlorophyllase treatment of oil.
Figure 55 shows the effect of temperature, pH adjustment and reaction time on pyropheophytin levels by different dosages of chlorophyllase treatment of oil.
Figure 56 shows the effect of temperature, pH adjustment and reaction time on levels of pheophytin *a* epimers by different dosages of chlorophyllase treatment of oil.

### DETAILED DESCRIPTION

In one aspect the present invention relates to a process for refining a plant oil. Typically the process is used to remove chlorophyll and/or chlorophyll derivatives from the oil, or to reduce the level of chlorophyll and/or chlorophyll derivatives in the oil, for instance where the chlorophyll and/or chlorophyll derivatives are present as a contaminant.

### Chlorophyll and chlorophyll derivatives

By "chlorophyll derivative" it is typically meant compounds which comprise both a porphyrin (chlorin) ring and a phytol group (tail), including magnesium-free phytol-containing derivatives such as pheophytin and pyropheophytin. Chlorophyll and (phytol-containing) chlorophyll derivatives are typically greenish is colour, as a result of the porphyrin (chlorin) ring present in the molecule. Loss of magnesium from the porphyrin ring means that pheophytin and pyropheophytin are more brownish in colour than chlorophyll. Thus the presence of chlorophyll and chlorophyll derivatives in an oil, can give such an oil an undesirable green, greenish or brownish colour. In one embodiment, the present process may be performed in order to remove or reduce the green or brown colouring present in the oil. Accordingly the present process may be referred to as a bleaching or de-colorizing process.

Enzymes used in the process may hydrolyse chlorophyll and phytol-containing chlorophyll derivatives to cleave the phytol tail from the chlorin ring. Hydrolysis of chlorophyll and chlorophyll derivatives typically results in compounds such as chlorophyllide, pheophorbide and pyropheophorbide which are phytol-free derivatives of chlorophyll. These compounds still contain the colour-bearing porphyrin ring, with chlorophyllide being green and pheophorbide and pyropheophorbide a reddish brown colour. In some embodiments, it may also be desirable to remove these phytol-free derivatives and to reduce the green/red/brown colouring in the oil. Thus in one embodiment of the invention, the process may further comprise a step of removing or reducing the level of phytol-free chlorophyll derivatives in the oil. The process may involve bleaching or de-colorizing to remove the green and/or red/brown colouring of the oil.

The chlorophyll or chlorophyll derivative may be either a or b forms. Thus as used herein, the term "chlorophyll" includes chlorophyll a and chlorophyll b. In a similar way both a and b forms are covered when referring to pheophytin, pyropheophytin, chlorophyllide, pheophorbide and pyropheophorbide.

### Plant oils

Any plant oil may be treated according to the present process, in order to remove undesirable contamination by chlorophyll and/or chlorophyll derivatives. The oil may be derived from any type of plant, and from any part of a plant, including whole plants, leaves, stems, flowers, roots, plant protoplasts, seeds and plant cells and progeny of same. The class of plants from which products can be treated in the method of the invention includes higher plants, including angiosperms (monocotyledonous and dicotyledonous plants), as well as gymnosperms. It includes plants of a variety of ploidy levels, including polyploid, diploid, haploid and hemizygous states.

In preferred embodiments, the oil may comprise a vegetable oil, including oils processed from oil seeds or oil fruits (e.g. seed oils such as canola (rapeseed) oil and fruit oils such as palm). Examples of suitable oils include rice bran, soy, canola (rape seed), palm, olive, cottonseed, corn, palm kernel, coconut, peanut, sesame or sunflower. The process of the invention can be used in conjunction with methods for processing essential oils, e.g., those from fruit seed oils, e.g. grapeseed, apricot, borage, etc. The process of the invention can be used in conjunction with methods for processing high phosphorus oils (e.g. a soy bean oil). Preferably the oil is a crude plant oil.

### Chlorophyll and chlorophyll derivatives in oil

The chlorophyll and/or chlorophyll derivatives (e.g. chlorophyll, pheophytin and/or pyropheophytin) may be present in the oil naturally, as a contaminant, or as an undesired component in a processed product. The chlorophyll and/or chlorophyll derivatives (e.g. chlorophyll, pheophytin and/or pyropheophytin) may be present at any level in the oil. Typically chlorophyll, pheophytin and/or pyropheophytin may be present as a natural contaminant in the oil at a concentration of 0.001 to 1000 mg/kg (0.001 to 1000 ppm, 10⁻⁷ to 10⁻¹ wt %), based on the total weight of the oil. In further embodiments, the chlorophyll and/or chlorophyll derivatives may be present in the oil at a concentration of 0.1 to 100, 0.5 to 50, 1 to 50, 1 to 30 or 1 to 10 mg/kg, based on the total weight of the oil.

Phytol-free chlorophyll derivatives may also be present in the oil. For instance, chlorophyllide, pyropheophorbide and/or pyropheophorbide may be present at any level in the oil. Typically chlorophyllide, pyropheophorbide and/or pyropheophorbide may be present in the oil, either before or after treatment with an enzyme according to the method of the present invention, at a concentration of 0.001 to 1000 mg/kg (0.001 to 1000 ppm, 10⁻⁷ to 10⁻¹ wt %), based on the total weight of the oil. In further embodiments, the chlorophyllide, pyropheophorbide and/or pyropheophorbide may be present in the composition at a concentration of 0.1 to 100, 0.5 to 50, 1 to 50, 1 to 30 or 1 to 10 mg/kg, based on the total weight of the composition.

### Enzymes hydrolysing chlorophyll or a chlorophyll derivative

The process of the present invention comprises a step of contacting the oil with an enzyme which is capable of hydrolysing chlorophyll or a chlorophyll derivative. Typically "hydrolyzing chlorophyll or a chlorophyll derivative" means hydrolysing an ester bond in chlorophyll or a (phytol-containing) chlorophyll derivative, e.g. to cleave a phytol group from the chlorin ring in the chlorophyll or chlorophyll derivative. Thus the enzyme typically has an esterase or hydrolase activity. Preferably the enzyme has esterase or hydrolase activity in an oil phase, and optionally also in an aqueous phase.

Thus the enzyme may, for example, be a chlorophyllase, pheophytinase or pyropheophytinase. Preferably, the enzyme is capable of hydrolysing at least one, at least two or all three of chlorophyll, pheophytin and pyropheophytin. In a particularly preferred embodiment, the enzyme has chlorophyllase, pheophytinase and pyropheophytinase activity. In further embodiments, two or more enzymes may be used in the method, each enzyme having a different substrate specificity. For instance, the method may comprise the combined use of two or three enzymes selected from a chlorophyllase, a pheophytinase and a pyropheophytinase.

Any polypeptide having an activity that can hydrolyse chlorophyll or a chlorophyll derivative can be used as the enzyme in the process of the invention. By "enzyme" it is intended to encompass any polypeptide having hydrolytic activity on chlorophyll or a chlorophyll derivative, including e.g. enzyme fragments, etc. Any isolated, recombinant or synthetic or chimeric (or a combination of synthetic and recombinant) polypeptide can be used.

### Enzyme (chlorophyllase, pheophytinase or pyropheophytinase) activity assay

Hydrolytic activity on chlorophyll or a chlorophyll derivative may be detected using any suitable assay technique, for example based on an assay described herein. For example, hydrolytic activity may be detected using fluorescence-based techniques. In one suitable assay, a polypeptide to be tested for hydrolytic activity on chlorophyll or a chlorophyll derivative is incubated in the presence of a substrate, and product or substrate levels are monitored by fluorescence measurement. Suitable substrates include e.g. chlorophyll, pheophytin and/or pyropheophytin. Products which may be detected include chlorophyllide, pheophorbide, pyropheophorbide and/or phytol.

Assay methods for detecting hydrolysis of chlorophyll or a chlorophyll derivative are disclosed in, for example, Ali Khamessan et al. (1994), Journal of Chemical Technology & Biotechnology, 60(1), pages 73 - 81; Klein and Vishniac (1961), J. Biol. Chem. 236: 2544-2547; and Kiani et al. (2006), Analytical Biochemistry 353: 93-98.

Alternatively, a suitable assay may be based on HPLC detection and quantitation of substrate or product levels following addition of a putative enzyme, e.g. based on the techniques described below. In one embodiment, the assay may be performed as described in Hornero-Mendez et al. (2005), Food Research International 38(8-9): 1067-1072. In another embodiment, the following assay may be used:
170 µl mM HEPES, pH 7.0 is added 20 µl 0.3 mM chlorophyll, pheophytin or pyropheophytin dissolved in acetone. The enzyme is dissolved in 50 mM HEPES, pH 7.0. 10 µl enzyme solution is added to 190 µl substrate solution to initiate the reaction and incubated at 40°C for various time periods. The reaction was stopped by addition of 350 µl acetone. Following centrifugation (2 min at 18,000 g) the supernatant was analyzed by HPLC, and the amounts of (i) chlorophyll and chlorophyllide (ii) pheophytin and pheophorbide or (iii) pyropheophytin and pyropheophorbide determined.

One unit of enzyme activity is defined as the amount of enzyme which hydrolyzes one micromole of substrate (e.g. chlorophyll, pheophytin or pyropheophytin) per minute at 40°C, e.g. in an assay method as described herein.

In preferred embodiments, the enzyme used in the present method has chlorophyllase, pheophytinase and/or pyropheophytinase activity of at least 1000 U/g, at least 5000 U/g, at least 10000 U/g, or at least 50000 U/g, based on the units of activity per gram of the purified enzyme, e.g. as determined by an assay method described herein.

### Chlorophyllases

In one embodiment, the enzyme is capable of hydrolyzing at least chlorophyll. Any polypeptide that catalyses the hydrolysis of a chlorophyll ester bond to yield chlorophyllide and phytol can be used in the process. For example, a chlorophyllase, chlase or chlorophyll chlorophyllido-hydrolyase or polypeptide having a similar activity (e.g., chlorophyll-chlorophyllido hydrolase 1 or chlase 1, or, chlorophyll-chlorophyllido hydrolase 2 or chlase 2, see, e.g. NCBI P59677-1 and P59678, respectively) can be used in the process.

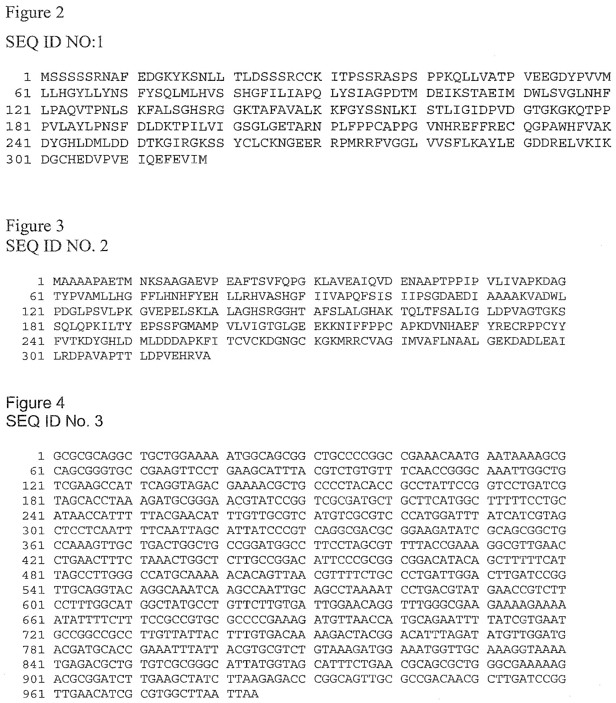

In one embodiment the enzyme is a chlorophyllase classified under the Enzyme Nomenclature classification (E.C. 3.1.1.14). Any isolated, recombinant or synthetic or chimeric (a combination of synthetic and recombinant) polypeptide (e.g., enzyme or catalytic antibody) can be used, see e.g. Marchler-Bauer (2003) Nucleic Acids Res. 31: 383-387. In one aspect, the chlorophyllase may be an enzyme as described in WO 0229022 or WO 2006009676. For example, the *Arabidopsis thaliana* chlorophyllase can be used as described, e.g. in NCBI entry NM_123753. Thus the chlorophyllase may be a polypeptide comprising the sequence of SEQ ID NO:1 (see Figure 2). In another embodiment, the chlorophyllase is derived from algae, e.g. from *Phaeodactylum tricornutum.*

In another embodiment, the chlorophyllase is derived from wheat, e.g. from *Triticum sp.,* especially from *Triticum aestivum.* For example, the chlorophyllase may be polypeptide comprising the sequence of SEQ ID NO:2 (see Figure 3), or may be encoded by the nucleotide sequence of SEQ ID NO:3 (see Figure 4).

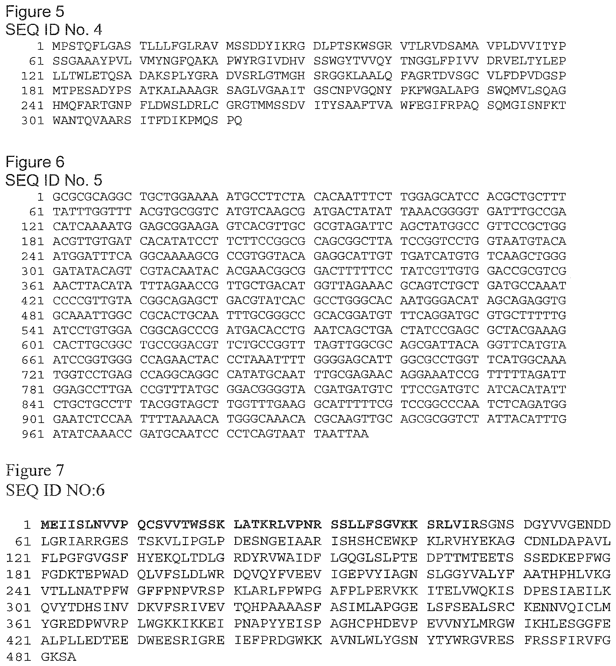

In another embodiment, the chlorophyllase is derived from *Chlamydomonas sp.,* especially from *Chlamydomonas reinhardtii.* For example, the chlorophyllase may be a polypeptide comprising the sequence of SEQ ID NO:4 (see Figure 5), or may be encoded by the nucleotide sequence of SEQ ID NO:5 (see Figure 6).

### Pheophytin pheophorbide hydrolase

In one embodiment, the enzyme is capable of hydrolyzing pheophytin and pyropheophytin. For example, the enzyme may be pheophytinase or pheophytin pheophorbide hydrolase (PPH), e.g. an enzyme as described in Schelbert et al., The Plant Cell 21:767-785 (2009).

PPH and related enzymes are capable of hydrolyzing pyropheophytin in addition to pheophytin. However PPH is inactive on chlorophyll. As described in Schelbert *et al.,* PPH orthologs are commonly present in eukaryotic photosynthesizing organisms. PPHs represent a defined sub-group of α/β hydrolases which are phylogenetically distinct from chlorophyllases, the two groups being distinguished in terms of sequence homology and substrates.

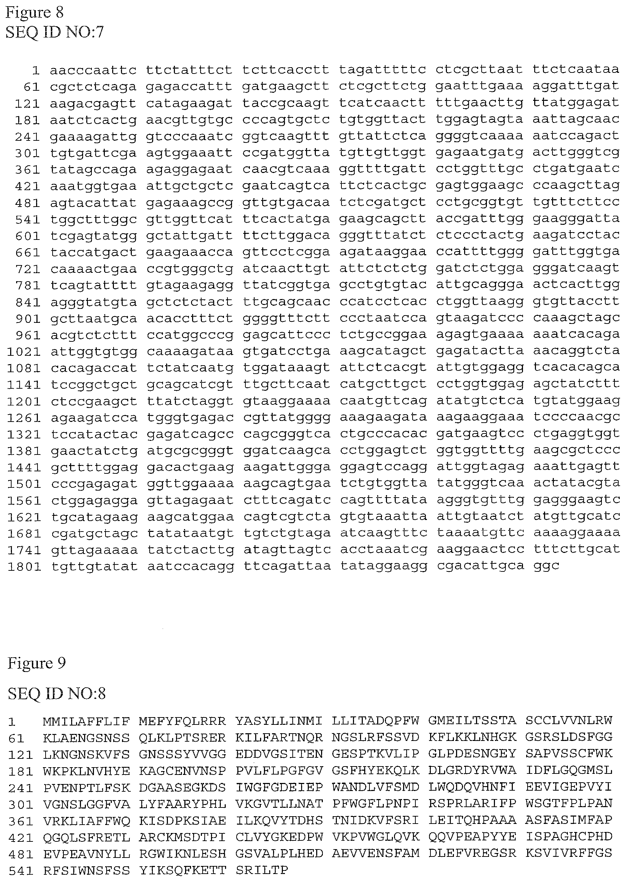

In specific embodiments of the invention, the enzyme may be any known PPH derived from any species or a functional variant or fragment thereof or may be derived from any known PPH enzyme. For example, in one embodiment, the enzyme is a PPH from *Arabidopsis thaliana,* e.g. a polypeptide comprising the amino acid sequence of SEQ ID NO:6 (see Figure 7), or a polypeptide encoded by the nucleotide sequence of SEQ ID NO:7 (see Figure 8, NCBI accession no. NP_196884, GenBank ID No. 15240707), or a functional variant or fragment thereof.

In further embodiments, the enzyme may be a PPH derived from any one of the following species: *Arabidopsis thaliana, Populus trichocarpa, Vitis vinifera, Oryza sativa, Zea mays, Nicotiana tabacum, Ostreococcus lucimarinus, Ostreococcus taurii, Physcomitrella patens, Phaeodactylum tricornutum, Chlamydomonas reinhardtii,* or *Micromonas sp. RCC299.* For example, the enzyme may be a polypeptide comprising an amino acid sequence, or encoded by a nucleotide sequence, defined in one of the following database entries shown in Table 1, or a functional fragment or variant thereof:

**Table 1**

| **Organism** | **Accession** | **Genbank ID** |
|---|---|---|
| Arabidopsis thaliana | NP_196884 | 15240707 |
| Populus trichocarpa | XP_002314066 | 224106163 |
| Vitis vinifera | CAO40741 | 157350650 |
| Oryza sativa (japonica) | NP_001057593 | 115467988 |
| Zea mays | ACF87407 | 194706646 |
| Nicotiana tabacum | CAO99125 | 156763846 |
| Ostreococcus lucimarinus | XP_001415589 | 145340970 |
| Ostreococcus tauri | CAL50341 | 116000661 |
| Physcomitrella patens | XP_001761725 | 168018382 |
| Phaeodactylum tricornutum | XP_002181821 | 219122997 |
| Chlamydomonas reinhardtii | XP_001702982 | 159490010 |
| Micromonas sp. RCC299 | ACO62405 | 226516410 |

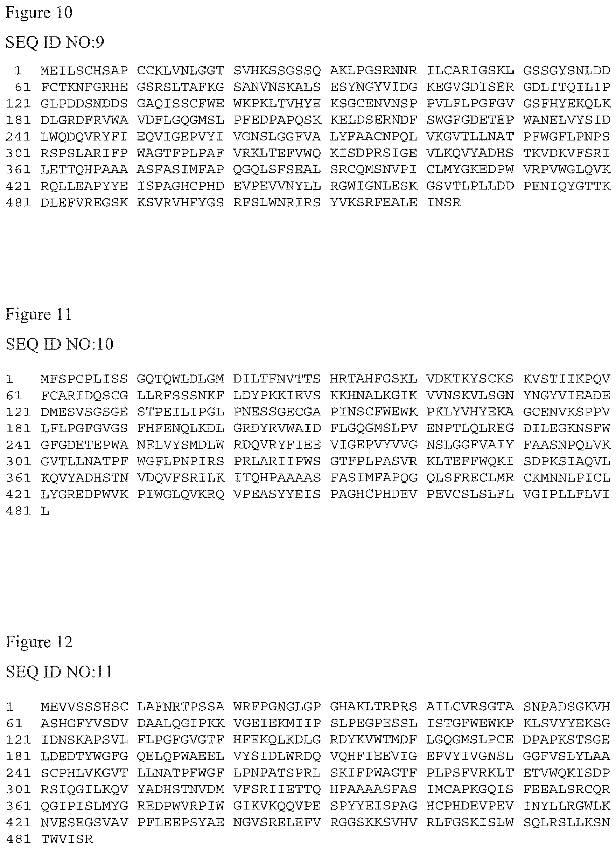

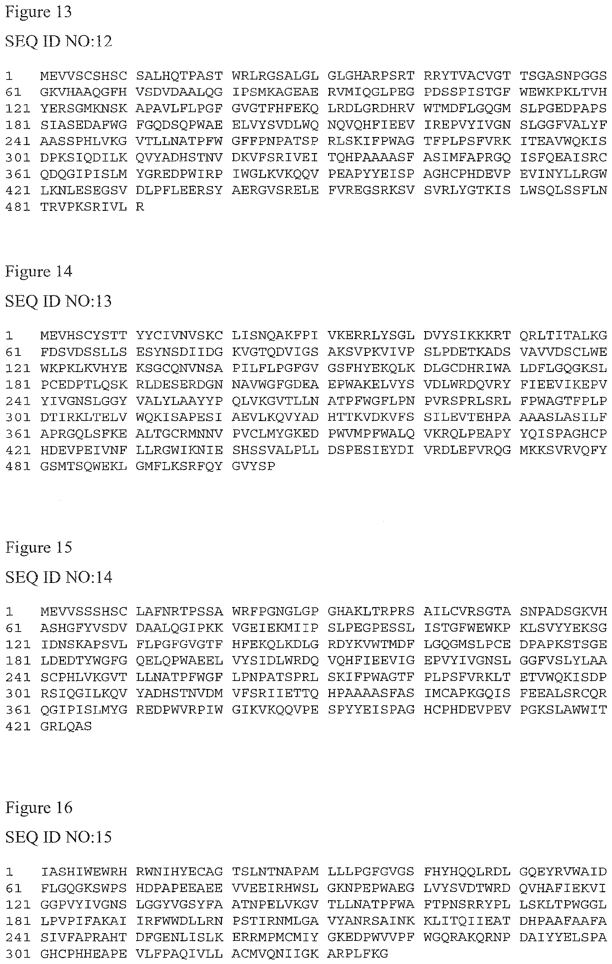

For example, the enzyme may be a polypeptide as defined in any of SEQ ID NO:s 8 to 15 (Figures 9 to 16), or a functional fragment or variant thereof.

### Variants and fragments

Functional variants and fragments of known sequences which hydrolyse chlorophyll or a chlorophyll derivative may also be employed in the present invention. By "functional" it is meant that the fragment or variant retains a detectable hydrolytic activity on chlorophyll or a chlorophyll derivative. Typically such variants and fragments show homology to a known chlorophyllase, pheophytinase or pyropheophytinase sequence, e.g. at least about 50%, 60%, 70%, 75%, 80%, 85%, 90%, 95%, 96%, 97%, 98%, 99%, or more sequence identity to a known chlorophyllase, pheophytinase or pyropheophytinase amino acid sequence, e.g. to SEQ ID NO:1 or any one of SEQ ID NOs: 1, 2, 4, 6 or 8 to 15, e.g. over a region of at least about 10, 20, 30, 50, 100, 200, 300, 500, or 1000 or more residues, or over the entire length of the sequence.

The percentage of sequence identity may be determined by analysis with a sequence comparison algorithm or by a visual inspection. In one aspect, the sequence comparison algorithm is a BLAST algorithm, e.g., a BLAST version 2.2.2 algorithm.

Other enzymes having chlorophyllase, pheophytinase and/or pyropheophytinase activity suitable for use in the process may be identified by determining the presence of conserved sequence motifs present e.g. in known chlorophyllase, pheophytinase or pyropheophytinase sequences. For example, conserved sequence motifs found in PPH enzymes include the following: LPGFGVG (SEQ ID NO:16), DFLGQG (SEQ ID NO:17), GNSLGG (SEQ ID NO:18), LVKGVTLLNATPFW (SEQ ID NO:19), HPAA (SEQ ID NO:20), EDPW (SEQ ID NO:21), and SPAGHCPH (SEQ ID NO:22). In some embodiments, an enzyme for use in the present invention may comprise one or more of these sequences. The GNSLGG (SEQ ID NO:18) motif contains an active site serine residue. Polypeptide sequences having suitable activity may be identified by searching genome databases, e.g. the microbiome metagenome database (JGI-DOE, USA), for the presence of these motifs.

### Isolation and production of enzymes

Enzymes for use in the present invention may be isolated from their natural sources or may be, for example, produced using recombinant DNA techniques. Nucleotide sequences encoding polypeptides having chlorophyllase, pheophytinase and/or pyropheophytinase activity may be isolated or constructed and used to produce the corresponding polypeptides.

For example, a genomic DNA and/or cDNA library may be constructed using chromosomal DNA or messenger RNA from the organism producing the polypeptide. If the amino acid sequence of the polypeptide is known, labeled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or antisense strand.

Typically, the nucleotide sequence encoding a polypeptide having chlorophyllase, pheophytinase and/or pyropheophytinase activity is prepared using recombinant DNA techniques. However, in an alternative embodiment of the invention, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

### Modification of enzyme sequences

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites. A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of enzymes which hydrolyse chlorophyll and/or chlorophyll derivatives with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of enzymes with preferred characteristics. Suitable methods for performing 'shuffling' can be found in EP0752008, EP1138763, EP1103606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known chlorophyllase, pheophytinase or pyropheophytinase enzymes, but have very low amino acid sequence homology.

As a non-limiting example, in addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.
As will be apparent to a person skilled in the art, using molecular evolution tools an enzyme may be altered to improve the functionality of the enzyme. Suitably, a nucleotide sequence encoding an enzyme (e.g. a chlorophyllase, pheophytinase and/or pyropheophytinase) used in the invention may encode a variant enzyme, i.e. the variant enzyme may contain at least one amino acid substitution, deletion or addition, when compared to a parental enzyme. Variant enzymes retain at least 1%, 2%, 3%, 5%, 10%, 15%, 20%, 30%, 40%, 50 %, 60%, 70%, 80%, 90%, 95%, 97%, or 99% identity with the parent enzyme. Suitable parent enzymes may include any enzyme with hydrolytic activity on chlorophyll and/or a chlorophyll derivative.

### Polypeptide sequences

Also disclosed is the use of amino acid sequences encoded by a nucleotide sequence which encodes a pyropheophytinase for use in any one of the methods and/or uses of the present invention.
As used herein, the term "amino acid sequence" is synonymous with the term "polypeptide" and/or the term "protein". In some instances, the term "amino acid sequence" is synonymous with the term "peptide". The amino acid sequence may be prepared/isolated from a suitable source, or it may be made synthetically or it may be prepared by use of recombinant DNA techniques. Suitably, the amino acid sequences may be obtained from the isolated polypeptides taught herein by standard techniques.
One suitable method for determining amino acid sequences from isolated polypeptides is as follows. Purified polypeptide may be freeze-dried and 100 µg of the freeze-dried material may be dissolved in 50 µ1 of a mixture of 8 M urea and 0.4 M ammonium hydrogen carbonate, pH 8.4. The dissolved protein may be denatured and reduced for 15 minutes at 50°C following overlay with nitrogen and addition of 5 µl of 45 mM dithiothreitol. After cooling to room temperature, 5 µ1 of 100 mM iodoacetamide may be added for the cysteine residues to be derivatized for 15 minutes at room temperature in the dark under nitrogen.
135 µ1 of water and 5 µg of endoproteinase Lys-C in 5 µl of water may be added to the above reaction mixture and the digestion may be carried out at 37°C under nitrogen for 24 hours. The resulting peptides may be separated by reverse phase HPLC on a VYDAC C18 column (0.46x15cm; 10µm; The Separation Group, California, USA) using solvent A: 0.1% TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

### Sequence comparison

Here, the term "homologue" means an entity having a certain homology with the subject amino acid sequences and the subject nucleotide sequences. Here, the term "homology" can be equated with "identity". The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences. % homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology therefore firstly requires the production of an optimal alignment, taking into consideration gap penalties. A suitable computer program for carrying out such an alignment is the Vector NTI Advance™ 11 (Invitrogen Corp.). Examples of other software that can perform sequence comparisons include, but are not limited to, the BLAST package (see Ausubel et al 1999 Short Protocols in Molecular Biology, 4th Ed - Chapter 18), and FASTA (Altschul et al 1990 J. Mol. Biol. 403-410). Both BLAST and FASTA are available for offline and online searching (see Ausubel et al 1999, pages 7-58 to 7-60). However, for some applications, it is preferred to use the Vector NTI Advance™ 11 program. A new tool, called BLAST 2 Sequences is also available for comparing protein and nucleotide sequence (see FEMS Microbiol Lett 1999 174(2): 247-50; and FEMS Microbiol Lett 1999 177(1): 187-8.).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI Advance™ 11 package.

Alternatively, percentage homologies may be calculated using the multiple alignment feature in Vector NTI Advance™ 11 (Invitrogen Corp.), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

Should Gap Penalties be used when determining sequence identity, then preferably the default parameters for the programme are used for pairwise alignment. For example, the following parameters are the current default parameters for pairwise alignment for BLAST 2:

| FOR BLAST2 | DNA | PROTEIN |
|---|---|---|
| EXPECT THRESHOLD | 10 | 10 |
| WORD SIZE | 11 | 3 |

| SCORING PARAMETERS | | |
|---|---|---|
| Match/Mismatch Scores | 2, -3 | n/a |
| Matrix | n/a | BLOSUM62 |
| Gap Costs | Existence: 5 | Existence: 11 |
| | Extension: 2 | Extension: 1 |

In one embodiment, preferably the sequence identity for the nucleotide sequences and/or amino acid sequences may be determined using BLAST2 (blastn) with the scoring parameters set as defined above.

For the purposes of the present invention, the degree of identity is based on the number of sequence elements which are the same. The degree of identity in accordance with the present invention for amino acid sequences may be suitably determined by means of computer programs known in the art such as Vector NTI Advance™ 11 (Invitrogen Corp.). For pairwise alignment the scoring parameters used are preferably BLOSUM62 with Gap existence penalty of 11 and Gap extension penalty of 1.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides. Suitably, the degree of identity with regard to a nucleotide sequence may be determined over the whole sequence.

### Amino acid mutations

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMA TIC | | H F W Y |

Also disclosed is homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine. Replacements may also be made by unnatural amino acids.
Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

### Nucleotide sequences

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences. Also disclosed is the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.
Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in plant cells, may be obtained and such homologues and fragments thereof in general will be capable of selectively hybridising to the sequences shown in the sequence listing herein. Such sequences may be obtained by probing cDNA libraries made from or genomic DNA libraries from other plant species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.
Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the pyropheophytinase sequence which it is desired to clone, bringing the primers into contact with mRNA or cDNA obtained from a plant cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

### Enzyme formulation and dosage

Enzymes used in the methods of the invention can be formulated or modified, e.g., chemically modified, to enhance oil solubility, stability, activity or for immobilization. For example, enzymes used in the methods of the invention can be formulated to be amphipathic or more lipophilic. For example, enzymes used in the methods of the invention can be encapsulated, e.g., in liposomes or gels, e.g., alginate hydrogels or alginate beads or equivalents. Enzymes used in the methods of the invention can be formulated in micellar systems, e.g., a ternary micellar (TMS) or reverse micellar system (RMS) medium. Enzymes used in the methods of the invention can be formulated as described in Yi (2002) J. of Molecular Catalysis B: Enzymatic, Vol. 19, pgs 319-325.

The enzymatic reactions of the methods of the invention, e.g. the step of contacting the oil with an enzyme which hydrolyses chlorophyll or a chlorophyll derivative, can be done in one reaction vessel or multiple vessels. In one aspect, the enzymatic reactions of the methods of the invention are done in a vegetable oil refining unit or plant.

The method of the invention can be practiced with immobilized enzymes, e.g. an immobilized chlorophyllase, pheophytinase and/or pyropheophytinase. The enzyme can be immobilized on any organic or inorganic support. Exemplary inorganic supports include alumina, celite, Dowex-1-chloride, glass beads and silica gel. Exemplary organic supports include DEAE-cellulose, alginate hydrogels or alginate beads or equivalents. In various aspects of the invention, immobilization of the enzyme can be optimized by physical adsorption on to the inorganic support. Enzymes used to practice the invention can be immobilized in different media, including water, Tris-HCl buffer solution and a ternary micellar system containing Tris-HCl buffer solution, hexane and surfactant. The enzyme can be immobilized to any type of substrate, e.g. filters, fibers, columns, beads, colloids, gels, hydrogels, meshes and the like.

The enzyme may be dosed into the oil in any suitable amount. For example, the enzyme may be dosed in a range of about 0.001 to 10U/g of the composition, preferably 0.01 to 1 U/g, e.g. 0.01 to 0.1 U/g of the oil. One unit is defined as the amount of enzyme which hydrolyses 1 µmol of substrate (e.g. chlorophyll, pheophytin and/or pyropheophytin) per minute at 40 °C, e.g. under assay conditions as described in J. Biol. Chem. (1961) 236: 2544-2547.

### Phospholipid content

In the process of the present invention, the enzyme is contacted with the oil in the presence of at least 1 % by weight phospholipid. For example, the phospholipid content of the oil may be at least 1 % by weight, e.g. based on the total weight of the oil composition, for at least a part of a time during which the enzyme is incubated with the oil (e.g. at least at a time when the enzyme is added to the oil).
In some embodiments, for example where the enzyme is added during a degumming step, the phospholipid content of the oil may decrease during the time in which the enzyme is incubated with the oil. However, provided that the phospholipid content of the oil is 1 % by weight or above for at least part of the incubation with the enzyme (e.g. at the start of the incubation), the enzyme is likely to be active. Therefore in some embodiments the phospholipid content of the oil may be less than 1 % by weight during a part of the incubation period with the enzyme.
The phospholipid is typically present as a natural component of a crude plant oil, although in some embodiments phospholipid may be added to the oil to be treated with the enzyme. Phospholipids commonly found in crude plant oils include phosphatidyl choline (PC), phosphatidyl inositol (PI), phosphatidyl ethanolamine (PE), phosphatidyl serine (PS) and phosphatidic acid (PA). In preferred embodiments, the phospholipid comprises one or more of PC, PI, PE, PS and PA. Phospholipids are typically present in crude oils in the form of lecithin, the major component of which is PC. Thus in one embodiment, the phospholipid comprises lecithin. The term lecithin as used herein encompasses phosphatidyl choline, phosphatidyl inositol, phosphatidyl ethanolamine, phosphatidyl serine and phosphatidic acid.

The phospholipid (e.g. PC, PI, PE, PS, PA and/or lecithin) content of the oil is at least 1 % by weight during contact with the enzyme. In particular embodiments, the phospholipid content is at least 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9% or at least 3.0% by weight, e.g. based on the total weight of the oil. Preferably the phospholipid content is up to 5.0%, up to 4.0%, or up to 3.0% by weight. For example, the phospholipid content of the oil may be 1.0 to 5.0%, 1.0 to 4.0%, 1.0 to 3.0%, 2.0 to 5.0%, 2.0 to 4.0% or 2.0 to 3.0% by weight, e.g. based on the total weight of the oil.

The phospholipid content of plant oils varies according to the particular source and nature of the oil and the stage of the refining process. The phospholipid content of crude plant oils may be up to 5% by weight at the start of the process, but following a water degumming step the phospholipid content typically falls to 1% by weight or below, e.g. around 0.3 % by weight. Following an enzymatic degumming step (e.g. using a phospholipase) or a total degumming step (e.g. comprising an acid treatment/caustic neutralization) the phospholipid content may fall much lower, for example below 0.1 % or even below 0.01% by weight based on the total weight of the oil. Typical phospholipid contents in % by weight of some common oils are shown below:

| | **Canola** | **Rapeseed** | **Soybean** |
|---|---|---|---|
| **Crude oil** | ≤2.5 | ≤3.5 | ≤4.0 |
| **Water-degummed oil** | ≤0.6 | ≤0.8 | ≤0.4 |
| **Acid-degummed oil** | ≤0.1 | - | ≤0.2 |

The values in the table above are taken from Bailey's Industrial Oil and Fat Products (2005), 6th edition, Ed. by Fereidoon Shahidi, John Wiley & Sons, and the phospholipid content of other oils is also described therein or is well-known in the art. The phospholipid content of oils may be determined using standard methods. For example, phospholipid levels in oils may be determined as described in J. Amer. Oil. Chem. Soc. 58, 561 (1981). In one embodiment phospholipid levels may be determined by thin-layer chromatography (TLC) analysis, e.g. as described in WO 2006/008508 or WO 03/100044. Phospholipid levels in oil can also be determined by (a) AOCS Recommended Practice Ca 19-86 (reapproved 2009), "Phospholipids in Vegetable Oils Nephelometric Method" or (b) AOCS Official Method Ca 20-99 (reapproved 2009), "Analysis for Phosphorus in Oil by Inductively Coupled Plasma Optical Emission Spectroscopy".
Thus in one preferred embodiment, the enzyme is contacted with a crude plant oil (e.g. an oil comprising at least 1.0% or at least 2% by weight phospholipid).

### Lysophospholipid content

The enzyme is contacted with the oil at a time when a concentration of lysophospholipid in the oil is as low as possible. The enzyme is contacted with the oil in the presence of less than 0.2% by weight lysophosholipid. By "in the presence of less than 0.2% by weight lysophosholipid" it is meant that the lysophospholipid content in the oil is less than 0.2% by weight, e.g. based on the total weight of the oil composition, for at least a part of a time during which the enzyme is incubated with the oil (e.g. at least at a time when the enzyme is added to the oil). The lysophospholipid content in the oil may be any value below 0.2% by weight, including zero.
In some embodiments, for example where the enzyme is added during a degumming step, the lysophospholipid content of the oil may increase during the time in which the enzyme is incubated with the oil. This is particularly the case where the process comprises an enzymatic degumming step using an enzyme which generates lysophospholipids. Lysophospholipids are typically produced during oil processing by cleavage of an acyl (fatty acid) chain from phospholipids, leaving a single acyl chain, a phosphate group, optionally a headgroup and a free alcohol attached to the glyceryl moiety. Enzymes used in degumming such as phospholipases (in particular phospholipase A1 and A2) and acyltransferases may generate lysophospholipids in the oil. In embodiments where the process comprises an enzymatic degumming step using an enzyme which generates lysophospholipids, the enzyme which hydrolyses chlorophyll or a chlorophyll derivative is preferably contacted with the oil before the enzymatic degumming step.
In one embodiment, a lysophospholipase may be used in combination with a phospholipase or acyltransferase in the degumming step. Lysophospholipases (EC 3.1.1.5) are enzymes that can hydrolyze lysophospholipids to release fatty acid. Use of a lysophospholipase may help to reduce the production of lysophospholipids in the oil during the degumming step, e.g. to maintain the lysophospholipid content of the oil below about 0.2% by weight. Suitable lysophospholipases are disclosed, for example, in Masuda et al., Eur. J. Biochem., 202,783-787 (1991); WO 98/31790; WO 01/27251 and WO 2008/040465.
Phospholipase C is another enzyme which may be used in degumming. Phospholipase C cleaves phospholipids between the glyceryl and phosphate moieties, leaving diacylglycerol and a phosphate group (attached to a headgroup if present). Thus in contrast to phospholipase A1 and A2, phospholipase C does not produce lysophospholipids. In embodiments where the process comprises an enzymatic degumming step using an enzyme which does not produce lysophospholipids (e.g. phospholipase C), the enzyme which hydrolyses chlorophyll or a chlorophyll derivative may be contacted with the oil either before or during the enzymatic degumming step.

In particular embodiments, the lysophospholipid content of the oil is less than 0.15%, less than 0.1% or less than 0.05% by weight, based on the total weight of oil. In general, concentrations of lysophospholipid which are as low as possible are desirable. Lysophospholipids which may be present in the oil include lysophosphatidylcholine (LPC), lysophosphatidylinositol (LPI), lysophosphatidylethanolamine (LPE), lysophosphatidylserine (LPS) and lysophosphatidic acid (LPA). It is particularly preferred that the level of LPC and LPE in the oil is as low as possible. In preferred embodiments, the concentration of LPC and/or LPE is less than 0.2%, less than 0.15%, less than 0.1% or less than 0.05% by weight, based on the total weight of oil.
The lysophospholipid content of oils may be determined using standard methods, e.g. as described above for phospholipids, including using HPLC or TLC analysis methods. Suitable methods are described in AOCS Recommended Practice Ja 7-86 (reapproved 2009), "Phospholipids in Lecithin Concentrates by Thin-Layer Chromatography" or Journal of Chromatography A, 864 (1999) 179-182.

### Enzyme reaction conditions

In general the oil may be incubated (or admixed) with the enzyme between about 5°C to and about 100°C, more preferably between 10°C to about 90°C, more preferably between about 15°C to about 80°C, more preferably between about 20°C to about 75°C.
At higher temperatures pheophytin is decomposed to pyropheophytin, which is generally less preferred because some chlorophyllases are less active on pyropheophytin compared to pheophytin. In addition, the chlorophyllase degradation product of pyropheophytin, pyropheophorbide, is less water soluble compared to pheophorbide and thus more difficult to remove from the oil afterwards. The enzymatic reaction rate is increased at higher temperatures but it is favourable to keep the conversion of pheophytin to pyropheophytin to a minimum.

In view of the above, in particularly preferred embodiments the oil is incubated with the enzyme at below about 80°C, preferably below about 70°C, preferably at about 68°C or below, preferably at about 65°C or below, in order to reduce the amount of conversion to pyropheophytin. However, in order to keep a good reaction rate it is preferred to keep the temperature of the oil above 50 °C during incubation with the enzyme. Accordingly preferred temperature ranges for the incubation of the enzyme with the oil include about 50°C to below about 70°C, about 50°C to about 65°C and about 55°C to about 65°C. Preferably the enzyme is contacted with the oil at about 57°C to about 63°C, preferably about 58°C to about 62°C, e.g about 60°C.

Preferably the temperature of the oil may be at the desired reaction temperature when the enzyme is admixed therewith. The oil may be heated and/or cooled to the desired temperature before and/or during enzyme addition. Therefore in one embodiment it is envisaged that a further step of the process according to the present invention may be the cooling and/or heating of the oil.

Suitably the reaction time (i.e. the time period in which the enzyme is incubated with the oil), preferably with agitation, is for a sufficient period of time to allow hydrolysis of chlorophyll and chlorophyll derivatives, e.g. to form phytol and chlorophyllide, pheophorbide and/or pyropheophorbide. For example, the reaction time may be at least about 1 minute, more preferable at least about 5 minutes, more preferably at least about 10 minutes. In some embodiments the reaction time may be between about 15 minutes to about 6 hours, preferably between about 15 minutes to about 60 minutes, preferably about 30 to about 120 minutes. In some embodiments, the reaction time may up to 6 hours.

Preferably the process is carried out between about pH 4.0 and about pH 10.0, more preferably between about pH 5.0 and about pH 10.0, more preferably between about pH 6.0 and about pH 10.0, more preferably between about pH 5.0 and about pH 7.0, more preferably between about pH 5.0 and about pH 7.0, more preferably between about pH 6.5 and about pH 7.0, e.g. at about pH 7.0 (i.e. neutral pH). In one embodiment preferably the process is carried out between about pH 5.5 and pH 6.0. In another embodiment, the process is carried out between about pH 6.0 to pH 6.8, e.g. between about pH 6.3 and pH 6.5, preferably about pH 6.4.

Suitably the water content of the oil when incubated (or admixed) with the enzyme is between about 0.5 to about 5% water, more preferably between about 1 to about 3% and more preferably between about 1.5 and about 2% by weight. In specific embodiments, the water content may be, for example, 0.7% to 1.2%, e.g. about 1% by weight; or 1.7% to 2.2%, e.g. about 2% by weight.

When an immobilised enzyme is used, suitably the water activity of the immobilised enzyme may be in the range of about 0.2 to about 0.98, preferably between about 0.4 to about 0.9, more preferably between about 0.6 to about 0.8.

### Oil separation

Following an enzymatic treatment step using an enzyme according to the present invention, in one embodiment the treated liquid (e.g. oil) is separated with an appropriate means such as a centrifugal separator and the processed oil is obtained. Upon completion of the enzyme treatment, if necessary, the processed oil can be additionally washed with water or organic or inorganic acid such as, e.g., acetic acid, citric acid, phosphoric acid, succinic acid, and the like, or with salt solutions.

### Chlorophyll and/or chlorophyll derivative removal

The process of the present invention involving an enzyme treatment typically reduces the level of chlorophyll and/or chlorophyll derivatives in the oil. For example, the process may reduce the concentration of chlorophyll, pheophytin and/or pyropheophytin by at least 5%, at least 10%, at least 20%, at least 30%, at least 40%, at least 50%, at least 60%, at least 70%, at least 80%, at least 90%, at least 95% or at least 99%, compared to the concentration of chlorophyll, pheophytin and/or pyropheophytin (by weight) present in the oil before treatment. Thus in particular embodiments, the concentration of chlorophyll and/or chlorophyll derivatives in the oil after treatment may be less than 100, less than 50, less than 30, less than 10, less than 5, less than 1, less than 0.5, less than 0.1 mg/kg or less than 0.02 mg/kg, based on the total weight of the oil.

### Further processing steps

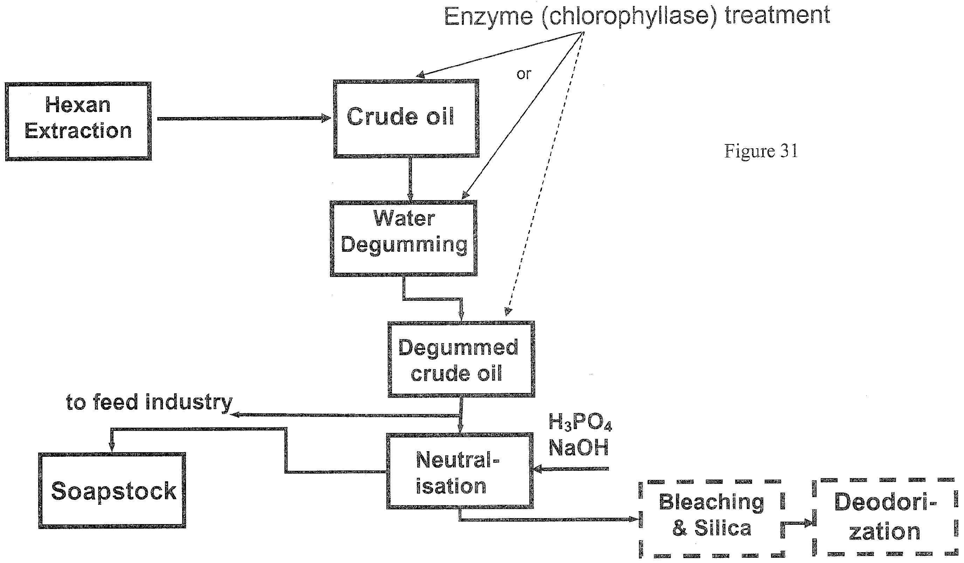

In a typical plant oil processing method, oil is extracted in hexane, the crude vegetable oil is degummed, optionally caustic neutralized, bleached using, e.g. clay adsorption with subsequent clay disposal, and deodorized to produce refined, bleached and deodorized or RBD oil (see Figure 31). The need for the degumming step depends on phosphorus content and other factors. The process of the present invention can be used in conjunction with processes based on extraction with hexane and/or enzyme assisted oil extraction (see Journal of Americal Oil Chemists' Society (2006), 83 (11), 973-979). In general, the process of the invention may be performed using oil processing steps as described in Bailey's Industrial Oil and Fat Products (2005), 6th edition, Ed. by Fereidoon Shahidi, John Wiley & Sons.
In embodiments of the present invention, an enzymatic reaction involving application of the enzyme capable of hydrolyzing chlorophyll or a chlorophyll derivative is preferably performed at specific stages in this process. In particular, according to the present invention the enzyme is contacted with the oil in the presence of at least 1 % by weight phospholipid and less than 0.2% by weight lysophospholipid. Although the level of phospholipid and lysophospholipid in the oil at different stages of the process will vary depending on the nature and source of the oil, it is generally preferred to contact the enzyme with the oil at a stage in the process before phospholipid levels are substantially reduced and before lysophospholipid levels are elevated.
Preferred stages of the process for using the enzyme according to the present process are shown in Figure 31. In particular embodiments the enzyme is preferably contacted with the oil before the degumming step. In another embodiment, the enzyme may be contacted with the oil during a water degumming step. The enzyme is typically contacted with the oil before degumming is complete (e.g. before a caustic neutralization step).
In some embodiments, the enzyme may be contacted with the oil after water degumming (e.g. the enzyme is added to water-degummed oil), provided that the enzymatic hydrolysis of chlorophyll and chlorophyll derivatives is performed before a total degumming step, e.g. before addition of acid and caustic neutralization. This is shown by a dashed line in Fig. 31. Thus the enzyme may be added after partial degumming of the oil, provided that at least 0.1 % phospholipid by weight is still present. In general however, it is preferable to add the enzyme at as high a phospholipid level as possible (at least 1.0 % by weight phospholipid) and using the enzyme after partial degumming is generally less preferred. Further processing steps, after treatment with the enzyme, may assist in removal of the products of enzymatic hydrolysis of chlorophyll and/or chlorophyll derivatives. For instance, further processing steps may remove chlorophyllide, pheophorbide, pyropheophorbide and/or phytol.

### Degumming

The degumming step in oil refining serves to separate phosphatides by the addition of water. The material precipitated by degumming is separated and further processed to mixtures of lecithins. The commercial lecithins, such as soybean lecithin and sunflower lecithin, are semisolid or very viscous materials. They consist of a mixture of polar lipids, primarily phospholipids such as phosphatidylcholine with a minor component of triglycerides. Thus as used herein, the term "degumming" means the refining of oil by removing phospholipids from the oil. In some embodiments, degumming may comprise a step of converting phosphatides (such as lecithin and phospholipids) into hydratable phosphatides.

The process of the invention can be used with any degumming procedure, particularly in embodiments where the chlorophyll- or chlorophyll derivative-hydrolyzing enzyme is contacted with the oil before the degumming step. Thus suitable degumming methods include water degumming, ALCON oil degumming (e.g., for soybeans), safinco degumming, "super degumming," UF degumming, TOP degumming, uni-degumming, dry degumming and ENZYMAX™ degumming. See e.g. U.S. Patent Nos. 6,355,693; 6,162,623; 6,103,505; 6,001,640; 5,558,781; 5,264,367, 5,558,781; 5,288,619; 5,264,367; 6,001,640; 6,376,689; WO 0229022; WO 98118912; and the like. Various degumming procedures incorporated by the methods of the invention are described in Bockisch, M. (1998), Fats and Oils Handbook, The extraction of Vegetable Oils (Chapter 5), 345-445, AOCS Press, Champaign, Illinois.

Water degumming typically refers to a step in which the oil is incubated with water (e.g. 1 to 5% by weight) in order to remove phosphatides. Typically water degumming may be performed at elevated temperature, e.g. at 50 to 90°C. The oil/water mixture may be agitated for e.g. 5 to 60 minutes to allow separation of the phosphatides into the water phase, which is then removed from the oil.

Acid degumming may also be performed. For example, oil may be contacted with acid (e.g. 0.1 to 0.5% of a 50% solution of citric or malic acid) at 60 to 70°C, mixed, contacted with 1 to 5% water and cooled to 25 to 45 °C.

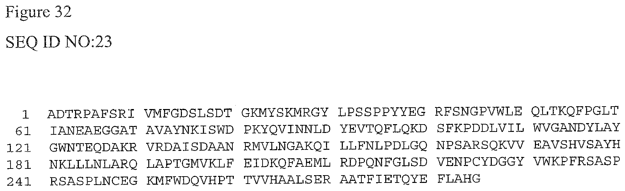

Further suitable degumming procedures for use with the process of the present invention are described in WO 2006/008508. In one embodiment the process comprises contacting the chlorophyll- or chlorophyll derivative-hydrolyzing enzyme with the oil and subsequently performing an enzymatic degumming step using an acyltransferase as described in WO 2006/008508. Acyltransferases suitable for use in the process are also described in WO 2004/064537, WO 2004/064987 and WO 2009/024736. Any enzyme having acyltransferase activity (generally classified as E.C.2.3.1) may be used, particularly enzymes comprising the amino acid sequence motif GDSX, wherein X is one or more of the following amino acid residues: L, A, V, I, F, Y, H, Q, T, N, M or S. In one embodiment, acyltransferase is a mutant *Aeromonas salmonicida* mature lipid acyltransferase (GCAT) with a mutation of Asn80Asp, e.g. an acyltransferase comprising the amino acid sequence of SEQ ID NO:23 after undergoing post-translational modification (see Figure 32), or an enzyme having at least 80% sequence identity thereto.

In another embodiment, the process comprises a degumming step using a phospholipase. Any enzyme having e.g. a phospholipase A1 (E.C.3.1.1.32) or a phospholipase A2 (E.C.3.1.1.4) activity may be used, for example Lecitase Ultra® or pancreatic phospholipase A2 (Novozymes, Denmark). In one embodiment the process comprises contacting the chlorophyll- or chlorophyll derivative-hydrolyzing enzyme with the oil and subsequently performing an enzymatic degumming step using a phospholipase, for example using a degumming step as described in US 5,264,367, EP 0622446, WO 00/32758 or Clausen (2001) "Enzymatic oil degumming by a novel microbial phospholipase," Eur. J. Lipid Sci. Technol. 103:333-340.

In embodiments where the degumming step is performed simultaneously with the chlorophyll or chlorophyll derivative hydrolysis step, preferably the degumming process does not produce lysophospholipids. For example, in these embodiments the degumming step may be a water degumming step. In another such embodiment, an enzymatic degumming step using an enzyme such as phospholipase C (IUB 3.1.4.1) may be used. Polypeptides having phospholipase C activity which are may be used in a degumming step are disclosed, for example, in WO2008143679, WO2007092314, WO2007055735, WO2006009676 and WO03089620. A suitable phospholipase C for use in the present invention is Purifine®, available from Verenium Corporation, Cambridge, MA.

### Acid treatment/caustic neutralization

In some embodiments, an acid treatment/caustic neutralization step may be performed in order to further reduce phospholipid levels in the oil after water degumming. In another embodiment, a single degumming step comprising acid treatment/caustic neutralization may be performed. Such methods are typically referred to as total degumming or alkali refining.

It has been found that an acid treatment/caustic neutralization step is particularly effective in removing products of the enzymatic hydrolysis of chlorophyll, e.g. chlorophyllide, pheophorbide and pyropheophorbide. Thus this step may be performed at any stage in the process after the enzyme treatment step. For example, such a step may comprise addition of an acid such as phosphoric acid followed by neutralization with an alkali such as sodium hydroxide. Following an acid/caustic neutralization treatment compounds such as chlorophyllide, pheophorbide and pyropheophorbide are extracted from the oil in an aqueous phase.

In such methods, the oil is typically first contacted with 0.05 to 0.5% by weight of concentrated phosphoric acid, e.g. at a temperature of 50 to 90°C, and mixed to help precipitate phosphatides. The contact time may be, e.g. 10 seconds to 30 minutes. Subsequently an aqueous solution of an alkali (e.g. 1 to 20% aqueous sodium hydroxide) is added, e.g. at a temperature of 50 to 90°C, followed by incubation and mixing for 10 seconds to 30 minutes. The oil may then be heated to about 90°C and the aqueous soap phase separated from the oil by centrifugation.

Optionally, further wash steps with e.g. sodium hydroxide or water may also be performed.

### Chlorophyllide, pheophorbide and pyropheophorbide removal

Thus the method of the present invention may optionally involve a step of removing phytol-free derivatives of chlorophyll such as chlorophyllide, pheophorbide and pyropheophorbide. Such products may be present in the composition due to the hydrolysis of chlorophyll or a chlorophyll derivative by the enzyme of the invention, or may be present naturally, as a contaminant, or as an undesired component in a processed product. Pyropheophorbide may also be present in the composition due to the breakdown of pheophorbide, which may itself be produced by the activity of an enzyme having pheophytinase activity on pheophytin, or pheophorbide may be formed from chlorophyllide following the action of chlorophyllase on chlorophyll (see Figure 1). Processing conditions used in oil refining, in particular heat, may favour the formation of pyropheophorbide as a dominant component, for instance by favouring the conversion of pheophytin to pyropheophytin, which is subsequently hydrolysed to pyropheophorbide.

In one embodiment the process of the present invention reduces the level of chlorophyllide, pheophorbide and/or pyropheophorbide in the oil, compared to either or both of the levels before and after enzyme treatment. Thus in some embodiments the chlorophyllide, pheophorbide and/or pyropheophorbide concentration may increase after enzyme treatment. Typically the process involves a step of removing chlorophyllide, pheophorbide and/or pyropheophorbide such that the concentration of such products is lower than after enzyme treatment. Preferably the chlorophyllide, pheophorbide and/or pyropheophorbide produced by this enzymatic step is removed from the oil, such that the final level of these products in the oil is lower than before enzyme treatment.

For example, the process may reduce the concentration of chlorophyllide, pheophorbide and/or pyropheophorbide by at least 5%, at least 10%, at least 20%, at least 30%, at least 40%, at least 50%, at least 60%, at least 70%, at least 80%, at least 90%, at least 95% or at least 99%, compared to the concentration of chlorophyllide, pheophorbide and/or pyropheophorbide (by weight) present in the oil before the chlorophyllide, pheophorbide and/or pyropheophorbide removal step, i.e. before or after enzyme treatment. Thus in particular embodiments, the chlorophyllide, pheophorbide and/or pyropheophorbide concentration in the oil after the removal step may be less than 100, less than 50, less than 30, less than 10, less than 5, less than 1, less than 0.5, less than 0.1 mg/kg, or less than 0.02 mg/kg, based on the total weight of the composition (e.g a vegetable oil).

It is an advantage of the present process that reaction products such as chlorophyllide, pheophorbide and/or pyropheophorbide may be simply and easily removed from the oil by a step such as acid treatment/caustic neutralization. Thus in preferred embodiments chlorophyll and chlorophyll derivatives may be substantially removed from the oil without the need for further processing steps such as clay and/or silica treatment and deodorization (as indicated by the dashed boxes shown in Fig. 31).

### Clay treatment

It is particularly preferred that the process does not comprise a clay treatment step. Avoiding the use of clay is advantageous for the reasons described earlier, in particular the reduction in cost, the reduced losses of oil through adherence to the clay and the increased retention of useful compounds such as carotenoids and tocopherol.

In some embodiments, the process may be performed with no clay treatment step and no deodorization step, which results in an increased concentration of such useful compounds in the refined oil, compared to a process involving clay treatment.

### Silica treatment

Although not always required, in some embodiments the process may comprise a step of silica treatment, preferably subsequent to the enzyme treatment. For example, the method may comprise use of an adsorbent-free or reduced adsorbent silica refining devices and processes, which are known in the art, e.g., using TriSyl Silica Refining Processes (Grace Davison, Columbia, MD), or, SORBSIL R™ silicas (INEOS Silicas, Joliet, IL).

The silica treatment step may be used to remove any remaining chlorophyllide, pheophorbide and/or pyropheophorbide or other polar components in the oil. For example, in some embodiments a silica treatment step may be used as an alternative to an acid treatment/caustic neutralization (total degumming or alkali refining) step.

In one embodiment the process comprises a two-stage silica treatment, e.g. comprising two silica treatment steps separated by a separation step in which the silica is removed, e.g. a filtration step. The silica treatment may be performed at elevated temperature, e.g. at above about 30°C, more preferably about 50 to 150°C, about 70 to 110°C, about 80 to 100°C or about 85 to 95°C , most preferably about 90°C.

### Deodorization

In some embodiments, the process may comprise a deodorization step, typically as the final refining step in the process. In one embodiment, deodorization refers to steam distillation of the oil, which typically removes volatile odor and flavor compounds, tocopherol, sterols, stanols, carotenoids and other nutrients. Typically the oil is heated to 220 to 260°C under low pressure (e.g. 0.1 to 1 kPa) to exclude air. Steam (e.g. 1-3% by weight) is blown through the oil to remove volatile compounds, for example for 15 to 120 minutes. The aqueous distillate may be collected.

In another embodiment, deodorization may be performed using an inert gas (e.g. nitrogen) instead of steam. Thus the deodoriztion step may comprise bubble refining or sparging with an inert gas (e.g. nitrogen), for example as described by A. V. Tsiadi et al. in "Nitrogen bubble refining of sunflower oil in shallow pools", Journal of the American Oil Chemists' Society (2001), Volume 78 (4), pages 381-385. The gaseous phase which has passed through the oil may be collected and optionally condensed, and/or volatile compounds extracted therefrom into an aqueous phase.

In some embodiments, the process of the present invention is performed with no clay treatment but comprising a deodorization step. Useful compounds (e.g. carotenoids, sterols, stanols and tocopherol) may be at least partially extracted from the oil in a distillate (e.g. an aqueous or nitrogenous distillate) obtained from the deodorization step. This distillate provides a valuable source of compounds such as carotenoids and tocopherol, which may be at least partially lost by entrainment in a process comprising clay treatment.

The loss of tocopherol during bleaching depends on bleaching conditions and the type of clay applied, but 20-40% removal of tocopherol in the bleaching step has been reported (K. Boki, M, Kubo, T. Wada, and T. Tamura, ibid., 69, 323 (1992)). During processing of soy bean oil a loss of 13% tocopherol in the bleaching step has been reported (S. Ramamurthi, A. R. McCurdy, and R. T. Tyler, in S. S. Koseoglu, K. C. Rhee, and R. F. Wilson, eds., Proc. World Conf. Oilseed Edible Oils Process, vol. 1, AOCS Press, Champaign, Illinois, 1998, pp. 130-134).

Carotenoids may be removed from the oil during deodorization in both clay-treated and non-clay-treated oil. Typically the removal of coloured carotenoids is controlled in order to produce an oil having a predetermined colour within a specified range of values. The level of carotenoids and other volatile compounds in the refined oil can be varied by modifying the deodorization step. For instance, in an embodiment where it is desired to retain a higher concentration of carotenoids in the oil, the deodorization step may be performed at a lower temperature (e.g. using steam at 200°C or below). In such embodiments it is particularly preferable to avoid a clay treatment step, since this will result in a higher concentration of carotenoids in the refined oil.

### Further enzyme treatments

In further aspects, the processes of the invention further comprise use of lipid acyltransferases, phospholipases, proteases, phosphatases, phytases, xylanases, amylases (e.g. α-amylases), glucanases, polygalacturonases, galactolipases, cellulases, hemicellulases, pectinases and other plant cell wall degrading enzymes, as well as mixed enzyme preparations and cell lysates. In alternative aspects, the processes of the invention can be practiced in conjunction with other processes, e.g., enzymatic treatments, e.g., with carbohydrases, including cellulase, hemicellulase and other side degrading activities, or, chemical processes, e.g., hexane extraction of soybean oil. In one embodiment the method of the present invention can be practiced in combination with a method as defined in WO 2006031699.

The invention will now be further illustrated with reference to the following non-limiting examples.

### EXAMPLE 1

### Cloning and expression of a chlorophyllase from Triticum aestivum (wheat) in Bacillus subtilis

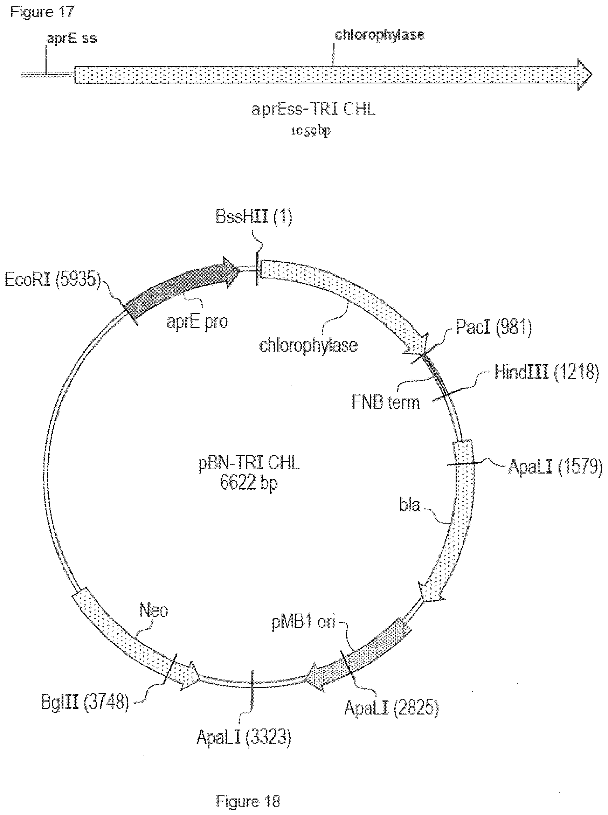

A nucleotide sequence (SEQ ID No. 3) encoding a wheat chlorophyllase (SEQ. ID No. 2, hereinafter wheat chlase) was expressed in *Bacillus subtilis* with the signal peptide of a *B. subtilis* alkaline protease (aprE) (see Fig. 17). For optimal expression in Bacillus, a codon optimized gene construct (TRI_CHL) was ordered at GenScript (GenScript Corporation, Piscataway, NJ 08854, USA).

The construct TRI_CHL contains 20 nucleotides with a BssHII restriction site upstream to the wheat chlase coding region to allow fusion to the aprE signal sequence and a PacI restriction site following the coding region for cloning into the bacillus expression vector pBNppt.

The construct TRI_CHL was digested with BssHII and PacI and ligated with T4 DNA ligase into BssHII and PacI digested pBNppt.

The ligation mixture was transformed into *E. coli* TOP10 cells. The sequence of the BssHII and Pac insert containing the TRI_CHL gene was confirmed by DNA sequencing (DNA Technology A/S, Risskov, Denmark) and one of the correct plasmid clones was designated pBN-TRI_CHL (Figure 18). pBN-TRI_CHL was transformed into *B.subtilis* strain BG 6002 a derivative of AK 2200, as described in WO 2003/099843.

One neomycin resistant (neoR) transformant was selected and used for expression of the wheat chlase.

### EXAMPLE 2

### Cloning and expression of a chlorophyllase from Chlamydomonas reinhardtii (green algae) in Bacillus subtilis

A nucleotide sequence (SEQ ID No. 5) encoding a *Chlamydomonas* chloryphyllase (SEQ. ID No. 4, hereinafter chlamy chlase) was expressed in *Bacillus subtilis* with the signal peptide of a *B. subtilis* alkaline protease (aprE) (see Fig.s 19 and 20). For optimal expression in Bacillus, a codon optimized gene construct (CHL_CHL) was ordered at GenScript (GenScript Corporation, Piscataway, NJ 08854, USA).

The construct CHL_CHL contains 20 nucleotides with a BssHII restriction site upstream to the chlamy chlase coding region to allow fusion to the aprE signal sequence and a PacI restriction site following the coding region for cloning into the bacillus expression vector pBNppt.

The construct CHL_CHL was digested with BssHII and PacI and ligated with T4 DNA ligase into BssHII and PacI digested pBNppt.

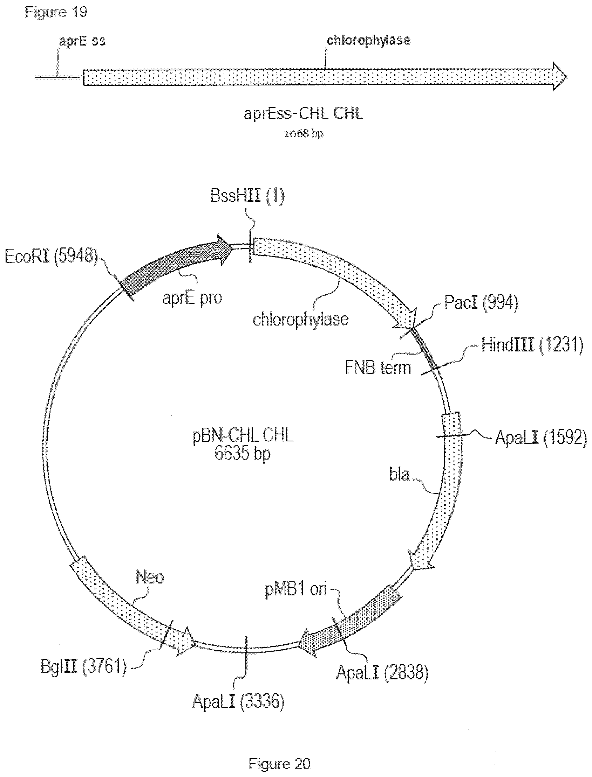

The ligation mixture was transformed into *E. coli* TOP10 cells. The sequence of the BssHII and Pac insert containing the CHL_CHL gene was confirmed by DNA sequencing (DNA Technology A/S, Risskov, Denmark) and one of the correct plasmid clones was designated pBN-CHL_CHL (Figure 20). pBN-CHL_CHL was transformed into *B.subtilis* strain BG 6002 a derivative of AK 2200, as described in WO 2003/099843.

One neomycin resistant (neoR) transformant was selected and used for expression of the chlamy chlase.

### EXAMPLE 3

### Effect of surfactants on chlorophyllase activity in plant oil

Activity of a chlorophyllase from *Arabidopsis thaliana* having the sequence of SEQ ID NO: 1 was tested in refined rapeseed oil with the addition of different surfactants, including soya lecithin. Samples 1 to 6 were prepared comprising the components defined in Table 1:

**Table 1**

| | | 1 | 2 | 3 | 4 | 5 | 6 |
|---|---|---|---|---|---|---|---|
| Refined rapeseed oil | g | 10 | 10 | 10 | 10 | 10 | 10 |
| Soya Lecithin | g | 0.2 | 0.2 | | | | |
| Sorbitan Mono-oleate | g | | | 0.2 | 0.2 | | |
| Sorbitan Tri-oleate | g | | | | | 0.2 | 0.2 |
| Chlorophyll 0.5 mg/ml in acetone | µl | 250 | 250 | 250 | 250 | 250 | 250 |
| Chlorophyllase in Buffer* | ml | 0 | 0.25 | 0 | 0.25 | 0 | 0.25 |
| Buffer* | ml | 0.3 | 0.05 | 0.3 | 0.05 | 0.3 | 0.05 |
| % Water | % | 3.00 | 3.00 | 3.00 | 3.00 | 3.00 | 3.00 |

| | | | | | | | |
|---|---|---|---|---|---|---|---|
| * Buffer: 0.24%Triton X100, 50mM KCL, 100mM Phosphate, pH=7.0 | | | | | | | |

Refined rapeseed oil and surfactant was heated to 45°C with agitation. Chlorophyll, buffer and chlorophyllase were added. The samples were incubated with agitation for 180 minutes and 1 ml sample was taken out and centrifuged for 3 minutes at 3000 rcf.

The oil phase was measured by fluorescence spectroscopy (excitation at 410 nm, emission at 672 nm) and the amount of chlorophyll was quantified (Table 2) from a calibration curve made from measurement of refined rapeseed oil to which a known concentration of chlorophyll had been added.

**Table 2**

| Sample | ppm Chlorophyll |
|---|---|
| 1 | 7.27 |
| 2 | 2.78 |
| 3 | 7.77 |
| 4 | 3.83 |
| 5 | 7.50 |
| 6 | 6.25 |

The results in Table 2 clearly indicate that different surfactants have a strong impact on the activity of chlorophyllase. Lecithin has a strong positive effect on the chlorophyllase activity and thus on the reduction of chlorophyll in oil. Sorbitan monooleate also has a positive effect on the chlorophyllase activity, but the chlorophyllase activity is very modest when sorbitan trioleate is added to the oil.

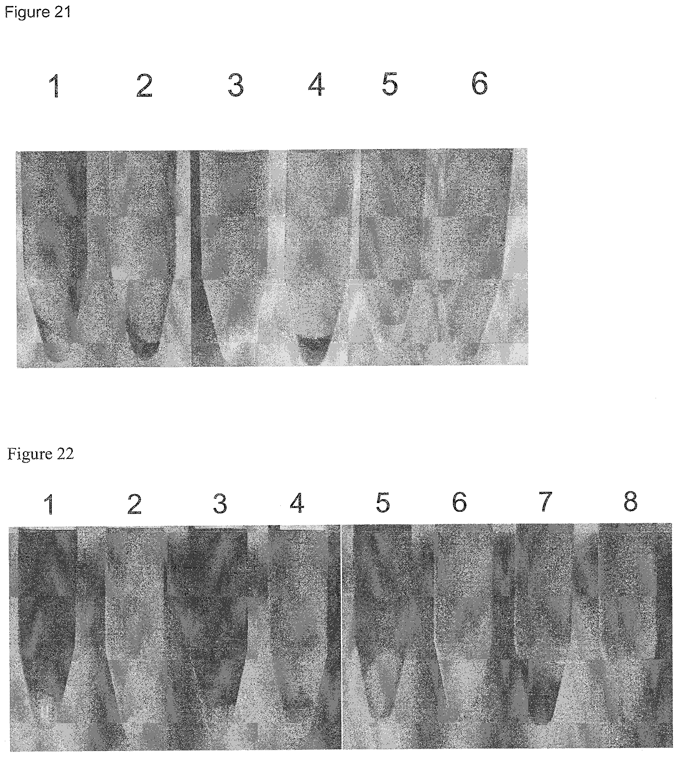

As can be seen from Fig. 21, chlorophyllase is most efficient in combination with lecithin (sample 1). In contrast, chlorophyllase activity is low in combination with sorbitan trioleate (sample 6). It is also observed that sample 2 is more brownish than the other. This may be explained by the fact that some of the phospholipid such as phosphatidic acid in lecithin effectively complexes with magnesium and thus converts chlorophyll into pheophytin (without magnesium). The results suggest that phospholipids (such as are present in lecithin) promote hydrolysis of chlorophyll and chlorophyll derivatives by chlorophyllase.

### EXAMPLE 4

### Effect of refining on chlorophyllase activity in plant oil

As shown in Example 3, surfactants influence chlorophyllase activity on refined oil. At different stages in the oil refining process, the amount of surfactant (particularly lecithin) may vary, thereby influencing chlorophyllase activity.

In the following example chlorophyllase activity was tested in a refined oil and in a combination of refined oil and crude soya oil according to the recipe in Table 3:

**Table 3**

| | | 1 | 2 | 3 | 4 | 5 | 6 | 7 | 8 |
|---|---|---|---|---|---|---|---|---|---|
| Refined oil | | 10 | 10 | 10 | 10 | | | | |
| Refined oil:Crude soya Oil 1:1 | g | | | | | 10 | 10 | 10 | 10 |
| Chlorophyllase (Arabidopsis) | ml | 0 | 0 | 0.25 | 0.25 | 0 | 0 | 0.25 | 0.25 |
| chlorophyll | ml | 0.2 | 0 | 0.2 | 0 | 0.2 | 0 | 0.2 | 0 |
| Extra Water | ml | 0.3 | 0.3 | 0.05 | 0.05 | 0.3 | 0.3 | 0.05 | 0.05 |
| | | | | | | | | | |
| % water | | 3 | 3 | 3 | 3 | 3 | 3 | 3 | 3 |

Oil was heated to 40°C. Chlorophyll, water and enzyme were added and the sample was homogenized with high shear mixing for 20 second and incubated at 40°C with magnetic stirring. After 90 minutes the samples were heated to 97°C for 10 minutes and centrifuged at 1780 rcf for 3 minutes.

The samples were evaluated visually as shown in Fig. 22. It is observed that the chlorophyllase treated sample 3 of refined oil is not very different form sample 1 where no chlorophyllase was added. If the enzyme had hydrolysed chlorophyll, the reaction product chlorophyllide would appear as a green colour in the lower water phase.

In sample 7 (a 1:1 mixture of refined oil:crude soya oil with chlorophyllase treatment) it is very clear that the green colour enters into the water phase and the water phase is very different from sample 5 where no chlorophyllase was added.

The results show that chlorophyllase is only active in hydrolyzing chlorophyll in the oil samples containing crude soya oil. This suggests that surfactants present in the crude soya oil facilitated the chlorophyllase reaction. The crude soya oil contains a high level of surfactant in the form of 2-3% lecithin (principally phospholipids). In the refined oil there are almost no surfactants and therefore no chlorophyllase activity is observed.

### EXAMPLE 5

### Effect of lecithin and acyltransferase on activity of chlorophyllase in oil

Examples 3 and 4 suggest that surfactants like lecithin (which comprises phospholipids) are important for the activity of a chlorophyllase. Lecithin is present at varying levels in some crude plant oils. It is a natural constituent of crude plant oils like soya oil and rapeseed oil, but during the oil refining process lecithin is typically removed from the oil by a degumming process. During the degumming step lecithin may be removed enzymatically by enzymes such as phospholipases. If chlorophyllase activity is dependent on the presence of lecithin, lecithin modification by enzymes will impact on the chlorophyllase activity. It is therefore of importance for efficient chlorophyll removal that the chlorophyllase is used in the presence of a minimum level of lecithin. This may be influenced by the level of lecithin naturally present in the particular oil, a point during the refining process at which chlorophyllase is applied, and the nature of the degumming step.

In the following example a crude oil was water degummed without and with a lipid acyltransferase (LysoMax Oil® from Danisco A/S). LysoMax Oil® is an *Aeromonas salmonicida* mature lipid acyltransferase (GCAT) with a mutation of Asn80Asp, comprising the amino acid sequence of SEQ ID NO:23 (see Fig. 32). This enzyme is known to be very active on phospholipids during formation of lysophospholipids. The isolated gum phase (lecithin or LysoMax Oil® modified lecithin) from the water degumming was isolated and added to a refined oil in different amounts and then combined with 3% water and chlorophyllase in order to investigate the effect of the amount of lecithin and the type of lecithin.

Water degumming was conducted with recipe in shown in Table 4:

**Table 4**

| | | A | B |
|---|---|---|---|
| Crude Rape Seed Oil | g | 150 | 150 |
| LysoMax Oil® 100 U/ml* | ml | 0 | 0.2 |
| Water | ml | 2.250 | 2.050 |
| | | | |
| Enzyme (LysoMax Oil®) U/g oil | | 0.00 | 0.13 |
| % water | | 1.50 | 1.50 |

| | | | |
|---|---|---|---|
| * Lipid acyltransferase activity may be determined as described in WO 2004064987. | | | |

Crude oil was heated to 55°C. Water and enzyme were added and mixed with high shear mixing for 20 seconds followed by incubation with magnetic stirring. After 30 minutes incubation the samples were heated to 97°C for 10 minutes and centrifuged at 1780 rcf for 3 minutes. The oil phase and the gum phase for the two experiments were isolated. The gum phases were dried on a rotary evaporator.

A chlorophyllase from *Triticum* (see Example 1) was tested in a water degumming (WDG) process using the oil and dried gum phase according to recipes in table 5. Oil and dried gum was heated to 55°C with agitation. Water and chlorophyllase were added. The samples were incubated at 65°C for 4 hours. The enzyme was inactivated by heating to 97°C for 10 minutes followed by centrifugation at 1780 rcf for 3 minutes.

**Table 5**

| | | 1 | 2 | 3 | 4 | 5 | 6 | 7 | 8 | 9 | 10 |
|---|---|---|---|---|---|---|---|---|---|---|---|
| Water degummed oil no A | g | 10 | 10 | 10 | 10 | 10 | 10 | 10 | 10 | 10 | |
| Crude Rapeseed Oil (Oct.2009) | g | | | | | | | | | | 10 |
| Lecithin A | g | 0.02 | 0 | 0.05 | | 0.1 | | 0.2 | | | |
| Lecithin B (enzyme modified) | g | | 0.02 | | 0.05 | | 0.1 | | 0.2 | | |
| water | ml | 0.338 | 0.338 | 0.338 | 0.338 | 0.338 | 0.338 | 0.338 | 0.338 | 0.338 | 0.338 |
| TRI CHL CoRe 20 12,5 U/ml | ml | 0.012 | 0.012 | 0.012 | 0.012 | 0.012 | 0.012 | 0.012 | 0.012 | 0.012 | 0.012 |
| Chlorophyllase U/ g oil | | 0.015 | 0.015 | 0.015 | 0.015 | 0.015 | 0.015 | 0.015 | 0.015 | 0.015 | 0.015 |
| % Water | | 3.50 | 3.50 | 3.50 | 3.50 | 3.50 | 3.50 | 3.50 | 3.50 | 3.50 | 3.50 |

The oil phases were isolated and analysed by HPLC with fluorescence detection. The fluorescence signal RFU was calculated based on the same amount of oil with results in table 6 and Fig.s 23 to 26.

**Table 6.**

| Gum A % | Gum B % | Oil | Rel. RFU Pheophorbide | Rel. RFU Pyropheophorbide | Rel. RFU Pheophytin A | Rel. RFU Pyropheophytin |
|---|---|---|---|---|---|---|
| 0.2 | 0 | WDG no A | 12.5 | 1.6 | 1.8 | 1.8 |
| 0 | 0.2 | WDG no A | 6.7 | 1.0 | 7.6 | 2.5 |
| 0.5 | 0 | WDG no A | 12.1 | 1.5 | 1.7 | 1.8 |
| 0 | 0.5 | WDG no A | 6.1 | 1.0 | 8.0 | 2.6 |
| 1 | 0 | WDG no A | 11.5 | 1.8 | 1.3 | 1.8 |
| 0 | 1 | WDG no A | 5.5 | 1.0 | 8.5 | 2.7 |
| 2 | 0 | WDG no A | 11.0 | 1.7 | 1.3 | 1.7 |
| 0 | 2 | WDG no A | 3.7 | 1.0 | 10.0 | 2.7 |
| WDG oil | 0 | WDG no A | 11.0 | 1.5 | 2.9 | 2.3 |
| Crude oil | 0 | Crude Oil | 10.5 | 1.3 | 0.6 | 0.9 |

| | | | | | | |
|---|---|---|---|---|---|---|
| WDG = Water degummed | | | | | | |

For comparison the crude rapeseed oil without chlorophyllase treatment was analysed with the following results:

| Oil | Rel. RFU Pheophorbide | Rel. RFU Pyropheophorbide | Rel. RFU Pheophytin A | Rel. RFU Pyropheophytin |
|---|---|---|---|---|
| Rapeseed oil | 4.5 | 0.9 | 11.9 | 2.4 |

The results from table 6 clearly indicate an effect of lecithin and enzyme modified lecithin on the activity of chlorophyllase. In the sample of crude oil, chlorophyllase is clearly more active than in the sample of water degummed oil (WDG). It is also observed that addition of lecithin to water degummed oil increases the activity of chlorophyllase, and there is a dosage response of adding lecithin from 0.2 to 2% added lecithin. The water degummed oil contains about 0.3% lecithin, which is consistent with the fact that chlorophyllase is somewhat active in the water degummed oil without addition of extra lecithin. As shown in Example 4, chlorophyllase activity is very low in refined rapeseed oil, so the results suggest that the remaining lecithin in water degummed oil has a clear impact on the chlorophyllase activity.

Addition of LysoMax Oil® (lipid acyl transferase from Danisco A/S) modified lecithin to water degummed oil has a strong impact on the chlorophyllase activity. Even a low level of enzyme modified lecithin (0.2%) has a strong negative impact on the activity of the chlorophyllase enzyme, and with addition of 2% enzyme modified lecithin the chlorophyllase activity is almost completely stopped.

When lysolecithin was added to chlorophyllase in an aqueous system comprising Triton X100 as surfactant, no reduction in chlorophyllase activity was observed (results not shown). This aqueous system differs markedly in terms of physical properties with an oil system containing only 3% water. The results suggest that lysophospholipids form different mesomorphic phases and thus prevent the interaction between substrate (e.g. pheophytin) and enzyme (chlorophyllase).

### EXAMPLE 6

### Effect of chlorophyllase and phospholipases in water degumming of rapeseed oil

The results in Example 5 indicate that enzyme modified lecithin has a strong impact on *Triticum* chlorophyllase activity. It was therefore investigated how chlorophyllases work in water degumming in combination with different phospholipases/acyltransferase.

In this experiment chlorophyllase from *Triticum* (see Example 1) and *Chlamydomonas* (see Example 2) were tested in combination with a LysoMax Oil® (lipid acyltransferase from Danisco A/S), a phopholipase A1 (PLA1, Lecitase Ultra®) and a phospholipase C (PLC, Purifine®).

The water degumming was conducted with the recipe shown in table 7.

**Table 7**

| | | 1 | 2 | 3 | 4 | 5 | 6 | 7 | 8 |
|---|---|---|---|---|---|---|---|---|---|
| Crude rapeseed oil | g | 10 | 10 | 10 | 10 | 10 | 10 | 10 | 10 |
| water | ml | 0.310 | 0.300 | 0.290 | 0.300 | 0.085 | 0.075 | 0.065 | 0.075 |
| Triticum CHL'ase, CoRe 20 | ml | 0.040 | 0.040 | 0.040 | 0.040 | | | | |
| Chlamydomonas CHL'ase CoRe31 | ml | | | | | 0.265 | 0.265 | 0.265 | 0.265 |
| LysoMax Oil® 100 U/ml | µl | | 10 | | | | 10 | | |
| Purifine® diluted 1:10 | µl | | | 20 | | | | 20 | |
| Lecitase Ultra® diluted 1:25 | µl | | | | 10 | | | | 10 |
| Chlorophyllase U/ g oil | | 0.050 | 0.050 | 0.050 | 0.050 | 0.050 | 0.050 | 0.050 | 0.050 |
| % Water | | 3.50 | 3.50 | 3.50 | 3.50 | 3.50 | 3.50 | 3.50 | 3.50 |

Crude rapeseed oil was heated to 55°C. Water and enzymes were added and the samples were homogenized with high shear mixing for 20 seconds, followed by agitation with a magnetic stirrer. After 4 hours incubation the samples were heated to 97°C for 10 minutes and centrifuged at 1780 rcf for 3 minutes.

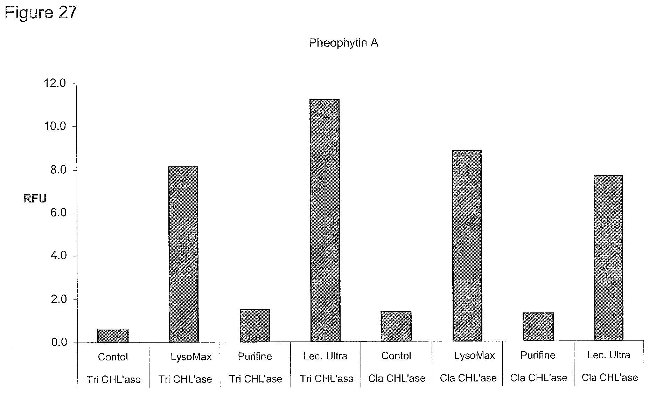

Chlorophyll components of the oil phase were analyzed by HPLC with results in table 8 and Fig. 27.

**Table 8**

| | | Rel. RFU | Rel. RFU | Rel. RFU | Rel. RFU |
|---|---|---|---|---|---|
| Chlorophyllase | Phospholipase | Pheophorbide | Pyropheophorbide | Pheophytin A | Pyropheophytin |
| Tri CHL'ase | - | 9.3 | 2.6 | 0.6 | 1.0 |
| Tri CHL'ase | LysoMax Oil® | 5.0 | 1.3 | 8.1 | 2.7 |
| Tri CHL'ase | Purifine | 10.4 | 2.6 | 1.5 | 1.1 |
| Tri CHL'ase | Lec. Ultra | 2.3 | 0.8 | 11.2 | 3.0 |
| Chla CHL'ase | Control | 7.0 | 2.2 | 1.4 | 1.1 |
| Chla CHL'ase | LysoMax Oil® | 3.3 | 1.2 | 8.8 | 3.5 |
| Chla CHL'ase | Purifine | 7.8 | 2.4 | 1.3 | 0.8 |
| Chla CHL'ase | Lec. Ultra | 3.8 | 1.1 | 7.6 | 2.9 |
| - | - | 1.7 | 0.5 | 11.4 | 1.8 |

The results from table 8 clearly indicate altered activity of chlorophyllase on pheophytin in the presence of phospholipases or an acyltransferase. For comparison, in a control sample comprising no enzyme the pheophytin A in crude rapeseed oil gives a relative fluorescence value of 11.4 RFU.

When chlorophyllases are combined with acyltransferase or PLA1 the amount of pheophytin is much higher than the control comprising chlorophyllase alone, but when chlorophyllases are combined with PLC the level of pheophytin is almost equal with the control oil only treated with chlorophyllase. The experiments indicate that chlorophyllases are inhibited when used in combination with enzymes which during incubation produce lysophospholipids from phospholipids. In contrast, the chlorophyllases retain most of their original activity when used in combination with a PLC, which produces diglyceride from phospholipids.

### EXAMPLE 7

### Chlorophyllase in total degumming

Example 6 shows that chlorophyllase can be used during oil refining in the water degumming process. Depending on the type of oil and depending on the refining process, different types of degumming processes may be used. Thus as an alternative to a water degumming process, a plant oil may be refined without water degumming in a total degumming process or neutralization. In the following example chlorophyllase was used in a total degumming/neutralization process. In this experiment chlorophyllase is also tested in combination with acyltransferase with the recipe shown in table 9:

**Table 9**

| | | 1 | 2 | 3 |
|---|---|---|---|---|
| Crude rapeseed no 6 | g | 10 | 10 | 10 |
| Water | ml | 0.253 | 0.243 | 0.293 |
| TRI_CHL CoRe 20 MRZ | µl | 40.0 | 40.0 | 0.0 |
| Acyltransferase, LysoMax Oil®, 100 U/ml | µl | 0.0 | 10.0 | 0.0 |
| 30% Phos. Acid | µl | 25.00 | 25.00 | 25.00 |
| 4M NaOH | µl | 47 | 47 | 47 |
| | | | | |
| Acyltransferase U/g oil | | 0.0 | 0.1 | 0 |
| Chl'ase Units/ g oil | | 0.050 | 0.050 | 0.000 |
| % Water | | 3.500 | 3.500 | 3.500 |
| Temperature | °C | 55 | 55 | 55 |
| pH | | 6.5 | 6.4 | 6.3 |

Crude rapeseed oil was heated to 55°C. 30% phosphoric acid was added and the sample was homogenized with high shear mixing for 10 second followed by magnetic stirring at 55°C. After 10 minutes water, NaOH and enzymes were added and incubated at 55°C with magnetic stirring. After 4 hours the samples were heated to 97°C for 10 minutes and centrifuged at 1780 rcf for 3 minutes.

The oil phase was analysed by HPLC with results shown in table 10:

**Table 10**

| | | Pheophorbide | Pyropheophorbide | Pheophytin A | Pyropheophytin |
|---|---|---|---|---|---|
| Sample | Enzyme | RFU | RFU | RFU | RFU |
| 1 | CHL'ase | 4.3 | 0.5 | 0.8 | 1.1 |
| 2 | CHL'ase+ LysoMax Oil® | 3.8 | 0.4 | 5.4 | 1.6 |
| 3 | Control | 0.2 | 0.2 | 14.6 | 2.3 |

The results in table 10 confirm high activity of chlorophyllase on pheophytin in a total oil degumming process. In combination with acyltransferase the chlorophyllase activity is significantly reduced.

### EXAMPLE 8

### Oil refining procedure with chlorophyllase, with and without bleaching

In this example a chlorophyllase gene from *Triticum* (called TRI_CHL, see Example 1) expressed in *E. Coli* is used in oil refining of crude rapeseed oil. This enzyme has activity on chlorophyll, pheophytin and pyropheophytin in oil. In the first step the oil is treated with TRI_CHL, where the chlorophyll, pheophytin and pyropheophytin is hydrolysed to chlorophyllide, pheophorbide and pyropheophorbide respectively. After TRI_CHL treatment the oil is further refined without the use of bleaching clay, and the oil is compared with the same oil refined by traditional oil refining using bleaching clay.

### Oil refining procedure with chlorophyllase and without bleaching

Water degumming: 175 g crude rapeseed oil is heated to 65°C during agitation and blanketed with nitrogen. 8.75 Units (0,05 Units/g) of TRI_CHL is added together with 6.125 g (3.5%) water. The reaction mixture is homogenized with high shear mixing for 20 seconds. The sample is incubated at 65°C with agitation and blanketed with nitrogen. After 4 hours reaction time the sample is centrifuged at 3000 rcf for 5 minutes and the water degummed oil is isolated.

Total degumming: 150 gram water degummed oil is dried by heating to 90°C for 20 minutes. The oil is cooled to 80°C and 0.33% phosphoric acid (30% aqueous solution) is added. The sample is homogenized by high shear mixing for 10 second and agitated for 10 minutes while cooling down to 70°C followed by addition of 1.28% 4N NaOH . The sample is agitated for 5 minutes blanketed with nitrogen. The sample is centrifuged at 3000 rcf for 5 minutes.

NaOH wash: The oil is isolated and heated to 90°C. 4% 0.1N NaOH is added and the sample is agitated for 5 minutes. The oil is centrifuged at 3000 rcf for 5 minutes.

Water washes: The isolated oil phase is heated to 50°C and washed with 4% water with agitation for 5 minutes and centrifuged at 300 rcf for 5 minutes. The oil phase is washed once more with 4% water. The isolated oil phase is dried by heating to 100°C under vacuum for 20 minutes.

Deodorization: The oil is deodorized at 240°C and 0.5 mBar for 1 hour with steam stripping. The oil is polished by filtering through a glass Microfiber filter (Whatman GF/C).

### Oil refining reference procedure with bleaching:

Water degumming: 175 g crude rapeseed oil is heated to 65°C during agitation and blanketed with nitrogen. 6.125 g (3.5%) water is added. The reaction mixture is homogenized with high shear mixing for 20 seconds. The sample is incubated at 65°C with agitation and blanketed with nitrogen. After 4 hours reaction time the sample is centrifuged at 3000 rcf for 5 minutes and the water degummed oil is isolated.

Total degumming: 150 gram water degummed oil is dried by heating to 90°C for 20 minutes. The oil is cooled to 80°C and 0.33% phosphoric acid (30% aqueous solution) is added. The sample is homogenized by high shear mixing for 10 second and agitated for 10 minutes while cooling down to 70°C followed by addition of 1.28% 4N NaOH. The sample is agitated for 5 minutes blanketed with nitrogen. The sample is centrifuged at 3000 rcf for 5 minutes.

Bleaching: The oil is added 1% bleaching clay (Tonsil Optimum FF210) and heated at 90°C under vacuum and agitation for 20 minutes. The oil is cooled to 80°C and filtered on a buchner funnel using filter paper. The isolated oil phase is dried by heating to 100°C under vacuum for 20 minutes.

Deodorization: The oil is deodorized at 240°C and 0.5 mBar for 1 hour with steam stripping. The oil is polished by filtering through a glass Microfiber filter (Whatman GF/C).

### Results

Crude rapeseed oil from AarhusKarlshamn was refined according to the method above using either (1) Oil refining procedure with chlorophyllase and without bleaching or (2) Oil refining reference procedure with bleaching.

The oil colour was measured according to LoviBond using Dr. Lange, LICO 200 apparatus. The results are shown in Table 11:

**Table 11**

| Lovibond 5¼ | Yellow | Red |
|---|---|---|
| Refined oil with bleaching | 6.4 | 0.5 |
| Refined oil without bleaching | 16 | 0.2 |
| Bunge specification for refined rapeseed oil (5¼ " Lovibond) | Max 20 | Max 1.5 |
| (http://www.bunge-austria.com/uploads/media/Spec_Rapeseed_Oil_refined_A_01.pdf) | | |

### HPLC/MS analysis:

Oil samples from the different processes in the oil refining were analysd by HPLC/MS and quantified relative to standards of the components with results shown in table 12:

**Table 12**

| | Pheophorbide | Pyropheophorbide | Pheophytin b | Pheophytin a | Pyropheophytin |
|---|---|---|---|---|---|
| | ng/mg | ng/mg | ng/mg | ng/mg | ng/mg |
| **Oil refining with bleaching:** | | | | | |
| After water degumming | 0.656 | 0.490 | 1.283 | 22.147 | 0.890 |
| After total degumming | 0.004 | 0.022 | 1.816 | 20.938 | 1.106 |
| After Bleaching | 0.017 | 0.039 | 0.005 | 0.062 | 0.007 |
| After Deodorization | 0.001 | 0.015 | 0.001 | 0.023 | 0.020 |

| **Oil refining with Chlorophyllase:** | | | | | |
|---|---|---|---|---|---|
| After water degumming | 2.547 | 0.845 | 0.027 | 0.855 | 0.254 |
| After total degumming | 0.034 | 0.033 | 0.037 | 0.963 | 0.254 |
| After NaOH wash | 0.002 | 0.019 | 0.034 | 0.812 | 0.250 |
| 1 . Water wash | 0.002 | 0.026 | 0.034 | 0.786 | 0.243 |
| 2. Water wash | 0.002 | 0.025 | 0.033 | 0.788 | 0.244 |
| After Deodorization | 0.002 | 0.025 | 0.001 | 0.022 | 0.327 |

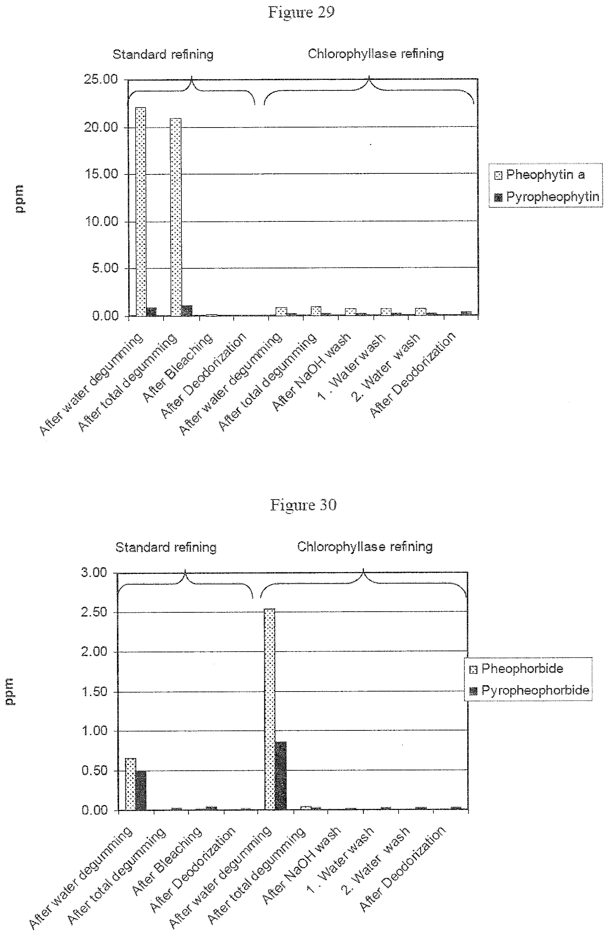

The results from table 12 are illustrated graphically in Figs. 29 and 30.

The results show that cholorophyllase is active on pheophytin and pyropheophytin in the oil during water degumming. More than 95% of pheophytin and more than 70% of pyropheophytin are removed in the chlorophyllase treated oil. These components are hydrolyzed to phytol and pheophorbide and pyropheophorbide respectively. It is observed that after the water degumming process these two components increase in the chlorophyllase treated oil. However a large proportion of these components are washed out by alkaline treatment in the total degumming process. The subsequent washing with NaOH and water only contribute marginally to further removal of the degradation products.

### Materials

In Examples 3 to 8, the following materials were generally used, except where otherwise specified:
Oil: crude extracted rapeseed oil from AarhusKarlshamn

### Enzymes:

Chlorophyllase from *Triticum* expressed in E. coli and purified, Labelled CoRe-20 (see Example 1);
Chlorophyllase from *Clamydomonas* expressed in Bacillus, Labeled CoRe-31 (see Example 2);
Lipid Acyltransferase, LysoMax Oil® from Danisco A/S, e.g. comprising the amino acid sequence of SEQ ID NO:23
Phospholipase C , Purifine® from Verenium Corporation
Phospholipase A1, Lecitase Ultra® from Novozymes A/S

### Emulsifiers:

Sorbitan monooleate, SMO from Danisco A/S
Sorbitan trioleate, STO from Danisco A/S

### HPLC analysis

In Examples 3 to 7, chlorophyll derivatives were in general quantified by HPLC analysis according to the following method. HPLC analysis was performed using a method in general terms as described in "Determination of chlorophylls and carotenoids by high-performance liquid chromatography during olive lactic fermentation", Journal of Chromatography, 585, 1991, 259-266.

The determination of pheophytin, pheophorbide, pyropheophytin and pyropheophorbide is performed by HPLC coupled to a diode array detector. The column employed in the method is packed with C18 material and the chlorophylls were separated by gradient elution. Peaks are assigned using standards of chlorophyll A and B from SigmaAldrich, e.g. based on the representative HPLC chromatogram from Journal of Chromatography, 585, 1991, 259-266 shown in Figure 28.

### EXAMPLE 9

### Chlorophyllase dosage in a water degumming process

Triticum chlorophyllase was tested in different dosages in water degumming of crude rapeseed oil according to the recipe in Table 13. The oil was heated to 65°C, water and enzyme was added. The sample was mixed with a high shear mixer for 20 seconds and incubated with magnetic agitation. Samples were taken out for analysis after ½, 1 and 2 hours, and heated to 97°C for 10 minutes to inactivate the enzyme. The samples were then centrifuged at 10000 rcf for 5 minutes and chlorophyll components in the oil were analysed by HPLC/MS.

**Table 13**

| | | 1 | 2 | 3 | 4 | 5 | 6 |
|---|---|---|---|---|---|---|---|
| Crude rape seed no 8 | g | 10 | 10 | 10 | 10 | 10 | 10 |
| water | ml | 0.350 | 0.348 | 0.345 | 0.338 | 0.315 | 0.232 |
| Triticum chlorophyllase 14 U/ml | ml | | 0.0024 | 0.0047 | 0.0118 | 0.0355 | 0.1183 |
| Units/ g oil | | 0.000 | 0.005 | 0.010 | 0.025 | 0.075 | 0.250 |
| % Water | | 3.500 | 3.500 | 3.500 | 3.500 | 3.500 | 3.500 |
| Temperature | °C | 65 | 65 | 65 | 65 | 65 | 65 |

The HPLC results are shown in Table 14:

| ng/mg = µg/g | | | | | | | | |
|---|---|---|---|---|---|---|---|---|
| ID | Hour | Pheophorbide | Pyropheophorbide | Pheophytin b | Pheophitin a | Pyropheophytin | Chlorophyll b | Chlorophyll a |
| 2516-186-1 | ½ | 0.27 | 023 | 0.48 | 9.41 | 0.60 | 0.63 | 1.12 |
| 2516-186-2 | ½ | 0.79 | 027 | 0.34 | 6.96 | 0.57 | 0.15 | 030 |
| 2516-186-3 | ½ | 0.81 | 026 | 0.30 | 6.25 | 0.57 | 0.12 | 025 |
| 2516-186-4 | ½ | 0.96 | 029 | 0.22 | 5.02 | 0.53 | 0.11 | 022 |
| 2516-186-5 | ½ | 1.09 | 031 | 0.17 | 4.00 | 0.49 | 0.08 | 0.18 |
| 2516-186-6 | ½ | 1.25 | 037 | 0.08 | 2.12 | 0.39 | 0.04 | 0.10 |
| 2516-186-1 | 1 | 0.41 | 026 | 0.48 | 9.47 | 0.63 | 0.34 | 0.66 |
| 2516-186-2 | 1 | 0.98 | 024 | 0.24 | 5.32 | 0.57 | 0.08 | 0.17 |
| 2516-186-3 | 1 | 0.97 | 0.27 | 0.21 | 4.56 | 0.55 | 0.07 | 0.16 |
| 2516-186-4 | 1 | 1.11 | 031 | 0.13 | 3.20 | 0.51 | 0.05 | 0.13 |
| 2516-186-5 | 1 | 1.28 | 0.41 | 0.09 | 2.39 | 0.44 | 0.04 | 0.09 |
| 2516-186-6 | 1 | 1.39 | 0.45 | 0.04 | 1.22 | 0.31 | 0.01 | 0.03 |
| 2516-186-1 | 2 | 0.53 | 025 | 0.45 | 9.06 | 0.66 | 0.14 | 028 |
| 2516-186-2 | 2 | 1.25 | 0.31 | 0.14 | 3.36 | 0.53 | 0.03 | 0.08 |
| 2516-186-3 | 2 | 1.28 | 0.32 | 0.11 | 2.68 | 0.52 | 0.02 | 0.07 |
| 2516-186-4 | 2 | 1.29 | 0.33 | 0.05 | 1.74 | 0.43 | 0.02 | 0.06 |
| 2516-186-5 | 2 | 1.41 | 0.39 | 0.04 | 1.29 | 0.35 | 0.01 | 0.03 |
| 2516-186-6 | 2 | 1.44 | 0.54 | 0.02 | 0.66 | 0.17 | 0.01 | 0.02 |

Each of the four chlorophyll derivatives chlorophyll a and b and pheophytin a and b exist as a pair of epimers determined by the stereochemistry of H and COOCH₃ around the carbon number 13² (numbering according to the IUPAC system). These are denoted *a*/*b* and *a'*/*b'* with the prime (') forms having S-stereochemistry and non-prime forms having R-stereochemistry.

From the results in Table 14, it is clear that the main component in this oil is pheophytin. Pheophytin is often the main green component in oil, because chlorophyll easily loses its magnesium and turns into pheophytin. Because pheophytin is the main component it is chosen to focus on this component for the analysis of the enzymatic degradation.

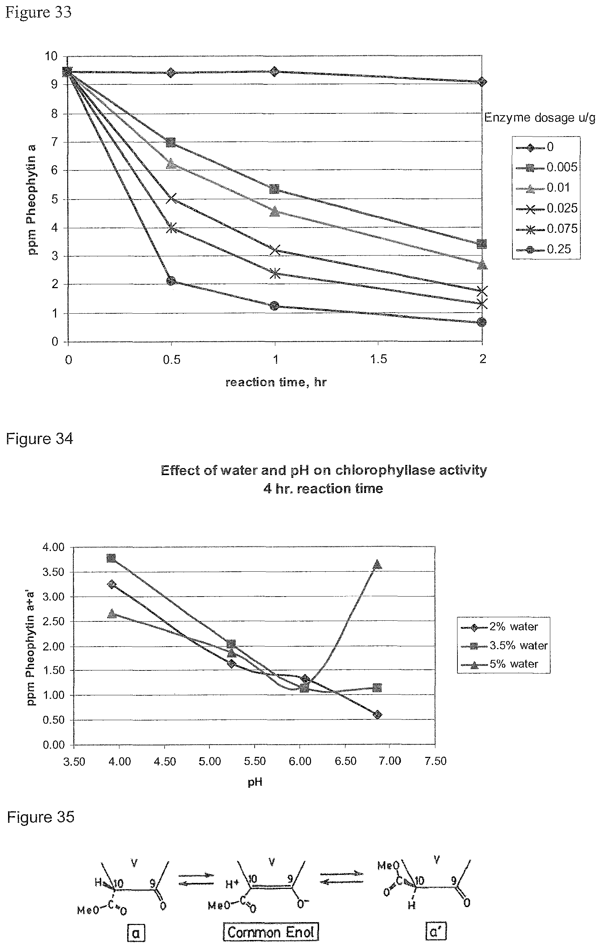

The effect of chlorophyllase as a function of enzyme dosage and reaction time is illustrated in Fig. 33. Initially (½ hr) there is a strong reduction in the amount of pheophytin even at a low enzyme dosage, and then the enzyme activity levels off over time, but the amount of pheophytin still decreases after 2 hour reaction time.

The results in Table 14 confirm the activity of *Triticum* chlorophyllase on degradation of chlorophyll, pheophytin and pyropheophytin. The results from the control sample without enzyme addition however reveals that chlorophyll a and b are thermally degraded, most probably because chlorophyll loses magnesium and is converted to pheophytin.

It is also observed that the amount of pyropheophytin increases as a function of time in the control sample, most probably because some of the pheophytin is converted to pyropheophytin. Formation of pyropheophytin is not preferred because the enzyme activity on this component is much lower than on pheophytin. The reaction product from hydrolysis of pyropheophytin is pyropheophorbide, which is more hydrophobic than pheophorbide and thus more difficult to wash out of the oil.

### EXAMPLE 10

### Effect of pH and water concentration on chlorophyllase activity

In this study *Triticum* chlorophyllase (TRI_CHL) was investigated in a total degumming process with different water dosages and different pH adjustments according to the recipe in Table 15.

**Table 15**

| 2516-190- | | 1 | 2 | 3 | 4 | 5 | 6 | 7 | 8 | 9 | 10 | 11 | 12 | 13 |
|---|---|---|---|---|---|---|---|---|---|---|---|---|---|---|
| Crude rape seed no 6 | g | 10 | 10 | 10 | 10 | 10 | 10 | 10 | 10 | 10 | 10 | 10 | 10 | 10 |
| water | ml | 0.135 | 0.111 | 0.087 | 0.039 | 0.285 | 0.261 | 0.237 | 0.189 | 0.435 | 0.411 | 0.387 | 0.339 | 0.261 |
| TRI_CHL CoRe 52 21, 14 U/ml | µl | 23.7 | 23.7 | 23.7 | 23.7 | 23.7 | 23.7 | 23.7 | 23.7 | 23.7 | 23.7 | 23.7 | 23.7 | 0.0 |
| 1M NaOH | µl | 50.0 | 75.0 | 100.0 | 150.0 | 50.0 | 75.0 | 100.0 | 150.0 | 50.0 | 75.0 | 100.0 | 150.0 | 100.0 |
| Citric acid, 50% solution | µl | 14.0 | 14.0 | 14.0 | 14.0 | 14.0 | 14.0 | 14.0 | 140 | 14.0 | 14.0 | 14.0 | 14.0 | 14.0 |
| | | | | | | | | | | | | | | |
| Units/ g oil | | 0.050 | 0.050 | 0.050 | 0.050 | 0.050 | 0.050 | 0.050 | 0.050 | 0.050 | 0.050 | 0.050 | 0.050 | 0.000 |
| % Water | | 2.000 | 2.000 | 2.000 | 2.000 | 3.500 | 3.500 | 3.500 | 3.500 | 5.000 | 5.000 | 5.000 | 5.000 | 3.500 |
| Temperature | °C | 55 | 55 | 55 | 55 | 55 | 55 | 55 | 55 | 55 | 55 | 55 | 55 | 55 |
| pH | | 3.9 | 5.2 | 6.1 | 6.9 | 4.7 | 5.3 | 6.1 | 7.0 | 5.0 | 5.6 | 5.9 | 6.9 | 6.1 |
| | | | | | | | | | | | | | | |
| µmol NaOH | | 0.05 | 0.075 | 0.1 | 0.15 | 0.05 | 0.075 | 0.1 | 0.15 | 0.05 | 0.075 | 0.1 | 0.15 | 0.1 |
| µmol Citr acid | | 0.033 | 0.033 | 0.033 | 0.033 | 0.033 | 0.033 | 0.033 | 0.033 | 0.033 | 0.033 | 0.033 | 0.033 | 0.033 |
| | | | | | | | | | | | | | | |
| Mol Ratio NaOH/Citr acid | | 1.500 | 2.250 | 3.000 | 4.500 | 1.500 | 2.250 | 3.000 | 4.500 | 1.500 | 2.250 | 3.000 | 4.500 | 3.000 |

The oil was heated to 55°C and citric acid added. The sample was mixed with high shear mixing for 20 seconds followed by agitation with magnetic stirrer for 10 min. NaOH and water and enzyme was added and mixed for 20 seconds with high shear mixing. The samples were incubated with magnetic stirring for 4 hours. Samples were taken out for analysis after ½, 1 and 2 hours, and heated to 97°C for 10 minutes to inactivate the enzyme. The samples were then centrifuged at 10000 rcf for 5 minutes and the oil phase was analysed by HPLC/MS.

The effect of pH and % water on chlorophyllase activity is illustrated in Figure 34. At 2 % water in the reaction mixture the chlorophyllase activity increases with increased pH up to almost pH 7. The same trend is also seen for 3.5 % water but in the experiment with 5% water the enzyme activity increases to pH 6, but between 6 and 7 the activity decreases again.

It is known that both chlorophyll and pheophytin exists in two epimer forms *a* and *a'*(R-isomer and S-isomer), and the chlorophyllase enzyme is only active on the *a*-isomer form. It is known that an equilibrium exists between the two forms and the rearrangement is dependent on pH (Fig. 35).

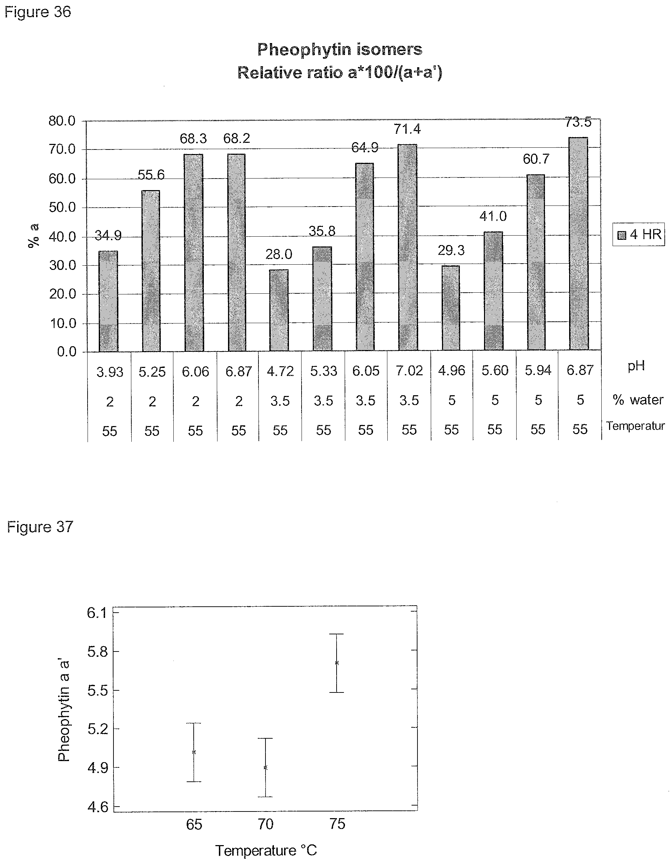

It is thus expected that the enzyme activity is dependent on pH because higher pH will favour the formation of the *a*-isomer. Based on the HPLC analysis of pheophytin *a* and *a'* epimers it is possible to calculate the ratio of the *a*-epimer at different pH as shown in Fig. 36. The results in Fig. 36 confirm that an increase in pH will favour formation of the *a*-epimer which then can explain the increased activity of the enzyme at higher pH. Part of this increase in activity can also be explained by other factors like the enzyme activity at different pH.

### EXAMPLE 11

### Effect of reaction temperature on activity of chlorophyllase

The above mentioned experiments were conducted at 55°C because this is the temperature typically used in water degumming with enzymes (e.g. LysoMax Oil®). Chlorophyllase from *Triticum* (TRI_CHL) is however known to be more heat stable, and in the following experiment the enzyme activity in oil was investigated at 65, 70 and 75°C according to the recipe in Table 16.

**Table 16**

| 2516-194- | | 1 | 2 | 3 | 4 | 5 | 6 | 7 | 8 | 9 | 10 | 11 | 12 |
|---|---|---|---|---|---|---|---|---|---|---|---|---|---|
| Crude rape seed no 8 | | 10 | 10 | 10 | 10 | 10 | 10 | 10 | 10 | 10 | 10 | 10 | 10 |
| water | ml | 0.350 | 0.334 | 0.318 | 0.302 | 0.350 | 0.334 | 0.318 | 0.302 | 0.350 | 0.334 | 0.318 | 0.302 |
| TR CHL CoRe 70-10. 31 U/ml | ml | | 0.0161 | 0.0323 | 0.0484 | 0.0000 | 0.0161 | 0.0323 | 0.0484 | 0.0000 | 0.0161 | 0.0323 | 0.0484 |
| | | | | | | | | | | | | | |
| Units/ g oil | | 0.000 | 0.050 | 0.100 | 0.150 | 0.000 | 0.050 | 0.100 | 0.150 | 0.000 | 0.050 | 0.100 | 0.150 |
| % Water | | 3.50 | 3.50 | 3.50 | 3.50 | 3.50 | 3.53 | 3.53 | 3.50 | 3.50 | 3.50 | 3.50 | 3.50 |
| Temperature | °C | 65 | 65 | 65 | 65 | 70 | 70 | 70 | 70 | 75 | 75 | 75 | 75 |

Oil was heated to set point 65, 70 or 75 °C. Water and enzyme was added and the sample was mixed with high shear mixing for 20 sec. and incubated with agitation at set temperature. Sample were taken out for analysis after ½, 1 and 2 hours, and heated to 97°C for 10 minutes to inactivate the enzyme. The samples were then centrifuged at 10000 rcf for 5 minutes and the oil phase was analysed by HPLC/MS. The results were evaluated statistically using ANOVA Statgraphic software.

The effect of temperature on level of pheophytin is shown in Fig 37. The results confirm that the activity decreases at 75°C but there is not any clear difference in enzyme activity at 65 and 70°C

The results indicate that enzymatic degradation of green colour is not limited to the degradation of pheophytin but also pyropheophytin is an important component. One of the limitations is that *Triticum* chlorophyllase has much lower activity on pyropheophytin compared to activity on pheophytin and therefore more enzyme and/or reaction time is needed to hydrolyze this component. Another aspect is that pheophytin might be converted into pyropheophytin and it could be expected that the temperature has an impact on this.

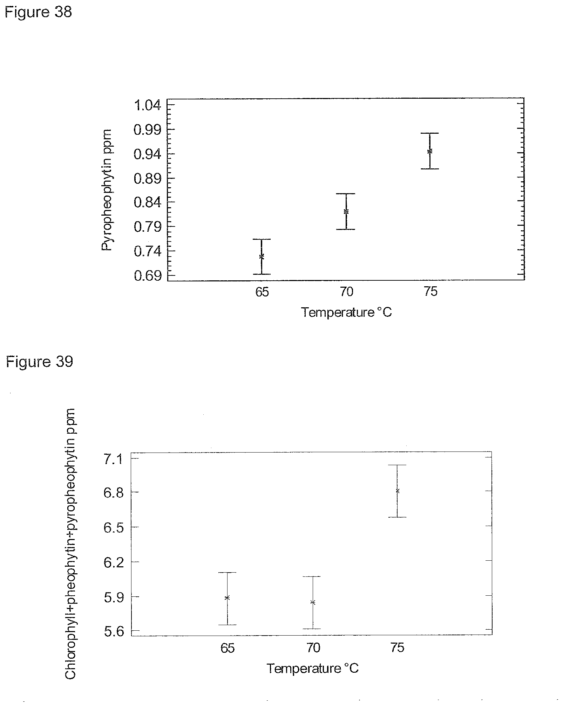

The effect of temperature on level of pyropheophytin is shown in Fig 38. The results clearly indicate that the amount of pyropheophytin is higher in samples incubated at higher temperature. This of course could be explained by lower activity of the enzyme but it was shown that the enzyme activity on pheophytin at 70°C was at least on level with the activity at 65°C. The results therefore indicate that more pyropheophytin is produced at higher temperature.

Taking into account that part of chlorophyll is converted to pheophytin and pheophytin is converted to pyropheophytin, the effect of temperature on the sum of the three components was also investigated (Fig. 39). It is concluded that the enzyme activity on these three components is on the same level at 65 and 70°C, but at 75°C the enzyme activity is lower.

### EXAMPLE 12

### Mixing conditions during chlorophyllase activity in oil/water

The enzymatic reaction of *Triticum* chlorophyllase (TRC_CHL) on chlorophyll in oil is conducted in a two phase oil/water reaction mixture. It could therefore be speculated that the reaction would depend on the water distribution and particle size of the water droplets. In order to investigate this in more details experiments were set up with and without high shear mixing. Also experiments were set up where enzyme and water were mixed before addition to the oil (Table 17). In all experiments 3.5% water was used.

**Table 17**

| | | 1 | 2 | 3 | 4 | 5 | 6 | 7 | 8 |
|---|---|---|---|---|---|---|---|---|---|
| Crude rapeseed oil no 8 | | 10 | 10 | 10 | 10 | 10 | 10 | 10 | 10 |
| water | ml | 0.350 | 0.334 | 0.334 | 0.484 | 0.484 | 0.334 | 0.334 | 0.334 |
| TRI_CHL CoRe 70-10.(31 U/ml) | ml | 0.0161 | 0.0161 | 0.0161 | 0.0161 | 0.0161 | 0.0161 | 0.0161 | 0.0161 |
| | | | | | | | | | |
| Units/g oil | | 0.000 | 0.050 | 0.050 | 0.050 | 0.050 | 0.050 | 0.050 | 0.050 |
| % Water | | 3.500 | 3.500 | 3.500 | 5.000 | 5.000 | 3.500 | 3.500 | 3.500 |
| Temperature | °C | 65 | 65 | 65 | 65 | 65 | 65 | 65 | 65 |
| | | | | | | | | | |
| Ultra Turrax 20 sec. | | + | - | + | - | + | + | - | + |
| | | | | | | | | | |
| Interval Ultra Turrax: 5 sec every 10. min | | | | | | | + | | |
| | | | | | | | | | |
| Enzyme+water mixed before addition | | | | | | | | + | + |

| | | | | | | | | | |
|---|---|---|---|---|---|---|---|---|---|
| Oil was heated to 65°C with magnetic stirring. Enzyme and water was added. The samples were treated with high shear mixing according to Table 17 and incubated with magnetic stirring. 1 ml samples were taken out after ½, 1 and 2 hours reaction time. The samples were then centrifuged at 10000 rcf for 5 minutes and the oil phase was analysed by HPLC/MS. | | | | | | | | | |

The results of pheophytin content are illustrated graphically in Fig .40, which shows that there is not much difference in the level of pheophytin in samples treated with or without high shear mixing (Ultra Turrax). This indicates that less strong mixing with a magnetic stirrer is sufficient to obtain a good enzyme reaction and this can not be improved by initially high shear mixing which produces much finer water droplets in the reaction mixture.

### EXAMPLE 13

### Effect of pH in water degumming

Experiments shown above (Example 10) indicated that the activity of chlorophyllase and also the rearrangement of pheophytin *a'* to pheophytin *a*, was dependent on pH. In the following experiment, the water degumming process was conducted both without pH adjustment and with pH adjustment with NaOH or with citrate buffer. In one experiment an acyltransferase was also tested in combination with chlorophyllase. The oil was heated to 65°C and water NaOH/buffer and enzyme was added.

The samples were mixed with Ultra Turrax for 20 sec. and incubated at 65°C with magnetic stirring. Samples were taken out after ½, 1, 2 and 4 hours reaction time. The samples were heated to 95°C for 10 minutes to inactivate the enzyme, and centrifuged at 10000 rcf for 5 minutes and the oil phase analysed by HPLC/MS.

**Table 18**

| | | 1 | 2 | 3 | 4 | 5 | 6 | 7 | 8 | 9 |
|---|---|---|---|---|---|---|---|---|---|---|
| Crude rape seed no 8 | | 10 | 10 | 10 | 10 | 10 | 10 | 10 | 10 | 10 |
| water | ml | 0.200 | 0.186 | 0.180 | 0.166 | 0.170 | 0.156 | 0.050 | 0.036 | 0.146 |
| 1N NaOH | ml | | | 0.020 | 0.020 | 0.030 | 0.030 | | | 0.030 |
| 100 mM Citrate pH 6 | ml | | | | | | | 0.150 | 0.150 | |
| LysoMax Oil® | ml | | | | | | | | | 0.010 |
| TRI_CHL CoRe 70_11 (69,77 U/ml | ml | 0 | 0.0143 | 0.0000 | 0.0143 | 0.0000 | 0.0143 | 0.0000 | 0.0143 | 0.0143 |
| | | | | | | | | | | |
| Units/ g oil | | 0.000 | 0.100 | 0.000 | 0.100 | 0.000 | 0.100 | 0.000 | 0.100 | 0.100 |
| % Water | | 2.00 | 2.00 | 2.00 | 2.00 | 2.00 | 2.00 | 2.00 | 2.00 | 2.00 |
| Temperature | °C | 65 | 65 | 65 | 65 | 65 | 65 | 65 | 65 | 65 |
| | | | | | | | | | | |
| pH | | 5.21 | 5.17 | 7.21 | 7.12 | 7.50 | 7.48 | 5.50 | 5.40 | 7.43 |

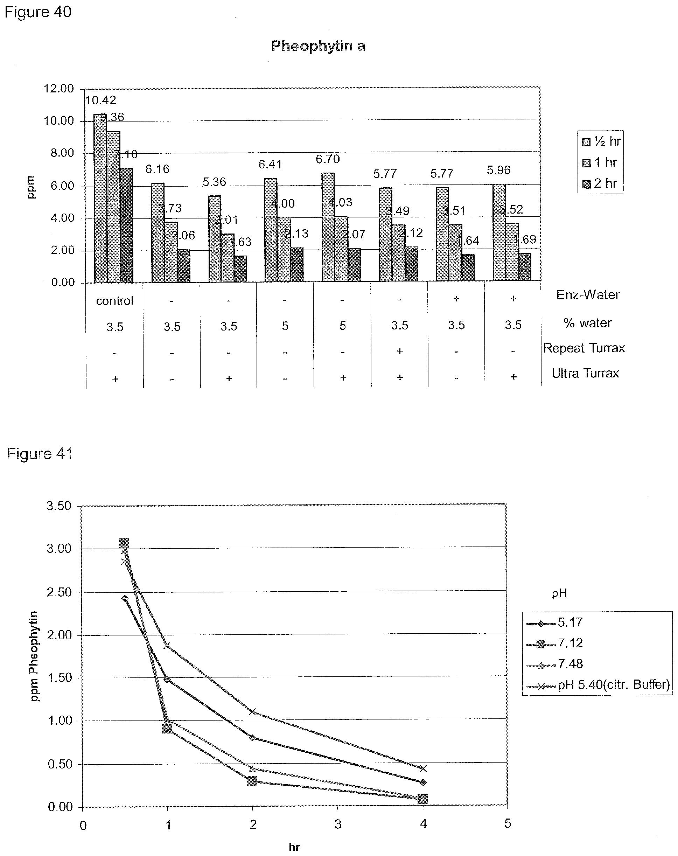

In Fig. 41, the amount of pheophytin (*a*+*a'*) is illustrated as a function of time and different pH conditions. It is observed that increasing the pH to more than 7 with addition of NaOH will increase the enzyme activity on pheophytin. This is probably explained by the fact that the rearrangement of pheophytin *a'* to pheophytin *a* is dependent on the pH.

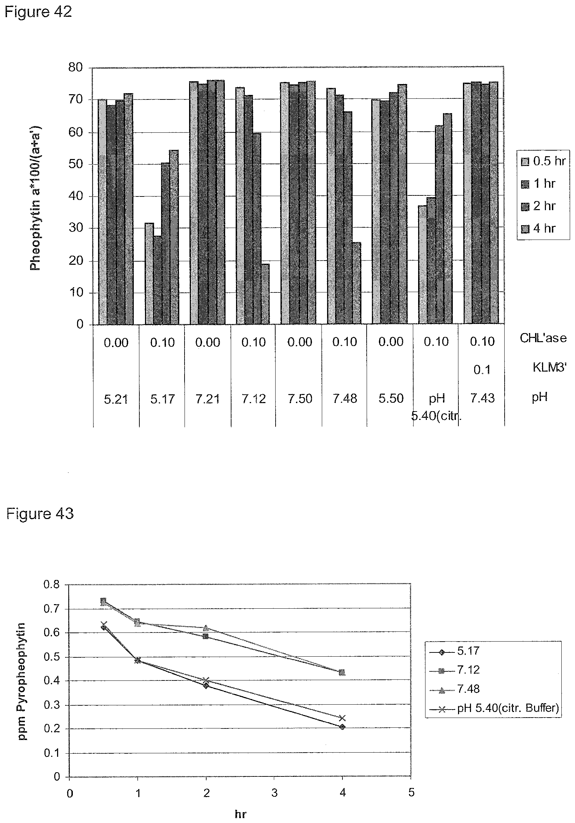

In Fig. 42 the ratio of the *a* epimer is illustrated, and it is very clear that at pH 5.12 and 5.4 the amount of the *a*-epimer is low because the chlorophyllase only is active on this epimer and the rearrangement is slow because of lower pH. At pH 7.12 and 7.48 the amount of the *a-*epimer is almost kept at the equilibrium concentration (approx. 70%) during the whole process. Only after 4 hours reaction time, the relative amount of the *a*-epimer is going down, probably because the total amount of pheophytin then is very low.

Although it is very important that the enzyme is active on pheophytin, it is also important for the green colour that the process can remove the other colour components including pyropheophytin. Fig. 43 illustrates the amount of pyropheophytin in the sample treated with *Triticum* chlorophyllase at different pH. It can be concluded that lower pH has a positive effect on removal of pyropheophytin. This can be explained by lower enzyme activity on pyropheophytin at higher pH, or by formation of pyropheophytin, which is catalyzed by higher pH.

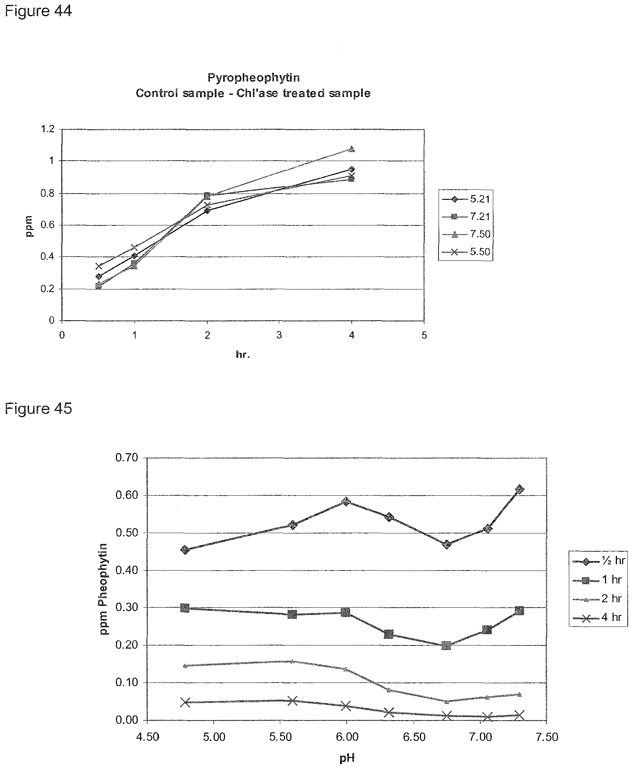

In Fig 44 the amount of pyropheophytin is subtracted from the amount of pyropheophytin in the control sample, where no chlorophyllase is added. This graph indicates that change in pyropheophytin caused by the enzyme is almost the same at different pHs. It is therefore concluded that higher pH promotes the formation of pyropheophytin from pheophytin, which explains the results in Fig 44.

### EXAMPLE 14

### Effect of pH on chlorophyllase activity in water degumming of oil.

The results reported above indicate the effect of pH with regard to the enzyme activity and the rearrangement of pheophytin *a'* to pheophytin *a.* Higher pH in the process also seems to have an impact on the conversion of pheophytin to pyropheophytin. In this study the pH was further investigated by narrowing the range of pH for the water degumming trials with *Triticum* chlorophyllase (TRI_CHL). The experiments were conducted according to Table 19.

**Table 19**

| | | 1 | 2 | 3 | 4 | 5 | 6 | 7 | 8 |
|---|---|---|---|---|---|---|---|---|---|
| Crude rape seed no 8 | | 10 | 10 | 10 | 10 | 10 | 10 | 10 | 10 |
| water | ml | 0.200 | 0.182 | 0.172 | 0.157 | 0.142 | 0.117 | 0.102 | 0.082 |
| 1 N NaOH | ml | | | 0.010 | 0.025 | 0.040 | 0.065 | 0.080 | 0.100 |
| TRI_CHL CoRe 70_11 (54,19 U/ml) | ml | 0 | 0.0185 | 0.0185 | 0.0185 | 0.0185 | 0.0185 | 0.0185 | 0.0185 |
| | | | | | | | | | |
| Units/ g oil | | 0.000 | 0.100 | 0.100 | 0.100 | 0.100 | 0.100 | 0.100 | 0.100 |
| % Water | | 2.000 | 2.000 | 2.000 | 2.000 | 2.000 | 2.000 | 2.000 | 2.000 |
| Temperature | °C | 65 | 65 | 65 | 65 | 65 | 65 | 65 | 65 |
| | | | | | | | | | |
| pH | | 5.03 | 4.78 | 5.60 | 5.99 | 6.32 | 6.75 | 7.06 | 7.30 |

| | | | | | | | | | |
|---|---|---|---|---|---|---|---|---|---|
| The oil was heated to 65°C and water, NaOH and enzyme was added. The samples were mixed with Ultra Turrax for 20 sec. and incubated at 65°C with magnetic stirring. Samples were taken out after ½, 1, 2 and 4 hours reaction time. The samples were heated to 95°C for 10 minutes to inactivate the enzyme, and centrifuged at 10000 rcf for 5 minutes and the oil phase was analysed by HPLC/MS. | | | | | | | | | |

The effect of adding NaOH and adjusting the pH has an impact on the ability of the enzyme to degrade pheophytin (see Fig. 45). At pH 4.5 to pH 6 the enzyme activity seems to be on the same level. There is even a tendency to decreased enzyme activity going from ph 4.5 to pH 6 after ½ hour reaction time, but this levels out at prolonged reaction time. Above pH 6 there is a clear reduction in the level of pheophytin indicating increased enzyme activity, and the optimum pH for the reaction is between pH 6.3 to 6.8.

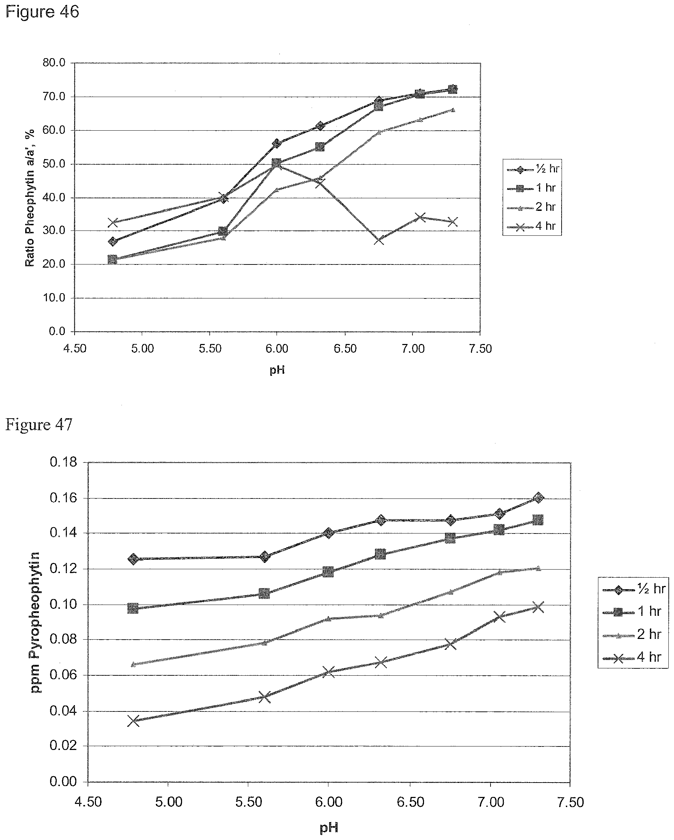

A possible explanation for the effect of pH on activity of chlorophyllase is the fact that the enzyme is only active on pheophytin *a* epimer (R-isomer) and not on pheophytin *a'* epimer (the S-isomer). The graphs in Fig. 46 illustrated the effect of pH on the relative amount of pheophytin *a* epimer. It is very clear that increasing the pH from 4.5 to 7 will result in higher amount of the *a* epimer, going from about 20% up to 70% of *a* epimer, which is the equilibrium concentration. The change in the ratio of the epimer *a* is explained by the fact that lower pH (higher concentration of H⁺) prevents the rearrangement from moving towards the equilibrium.

*Triticum* chlorophyllase has much lower activity on pyropheophytin than on pheophytin. In the reaction of chlorophyllase with oil it is therefore also important that the process is optimized to produce as low as possible amount of pyropheophytin. In Fig. 47 the amount of pyropheophytin as a function of pH is illustrated.

The results clearly show a decrease in pyropheophytin as a function of reaction time, but it is also observed that sample with higher pH contain more pyropheophytin. This can be explained by the conversion of pheophytin to pyropheophytin, which is promoted by higher pH.

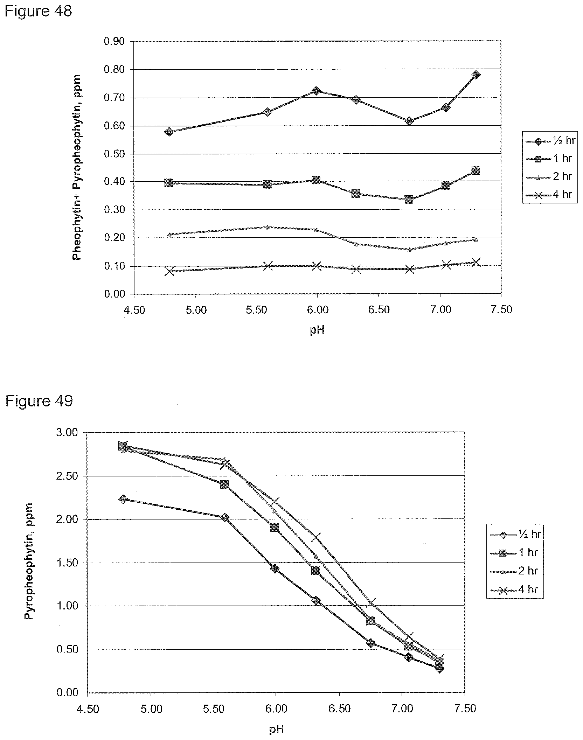

In the selection of optimal pH condition for the enzymatic degradation of chlorophyll components it is important to not only look at the enzyme kinetics but also look at the different epimers of pheophytin and the conversion of pheophytin to pyropheophytin. In Fig. 48 the effect of chlorophyllase on the amount of pheophytin + pyropheophytin is illustrated as a function of pH.

The results in Fig. 48 confirm that pH 6.3 to 6.8 is the best range for the process. But it is also seen that after 4 hours reaction time there is no strong effect of pH on the degradation of pheophytin plus pyropheophytin.

During sample preparation of samples for HPLC/MS analysis the samples were centrifuged and the oil phase was analysed. The reaction products for the hydrolysis of pheophytin will then be distributed between the oil and the water phase. The residual amount of pheophorbide in the oil phase as a function of pH is illustrated in Fig. 49. The graphs in Fig. 49 confirm that the amount of pheophorbide dramatically goes down when the pH increases. This is explained by the fact that increased pH converts pheophorbide into its ionized salt form, which is much more water soluble.

### EXAMPLE 15

### Effect of pH on chlorophyllase activity in total degumming process

In the examples above, it was observed that it could be beneficial to adjust the pH in the water degumming process when using *Triticum* chlorophyllase (TRI_CHL). In the total degumming process, acid and alkali are always added during the process. In the following experiments the effect of pH on *Triticum* chlorophyllase activity in the total degumming process was investigated by first treating the oil with citric acid followed by addition of different amounts of NaOH according to Table 20.

**Table 20**

| 2516-208- | | 1 | 2 | 3 | 4 | 5 | 6 | 7 | 8 |
|---|---|---|---|---|---|---|---|---|---|
| Crude rape seed no 8 | | 10 | 10 | 10 | 10 | 10 | 10 | 10 | 10 |
| water | ml | 0.036 | 0.097 | 0.072 | 0.052 | 0.037 | 0.018 | 0.000 | 0.000 |
| Citric acid, 50% solution | ml | 0.020 | 0.020 | 0.020 | 0.020 | 0.020 | 0.020 | 0.020 | 0.020 |
| 1 N NaOH | ml | 0.160 | 0.078 | 0.104 | 0.125 | 0.140 | 0.160 | 0.180 | 0.198 |
| TRI_CHL CoRe 70_11 (54,19 U/ml) | ml | 0 | 0.0185 | 0.0185 | 0.0185 | 0.0185 | 0.0185 | 0.0185 | 0.0185 |
| Units/ g oil | | 0.000 | 0.100 | 0.100 | 0.100 | 0.100 | 0.100 | 0.100 | 0.100 |
| % Water | | 2.00 | 2.00 | 2.00 | 2.00 | 2.00 | 2.00 | 2.00 | 2.00 |
| Temperature | °C | 65 | 65 | 65 | 65 | 65 | 65 | 65 | 65 |
| pH | | 6.34 | 3.99 | 4.30 | 5.20 | 5.87 | 6.16 | 6.54 | 6.59 |

| | | | | | | | | | |
|---|---|---|---|---|---|---|---|---|---|
| The oil was heated to 65°C and citric acid was added. The samples were mixed with Ultra Turrax for 20 sec. and incubated at 65°C with magnetic stirring for 10 minutes. NaOH, water and enzyme was added, and the samples were mixed with Ultra Turrax for 20 seconds, followed by incubation at 65°C with magnetic stirring. Samples were taken out after ½, 1 and 2 hours reaction time. The samples were heated to 95°C for 10 minutes to inactivate the enzyme, and centrifuged at 10000 rcf for 5 minutes and the oil phase analysed by HPLC/MS. | | | | | | | | | |

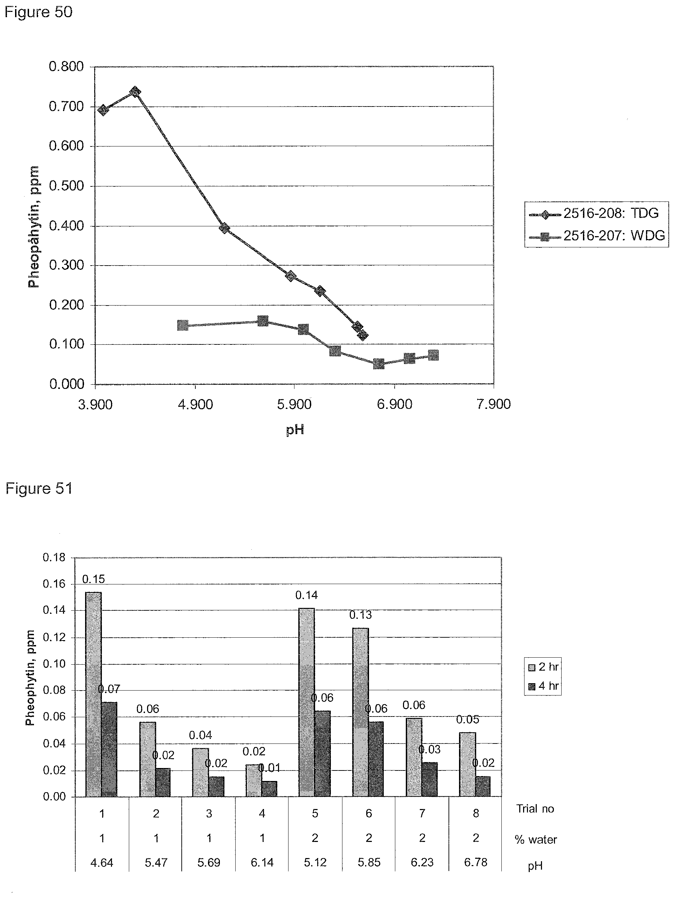

In this total degumming example, the same trend is observed as in the water degumming process. That is, *Triticum* chlorophyllase is more active on pheophytin when increasing the pH from 4 to 6.6, which again is linked to the amount of the two epimer forms of pheophytin. The experiments mentioned above with water degumming was conducted with the same enzyme dosage and oil as in this total degumming process, and therefore the results of pheophytin degradation after 2 hr. reaction time as a function of pH in the two processes were compared as shown in Fig. 50. The graphs in Fig. 50 indicate that the effect of pH adjustment is even stonger in total degumming than in water degumming. This could be explained by the addition of citric acid which increases the ionic strength in the water phase and also has an impact on hydration of phospholipids.

### EXAMPLE 16

### Effect of water content and pH on chlorophyllase activity in oil

Earlier studies had shown that *Triticum* chlorophyllase (TRI_CHL) needed at least 1.5 % water in oil for good activity on pheophytin. Under certain conditions it could however be preferred to use a lower water content. In the following experiments the water degumming process was conducted with 1 and 2 % water and with different NaOH addition as shown in Table 21.

**Table 21**

| | | 1 | 2 | 3 | 4 | 5 | 6 | 7 | 8 | 9 | 10 | 11 | 12 |
|---|---|---|---|---|---|---|---|---|---|---|---|---|---|
| Crude rape seed no 8 | | 10 | 10 | 10 | 10 | 10 | 10 | 10 | 10 | 10 | 10 | 10 | 10 |
| water | ml | 0.082 | 0.057 | 0.042 | 0.017 | 0.182 | 0.157 | 0.142 | 0.117 | 0.100 | 0.035 | 0.200 | 0.135 |
| 1 N NaOH | ml | | 0.025 | 0.040 | 0.065 | | 0.025 | 0.040 | 0.065 | | 0.065 | | 0.065 |
| TRI_CHL CoRe 70_11 (54,19 U/ml) | ml | 0.0185 | 0.0185 | 0.0185 | 0.0185 | 0.0185 | 0.0185 | 0.0185 | 0.0185 | 0.0000 | 0.0000 | 0.0000 | 0.0000 |
| | | | | | | | | | | | | | |
| Units/ g oil | | 0.100 | 0.100 | 0.100 | 0.100 | 0.100 | 0.100 | 0.100 | 0.100 | 0.000 | 0.000 | 0.000 | 0.000 |
| % Water | | 1.000 | 1.000 | 1.000 | 1.000 | 2.000 | 2.000 | 2.000 | 2.000 | 1.000 | 1.000 | 2.000 | 2.000 |
| Temperature | °C | 65 | 65 | 65 | 65 | 65 | 65 | 65 | 65 | 65 | 65 | 65 | 65 |
| | | | | | | | | | | | | | |
| pH | | 4.64 | 5.47 | 5.69 | 6.14 | 5.12 | 5.85 | 6.23 | 6.78 | 4.70 | 6.35 | 4.93 | 6.79 |

| | | | | | | | | | | | | | |
|---|---|---|---|---|---|---|---|---|---|---|---|---|---|
| The process was conducted according to standard conditions mentioned in Example 14 and samples taken out after 2 and 4 hours were analysed by HPLC/MS. | | | | | | | | | | | | | |

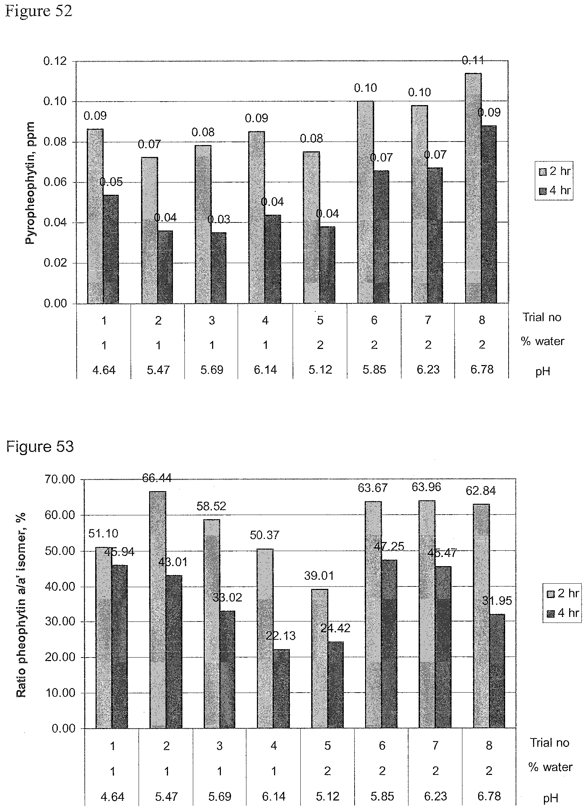

The effect of pH and water contents on the amount of pheophytin in chlorophyllase treated oil is illustrated in Fig. 51 and Fig. 52. The results clearly show that the *Triticum* chlorophyllase is active on pheophytin in a process with 1% water and the activity is better at 1% water compared with 2% water. The higher activity on pheophytin in 1% water can not be explained by higher conversion to pyropheophytin (Fig. 52) or explained by the rearrangement of the *a*'-epimer to the *a*-epimer (Fig. 53). It could therefore be speculated that the improved activity at 1% water is explained by different physical properties (mesomorphic properties) of the polar lipids at 1% water compared with 2% water, which in turn could change the enzyme activity or substrate accessibility.

### EXAMPLE 17

### Optimizing temperature for chlorophyllase treatment of oil

Studies have shown that *Triticum* chlorophyllase is active in oil at temperatures above 70°C. At 70°C the enzyme has its maximum activity but at this temperature the conversion of pheophytin to pyropheophytin increases significantly. Earlier studies concluded that reaction temperature of 65°C was better than 70 °C. In this study the reaction temperature was further investigated by running the water degumming and enzyme reaction process at 60 and 65°C with variation in enzyme dosage and pH adjustment according to experiments shown in Tables 22a and 22b.

**Table 22a**

| 2610-018- | | 1 | 2 | 3 | 4 | 5 | 6 | 7 | 8 |
|---|---|---|---|---|---|---|---|---|---|
| Crude rape seed no 8 | | 10 | 10 | 10 | 10 | 10 | 10 | 10 | 10 |
| water | ml | 0.200 | 0.190 | 0.170 | 0.100 | 0.152 | 0.142 | 0.122 | 0.052 |
| 1N NaOH | | | | | | 0.050 | 0.050 | 0.050 | 0.050 |
| TRI_CHL CoRe 70_11 | ml | | 0.0100 | 0.0300 | 0.1000 | | 0.0100 | 0.0300 | 0.1000 |
| | | | | | | | | | |
| Units/ g oil | | 0.000 | 0.005 | 0.015 | 0.050 | 0.000 | 0.005 | 0.015 | 0.050 |
| % Water | | 2.000 | 2.000 | 2.000 | 2.000 | 2.000 | 2.000 | 2.000 | 2.000 |
| Temperature | °C | 65 | 65 | 65 | 65 | 65 | 65 | 65 | 65 |

**Table 22b**

| 2610-018- | | 9 | 10 | 11 | 12 | 13 | 14 | 15 | 16 |
|---|---|---|---|---|---|---|---|---|---|
| Crude rape seed no 8 | | 10 | 10 | 10 | 10 | 10 | 10 | 10 | 10 |
| water | ml | 0.200 | 0.190 | 0.170 | 0.100 | 0.152 | 0.142 | 0.122 | 0.052 |
| 1N NaOH | | | | | | 0.050 | 0.050 | 0.050 | 0.050 |
| TRI_CHL CoRe 70_11 | ml | | 0.0100 | 0.0300 | 0.1000 | | 0.0100 | 0.0300 | 0.1000 |
| | | | | | | | | | |
| Units/ g oil | | 0.000 | 0.005 | 0.015 | 0.050 | 0.000 | 0.005 | 0.015 | 0.050 |
| % Water | | 2.000 | 2.000 | 2.000 | 2.000 | 2.000 | 2.000 | 2.000 | 2.000 |
| Temperature | °C | 60 | 60 | 60 | 60 | 60 | 60 | 60 | 60 |

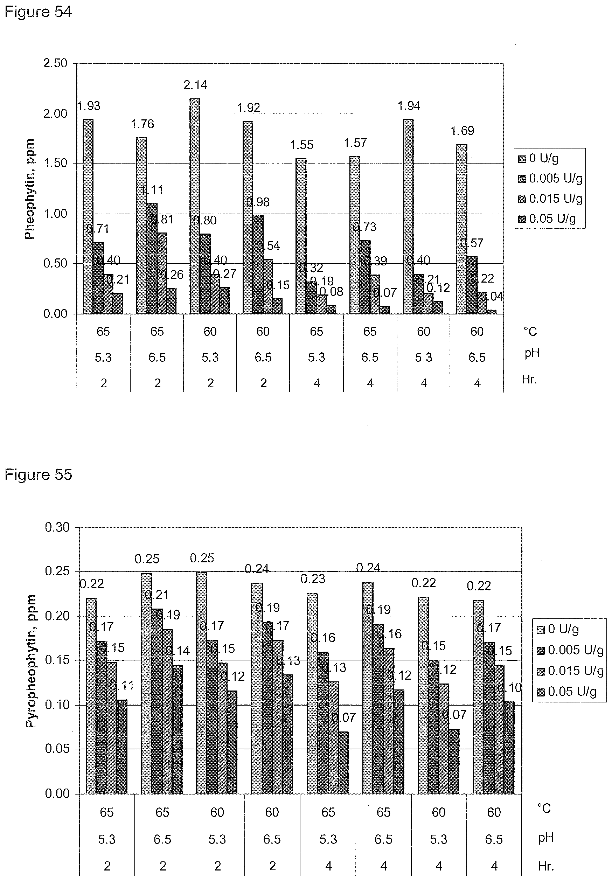

The oil was heated to 65°C/60°C. NaOH, water and enzyme was added, and the samples were mixed with Ultra Turrax for 20 seconds, followed by incubation with magnetic stirring. Samples were taken out after 2 and 4 hours reaction time. The samples were heated to 95°C for 10 minutes to inactivate the enzyme, and centrifuged at 10000 rcf for 5 minutes and the oil phase analysed by HPLC/MS. The results are graphically illustrated in Fig. 54 to 56.

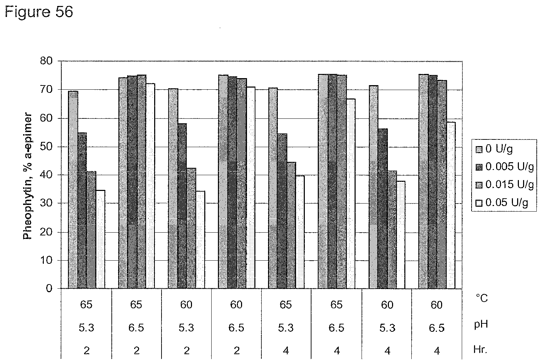

The results in Fig. 54 confirms that the activity of chlorophyllase is correlated to the enzyme dosage, but even a dosage of 0.005 U/g enzyme has a significant effect on pheophytin. It is also observed that the activity at 60°C is at least on level with the activity at 65°C. pH adjustment in the process also seem to have a positive effect on enzyme activity, which is most likely explained by change in epimer re-arrangement when pH is raised (Fig. 56).

There are also some indication that the level of pyropheophytin is lower at 60°C (Fig. 55). The results altogether indicated that 60°C is a preferred reaction temperature for *Triticum* cholorophyllase when used in a water degumming process.

### Conclusion

*Triticum* chlorophyllase was shown to be active on the three main chlorophyll components, chlorophyll, pheophytin and pyropheophytin in an oil system. The enzyme activity was dependent of a number of process parameters including temperature, pH, mixing, %water and reaction time.

The experiments showed that pH 6.3 to 6.5 was the best range for the activity of *Triticum* chlorophyllase because the enzyme is only active on the pheophytin *a* epimer. At pH 6.3-6.5 the rearrangement of epimer *a'* to epimer *a* can take place in the process. At higher pH even stronger rearrangement of pheophytin *a'* to *a* is observed, but it is also observed that at pH higher than 6.5 more pyropheophytin is produced from pheophytin. This is not preferred because *Triticum* chlorophyllase is less active on pyropheophytin.

The conversion of pheophytin to pyropheophytin was also found to be dependent on temperature. At 70°C and above significantly more pyropheophytin is produced from pheophytin and this is not preferred. The experiments showed that the optimum temperature for *Triticum* chlorophyllase was 60°C which is the best compromise between enzyme kinetic and conversion of pheophytin to pyropheophytin.

In a normal water degumming process, typically 2% water is used and experiments showed that *Triticum* chlorophyllase was very active at 2% water. It was found that the enzyme was even more active at 1% water. This lower water concentration could in some instances be advantageous, if the viscosity of the gum phase coming out of this process is not too high for proper handling and pumping.

## Claims

1. A process for refining a plant oil, comprising a step of contacting the oil with an enzyme which is capable of hydrolysing chlorophyll or a chlorophyll derivative, wherein the enzyme is contacted with the oil in the presence of at least 1% by weight phospholipid, and wherein the enzyme is contacted with the oil in the presence of less than 0.2% by weight lysophosholipid wherein the enzyme is contacted with the oil before or during a step of degumming of the oil.

2. A process according to claim 1, wherein the process comprises (a) contacting the enzyme with the oil before (b) degumming the oil using a phospholipase or an acyltransferase.

3. A process according to claim 1, wherein the process comprises contacting the oil with the enzyme and a phospholipase C in a single step.

4. A process according to any preceding claim, wherein the enzyme is contacted with the oil at a temperature of less than 70°C.

5. A process according to any preceding claim, wherein the enzyme is contacted with the oil in the presence of 1 to 5% by weight water.

6. A process according to any preceding claim, wherein the degumming step comprises water degumming.

7. A process according to any preceding claim, wherein the degumming step comprises addition of an acid to the oil followed by neutralisation with an alkali.

8. A process according to any preceding claim, wherein the process does not comprise a step of clay treatment.

9. A process according to any preceding claim, wherein the process further comprises performing a deodorisation step to produce a deodorized oil and a distillate.

10. A process according to claim 8 or claim 9, wherein the process produces a level of carotenoids and/or tocopherol in the refined oil and/or distillate which is elevated compared to a process comprising a clay treatment step.

11. A process according to any preceding claim, wherein the enzyme comprises a chlorophyllase, pheophytinase, pyropheophytinase or pheophytin pheophorbide hydrolase.

12. A process according to any preceding claim, wherein the enzyme comprises a polypeptide sequence as defined in any one of SEQ ID NOs: 1, 2, 4, 6 or 8 to 15, or a functional fragment or variant thereof.

13. A process according to claim 12, wherein the enzyme comprises a polypeptide sequence having at least 75% sequence identity to any one of SEQ ID NOs: 1, 2, 4, 6 or 8 to 15 over at least 50 amino acid residues.

14. Use of a process as defined in any of claims 8 to 13, to increase a level of carotenoids and/or tocopherol in a refined oil and/or a distillate obtained by deodorization of the oil.

15. A process according to claim 4, wherein the enzyme is contacted with the oil at a temperature of 58° to 62°C.

16. A process according to claim 5, wherein the enzyme is contacted with the oil in the presence of about 1% by weight water.

17. A process according to any of claims 1 to 13 or 15, wherein the enzyme is contacted with the oil at a pH of 6.3 to 6.5.

## Patentansprüche

1. Verfahren zum Raffinieren eines Pflanzenöls, umfassend einen Schritt zum In-Kontakt-Bringen des Öls mit einem Enzym, das zum Hydrolysieren von Chlorophyll oder einem Chlorophyllderivat fähig ist, wobei das Enzym mit dem Öl in Anwesenheit von zumindest 1 Gew.-% Phospholipid in Kontakt gebracht wird, und wobei das Enzym mit dem Öl in Anwesenheit von weniger als 0,2 Gew.-% Lysophospholipid in Kontakt gebracht wird, wobei das Enzym mit dem Öl vor oder während eines Schritts zum Entschleimen des Öls in Kontakt gebracht wird.

2. Verfahren nach Anspruch 1, wobei das Verfahren (a) das In-Kontakt-Bringen des Enzyms mit dem Öl vor (b) dem Entschleimen des Öls unter Verwendung einer Phospholipase oder einer Acyltransferase umfasst.

3. Verfahren nach Anspruch 1, wobei das Verfahren das In-Kontakt-Bringen des Öls mit dem Enzym und einer Phospholipase C in einem einzigen Schritt umfasst.

4. Verfahren nach einem der vorstehenden Ansprüche, wobei das Enzym mit dem Öl bei einer Temperatur von weniger als 70 °C in Kontakt gebracht wird.

5. Verfahren nach einem der vorstehenden Ansprüche, wobei das Enzym mit dem Öl in Anwesenheit von 1 bis 5 Gew.-% Wasser in Kontakt gebracht wird.

6. Verfahren nach einem der vorstehenden Ansprüche, wobei der Entschleimungsschritt Wasserentschleimung umfasst.

7. Verfahren nach einem der vorstehenden Ansprüche, wobei der Entschleimungsschritt die Zugabe einer Säure zum Öl, gefolgt von Neutralisierung mit einem Alkali, umfasst.

8. Verfahren nach einem der vorstehenden Ansprüche, wobei das Verfahren keinen Schritt zur Bleicherdenbehandlung umfasst.

9. Verfahren nach einem der vorstehenden Ansprüche, wobei das Verfahren weiterhin das Durchführen eines Desodorierungsschritts umfasst, um ein desodoriertes Öl und ein Destillat herzustellen.

10. Verfahren nach Anspruch 8 oder Anspruch 9, wobei das Verfahren einen Gehalt an Carotinoiden und/oder Tocopherol im raffinierten Öl und/oder Destillat herstellt, der im Vergleich zu einem Verfahren, das einen Bleicherdenbehandlungsschritt umfasst, erhöht ist.

11. Verfahren nach einem der vorstehenden Ansprüche, wobei das Enzym eine Chlorophyllase, Phäophytinase, Pyrophäophytinase oder Phäophytin-Phäophorbid-Hydrolase umfasst.

12. Verfahren nach einem der vorstehenden Ansprüche, wobei das Enzym eine Polypeptidsequenz gemäß Definition in einer von SEQ ID NO: 1, 2, 4, 6 oder 8 bis 15, oder ein funktionelles Fragment oder eine funktionelle Variante davon umfasst.

13. Verfahren nach Anspruch 12, wobei das Enzym eine Polypeptidsequenz umfasst, die zumindest 75% Sequenzidentität mit einer von SEQ ID NO: 1, 2, 4, 6 oder 8 bis 15 über zumindest 50 Aminosäurereste aufweist.

14. Verwendung eines Verfahrens gemäß Definition in einem der Ansprüche 8 bis 13, um einen Gehalt an Carotinoiden und/oder Tocopherol in einem raffinierten Öl und/oder einem Destillat, erhalten durch Desodorierung des Öls, zu steigern.

15. Verfahren nach Anspruch 4, wobei das Enzym mit dem Öl bei einer Temperatur von 58 °C bis 62 °C in Kontakt gebracht wird.

16. Verfahren nach Anspruch 5, wobei das Enzym mit dem Öl in Anwesenheit von ungefähr 1 Gew.-% Wasser in Kontakt gebracht wird.

17. Verfahren nach einem der Ansprüche 1 bis 13 oder 15, wobei das Enzym mit dem Öl bei einem pH-Wert von 6,3 bis 6,5 in Kontakt gebracht wird.

## Revendications

1. Procédé de raffinage d'une huile végétale, comprenant une étape de mise en contact de l'huile avec une enzyme qui est capable d'hydrolyse de la chlorophylle ou d'un dérivé de chlorophylle, l'enzyme étant mise en contact avec l'huile en présence d'au moins 1 % en poids de phospholipide, et l'enzyme étant mise en contact avec l'huile en la présence de moins de 0,2 % en poids de lysophospholipide, l'enzyme étant mise en contact avec l'huile avant ou durant une étape de dégommage de l'huile.

2. Procédé selon la revendication 1, le procédé comprenant (a) la mise en contact de l'enzyme avec l'huile avant (b) le dégommage de l'huile en utilisant une phospholipase ou une acyltransférase.

3. Procédé selon la revendication 1, le procédé comprenant la mise en contact de l'huile avec l'enzyme et une phospholipase C en une étape unique.

4. Procédé selon l'une quelconque des revendications précédentes, l'enzyme étant mise en contact avec l'huile à une température inférieure à 70°C.

5. Procédé selon l'une quelconque des revendications précédentes, l'enzyme étant mise en contact avec l'huile en la présence de 1 à 5 % en poids d'eau.

6. Procédé selon l'une quelconque des revendications précédentes, l'étape de dégommage comprenant le dégommage à l'eau.

7. Procédé selon l'une quelconque des revendications précédentes, l'étape de dégommage comprenant l'addition d'un acide à l'huile suivie de la neutralisation avec un agent alcalin.

8. Procédé selon l'une quelconque des revendications précédentes, le procédé ne comprenant pas d'étape de traitement à l'argile.

9. Procédé selon l'une quelconque des revendications précédentes, le procédé comprenant en outre l'exécution d'une étape de désodorisation pour produire une huile désodorisée et un distillat.

10. Procédé selon la revendication 8 ou la revendication 9, le procédé produisant un niveau de caroténoïdes et/ou de tocophérol dans l'huile raffinée et/ou le distillat qui est élevé comparé à un procédé comprenant une étape de traitement à l'argile.

11. Procédé selon l'une quelconque des revendications précédentes, l'enzyme comprenant une chlorophyllase, phéophytinase, pyrophéophytinase ou phéophytine phéophorbide hydrolase.

12. Procédé selon l'une quelconque des revendications précédentes, l'enzyme comprenant une séquence polypeptidique telle que définie selon l'une quelconque de SEQ ID n^{os}: 1, 2, 4, 6 ou 8 à 15, ou son fragment fonctionnel ou variant.

13. Procédé selon la revendication 12, l'enzyme comprenant une séquence polypeptidique ayant au moins 75 % d'identité de séquence vis-à-vis de l'une quelconque de SEQ ID n^{os}: 1, 2, 4, 6 ou 8 à 15 sur au moins 50 résidus d'acides aminés.

14. Utilisation d'un procédé tel que défini selon l'une quelconque des revendications 8 à 13, pour accroître un niveau de caroténoïdes et/ou de tocophérol dans une huile raffinée et/ou un distillat obtenu par désodorisation de l'huile.

15. Procédé selon la revendication 4, l'enzyme étant mise en contact avec l'huile à une température de 58° à 62°C.

16. Procédé selon la revendication 5, l'enzyme étant mise en contact avec l'huile en présence d'environ 1 % en poids d'eau.

17. Procédé selon l'une quelconque des revendications 1 à 13 ou 15, l'enzyme étant mise en contact avec l'huile à un pH de 6,3 à 6,5.


### SEQUENCE LISTING

<110> DUPONT NUTRITION BIOSCIENCES APS
<120> PROCESS
<130> P039711EPP
<140> 11709190.0
   <141> 2011-02-23
<150> EP10156412.8
   <151> 2010-03-12
<150> US 61/313194
   <151> 2010-03-12
<160> 24
<170> PatentIn version 3.5
<210> 1
   <211> 318
   <212> PRT
   <213> Arabidopsis thaliana
<400> 1
   Met Ser Ser Ser Ser Ser Arg Asn Ala Phe Glu Asp Gly Lys Tyr Lys
   1               5                   10                  15
   Ser Asn Leu Leu Thr Leu Asp Ser Ser Ser Arg Cys Cys Lys Ile Thr
               20                  25                  30
   Pro Ser Ser Arg Ala Ser Pro Ser Pro Pro Lys Gln Leu Leu Val Ala
           35                  40                  45
   Thr Pro Val Glu Glu Gly Asp Tyr Pro Val Val Met Leu Leu His Gly
       50                  55                  60
   Tyr Leu Leu Tyr Asn Ser Phe Tyr Ser Gln Leu Met Leu His Val Ser
   65                  70                  75                  80
   Ser His Gly Phe Ile Leu Ile Ala Pro Gln Leu Tyr Ser Ile Ala Gly
                   85                  90                  95
   Pro Asp Thr Met Asp Glu Ile Lys Ser Thr Ala Glu Ile Met Asp Trp
               100                 105                 110
   Leu Ser Val Gly Leu Asn His Phe Leu Pro Ala Gln Val Thr Pro Asn
           115                 120                 125
   Leu Ser Lys Phe Ala Leu Ser Gly His Ser Arg Gly Gly Lys Thr Ala
       130                 135                 140
   Phe Ala Val Ala Leu Lys Lys Phe Gly Tyr Ser Ser Asn Leu Lys Ile
   145                 150                 155                 160
   Ser Thr Leu Ile Gly Ile Asp Pro Val Asp Gly Thr Gly Lys Gly Lys
                   165                 170                 175
   Gln Thr Pro Pro Pro Val Leu Ala Tyr Leu Pro Asn Ser Phe Asp Leu
               180                 185                 190
   Asp Lys Thr Pro Ile Leu Val Ile Gly Ser Gly Leu Gly Glu Thr Ala
           195                 200                 205
   Arg Asn Pro Leu Phe Pro Pro Cys Ala Pro Pro Gly Val Asn His Arg
       210                 215                 220
   Glu Phe Phe Arg Glu Cys Gln Gly Pro Ala Trp His Phe Val Ala Lys
   225                 230                 235                 240
   Asp Tyr Gly His Leu Asp Met Leu Asp Asp Asp Thr Lys Gly Ile Arg
                   245                 250                 255
   Gly Lys Ser Ser Tyr Cys Leu Cys Lys Asn Gly Glu Glu Arg Arg Pro
               260                 265                 270
   Met Arg Arg Phe Val Gly Gly Leu Val Val Ser Phe Leu Lys Ala Tyr
           275                 280                 285
   Leu Glu Gly Asp Asp Arg Glu Leu Val Lys Ile Lys Asp Gly Cys His
       290                 295                 300
   Glu Asp Val Pro Val Glu Ile Gln Glu Phe Glu Val Ile Met
   305                 310                 315
<210> 2
   <211> 319
   <212> PRT
   <213> Triticum aestivum
<400> 2
   Met Ala Ala Ala Ala Pro Ala Glu Thr Met Asn Lys Ser Ala Ala Gly
   1               5                   10                  15
   Ala Glu Val Pro Glu Ala Phe Thr Ser Val Phe Gln Pro Gly Lys Leu
               20                  25                  30
   Ala Val Glu Ala Ile Gln Val Asp Glu Asn Ala Ala Pro Thr Pro Pro
           35                  40                  45
   Ile Pro Val Leu Ile Val Ala Pro Lys Asp Ala Gly Thr Tyr Pro Val
       50                  55                  60
   Ala Met Leu Leu His Gly Phe Phe Leu His Asn His Phe Tyr Glu His
   65                  70                  75                  80
   Leu Leu Arg His Val Ala Ser His Gly Phe Ile Ile Val Ala Pro Gln
                   85                  90                  95
   Phe Ser Ile Ser Ile Ile Pro Ser Gly Asp Ala Glu Asp Ile Ala Ala
               100                 105                 110
   Ala Ala Lys Val Ala Asp Trp Leu Pro Asp Gly Leu Pro Ser Val Leu
           115                 120                 125
   Pro Lys Gly Val Glu Pro Glu Leu Ser Lys Leu Ala Leu Ala Gly His
       130                 135                 140
   Ser Arg Gly Gly His Thr Ala Phe Ser Leu Ala Leu Gly His Ala Lys
   145                 150                 155                 160
   Thr Gln Leu Thr Phe Ser Ala Leu Ile Gly Leu Asp Pro Val Ala Gly
                   165                 170                 175
   Thr Gly Lys Ser Ser Gln Leu Gln Pro Lys Ile Leu Thr Tyr Glu Pro
               180                 185                 190
   Ser Ser Phe Gly Met Ala Met Pro Val Leu Val Ile Gly Thr Gly Leu
           195                 200                 205
   Gly Glu Glu Lys Lys Asn Ile Phe Phe Pro Pro Cys Ala Pro Lys Asp
       210                 215                 220
   Val Asn His Ala Glu Phe Tyr Arg Glu Cys Arg Pro Pro Cys Tyr Tyr
   225                 230                 235                 240
   Phe Val Thr Lys Asp Tyr Gly His Leu Asp Met Leu Asp Asp Asp Ala
                   245                 250                 255
   Pro Lys Phe Ile Thr Cys Val Cys Lys Asp Gly Asn Gly Cys Lys Gly
               260                 265                 270
   Lys Met Arg Arg Cys Val Ala Gly Ile Met Val Ala Phe Leu Asn Ala
           275                 280                 285
   Ala Leu Gly Glu Lys Asp Ala Asp Leu Glu Ala Ile Leu Arg Asp Pro
       290                 295                 300
   Ala Val Ala Pro Thr Thr Leu Asp Pro Val Glu His Arg Val Ala
   305                 310                 315
<210> 3
   <211> 984
   <212> DNA
   <213> Triticum aestivum
<400> 3
   gcgcgcaggc tgctggaaaa atggcagcgg ctgccccggc cgaaacaatg aataaaagcg      60
   cagcgggtgc cgaagttcct gaagcattta cgtctgtgtt tcaaccgggc aaattggctg     120
   tcgaagccat tcaggtagac gaaaacgctg cccctacacc gcctattccg gtcctgatcg     180
   tagcacctaa agatgcggga acgtatccgg tcgcgatgct gcttcatggc tttttcctgc     240
   ataaccattt ttacgaacat ttgttgcgtc atgtcgcgtc ccatggattt atcatcgtag     300
   ctcctcaatt ttcaattagc attatcccgt caggcgacgc ggaagatatc gcagcggctg     360
   ccaaagttgc tgactggctg ccggatggcc ttcctagcgt tttaccgaaa ggcgttgaac     420
   ctgaactttc taaactggct cttgccggac attcccgcgg cggacataca gctttttcat     480
   tagccttggg ccatgcaaaa acacagttaa cgttttctgc cctgattgga cttgatccgg     540
   ttgcaggtac aggcaaatca agccaattgc agcctaaaat cctgacgtat gaaccgtctt     600
   cctttggcat ggctatgcct gttcttgtga ttggaacagg tttgggcgaa gaaaagaaaa     660
   atattttctt tccgccgtgc gccccgaaag atgttaacca tgcagaattt tatcgtgaat     720
   gccggccgcc ttgttattac tttgtgacaa aagactacgg acatttagat atgttggatg     780
   acgatgcacc gaaatttatt acgtgcgtct gtaaagatgg aaatggttgc aaaggtaaaa     840
   tgagacgctg tgtcgcgggc attatggtag catttctgaa cgcagcgctg ggcgaaaaag     900
   acgcggatct tgaagctatc ttaagagacc cggcagttgc gccgacaacg cttgatccgg     960
   ttgaacatcg cgtggcttaa ttaa                                            984
<210> 4
   <211> 322
   <212> PRT
   <213> Chlamydomonas reinhardtii
<400> 4
   Met Pro Ser Thr Gln Phe Leu Gly Ala Ser Thr Leu Leu Leu Phe Gly
   1               5                   10                  15
   Leu Arg Ala Val Met Ser Ser Asp Asp Tyr Ile Lys Arg Gly Asp Leu
               20                  25                  30
   Pro Thr Ser Lys Trp Ser Gly Arg Val Thr Leu Arg Val Asp Ser Ala
           35                  40                  45
   Met Ala Val Pro Leu Asp Val Val Ile Thr Tyr Pro Ser Ser Gly Ala
       50                  55                  60
   Ala Ala Tyr Pro Val Leu Val Met Tyr Asn Gly Phe Gln Ala Lys Ala
   65                  70                  75                  80
   Pro Trp Tyr Arg Gly Ile Val Asp His Val Ser Ser Trp Gly Tyr Thr
                   85                  90                  95
   Val Val Gln Tyr Thr Asn Gly Gly Leu Phe Pro Ile Val Val Asp Arg
               100                 105                 110
   Val Glu Leu Thr Tyr Leu Glu Pro Leu Leu Thr Trp Leu Glu Thr Gln
           115                 120                 125
   Ser Ala Asp Ala Lys Ser Pro Leu Tyr Gly Arg Ala Asp Val Ser Arg
       130                 135                 140
   Leu Gly Thr Met Gly His Ser Arg Gly Gly Lys Leu Ala Ala Leu Gln
   145                 150                 155                 160
   Phe Ala Gly Arg Thr Asp Val Ser Gly Cys Val Leu Phe Asp Pro Val
                   165                 170                 175
   Asp Gly Ser Pro Met Thr Pro Glu Ser Ala Asp Tyr Pro Ser Ala Thr
               180                 185                 190
   Lys Ala Leu Ala Ala Ala Gly Arg Ser Ala Gly Leu Val Gly Ala Ala
           195                 200                 205
   Ile Thr Gly Ser Cys Asn Pro Val Gly Gln Asn Tyr Pro Lys Phe Trp
       210                 215                 220
   Gly Ala Leu Ala Pro Gly Ser Trp Gln Met Val Leu Ser Gln Ala Gly
   225                 230                 235                 240
   His Met Gln Phe Ala Arg Thr Gly Asn Pro Phe Leu Asp Trp Ser Leu
                   245                 250                 255
   Asp Arg Leu Cys Gly Arg Gly Thr Met Met Ser Ser Asp Val Ile Thr
               260                 265                 270
   Tyr Ser Ala Ala Phe Thr Val Ala Trp Phe Glu Gly Ile Phe Arg Pro
           275                 280                 285
   Ala Gln Ser Gln Met Gly Ile Ser Asn Phe Lys Thr Trp Ala Asn Thr
       290                 295                 300
   Gln Val Ala Ala Arg Ser Ile Thr Phe Asp Ile Lys Pro Met Gln Ser
   305                 310                 315                 320
   Pro Gln
<210> 5
   <211> 997
   <212> DNA
   <213> Chlamydomonas reinhardtii
<400> 5
   gcgcgcaggc tgctggaaaa atgccttcta cacaatttct tggagcatcc acgctgcttt      60
   tatttggttt acgtgcggtc atgtcaagcg atgactatat taaacggggt gatttgccga     120
   catcaaaatg gagcggaaga gtcacgttgc gcgtagattc agctatggcc gttccgctgg     180
   acgttgtgat cacatatcct tcttccggcg cagcggctta tccggtcctg gtaatgtaca     240
   atggatttca ggcaaaagcg ccgtggtaca gaggcattgt tgatcatgtg tcaagctggg     300
   gatatacagt cgtacaatac acgaacggcg gactttttcc tatcgttgtg gaccgcgtcg     360
   aacttacata tttagaaccg ttgctgacat ggttagaaac gcagtctgct gatgccaaat     420
   ccccgttgta cggcagagct gacgtatcac gcctgggcac aatgggacat agcagaggtg     480
   gcaaattggc cgcactgcaa tttgcgggcc gcacggatgt ttcaggatgc gtgctttttg     540
   atcctgtgga cggcagcccg atgacacctg aatcagctga ctatccgagc gctacgaaag     600
   cacttgcggc tgccggacgt tctgccggtt tagttggcgc agcgattaca ggttcatgta     660
   atccggtggg ccagaactac cctaaatttt ggggagcatt ggcgcctggt tcatggcaaa     720
   tggtcctgag ccaggcaggc catatgcaat ttgcgagaac aggaaatccg tttttagatt     780
   ggagccttga ccgtttatgc ggacggggta cgatgatgtc ttccgatgtc atcacatatt     840
   ctgctgcctt tacggtagct tggtttgaag gcatttttcg tccggcccaa tctcagatgg     900
   gaatctccaa ttttaaaaca tgggcaaaca cgcaagttgc agcgcggtct attacatttg     960
   atatcaaacc gatgcaatcc cctcagtaat taattaa                             997
<210> 6
   <211> 484
   <212> PRT
   <213> Arabidopsis thaliana
<400> 6
   Met Glu Ile Ile Ser Leu Asn Val Val Pro Gln Cys Ser Val Val Thr
   1               5                   10                  15
   Trp Ser Ser Lys Leu Ala Thr Lys Arg Leu Val Pro Asn Arg Ser Ser
               20                  25                  30
   Leu Leu Phe Ser Gly Val Lys Lys Ser Arg Leu Val Ile Arg Ser Gly
           35                  40                  45
   Asn Ser Asp Gly Tyr Val Val Gly Glu Asn Asp Asp Leu Gly Arg Ile
       50                  55                  60
   Ala Arg Arg Gly Glu Ser Thr Ser Lys Val Leu Ile Pro Gly Leu Pro
   65                  70                  75                  80
   Asp Glu Ser Asn Gly Glu Ile Ala Ala Arg Ile Ser His Ser His Cys
                   85                  90                  95
   Glu Trp Lys Pro Lys Leu Arg Val His Tyr Glu Lys Ala Gly Cys Asp
               100                 105                 110
   Asn Leu Asp Ala Pro Ala Val Leu Phe Leu Pro Gly Phe Gly Val Gly
           115                 120                 125
   Ser Phe His Tyr Glu Lys Gln Leu Thr Asp Leu Gly Arg Asp Tyr Arg
       130                 135                 140
   Val Trp Ala Ile Asp Phe Leu Gly Gln Gly Leu Ser Leu Pro Thr Glu
   145                 150                 155                 160
   Asp Pro Thr Thr Met Thr Glu Glu Thr Ser Ser Ser Glu Asp Lys Glu
                   165                 170                 175
   Pro Phe Trp Gly Phe Gly Asp Lys Thr Glu Pro Trp Ala Asp Gln Leu
               180                 185                 190
   Val Phe Ser Leu Asp Leu Trp Arg Asp Gln Val Gln Tyr Phe Val Glu
           195                 200                 205
   Glu Val Ile Gly Glu Pro Val Tyr Ile Ala Gly Asn Ser Leu Gly Gly
       210                 215                 220
   Tyr Val Ala Leu Tyr Phe Ala Ala Thr His Pro His Leu Val Lys Gly
   225                 230                 235                 240
   Val Thr Leu Leu Asn Ala Thr Pro Phe Trp Gly Phe Phe Pro Asn Pro
                   245                 250                 255
   Val Arg Ser Pro Lys Leu Ala Arg Leu Phe Pro Trp Pro Gly Ala Phe
               260                 265                 270
   Pro Leu Pro Glu Arg Val Lys Lys Ile Thr Glu Leu Val Trp Gln Lys
           275                 280                 285
   Ile Ser Asp Pro Glu Ser Ile Ala Glu Ile Leu Lys Gln Val Tyr Thr
       290                 295                 300
   Asp His Ser Ile Asn Val Asp Lys Val Phe Ser Arg Ile Val Glu Val
   305                 310                 315                 320
   Thr Gln His Pro Ala Ala Ala Ala Ser Phe Ala Ser Ile Met Leu Ala
   325 330 335
   Pro Gly Gly Glu Leu Ser Phe Ser Glu Ala Leu Ser Arg Cys Lys Glu
   340 345 350
   Asn Asn Val Gln Ile Cys Leu Met Tyr Gly Arg Glu Asp Pro Trp Val
   355 360 365
   Arg Pro Leu Trp Gly Lys Lys Ile Lys Lys Glu Ile Pro Asn Ala Pro
   370 375 380
   Tyr Tyr Glu Ile Ser Pro Ala Gly His Cys Pro His Asp Glu Val Pro
   385 390 395 400
   Glu Val Val Asn Tyr Leu Met Arg Gly Trp Ile Lys His Leu Glu Ser
   405 410 415
   Gly Gly Phe Glu Ala Leu Pro Leu Leu Glu Asp Thr Glu Glu Asp Trp
   420 425 430
   Glu Glu Ser Arg Ile Gly Arg Glu Ile Glu Phe Pro Arg Asp Gly Trp
   435 440 445
   Lys Lys Ala Val Asn Leu Trp Leu Tyr Gly Ser Asn Tyr Thr Tyr Trp
   450 455 460
   Arg Gly Val Arg Glu Ser Phe Arg Ser Ser Phe Ile Arg Val Phe Gly
   465 470 475 480
   Gly Lys Ser Ala
<210> 7
   <211> 1853
   <212> DNA
   <213> Arabidopsis thaliana
<400> 7
   aacccaattc ttctatttct tcttcacctt tagatttttc ctcgcttaat ttctcaataa      60
   cgctctcaga gagaccattt gatgaagctt ctcgcttctg gaatttgaaa aggatttgat     120
   aagacgagtt catagaagat taccgcaagt tcatcaactt tttgaacttg ttatggagat     180
   aatctcactg aacgttgtgc cccagtgctc tgtggttact tggagtagta aattagcaac     240
   gaaaagattg gtcccaaatc ggtcaagttt gttattctca ggggtcaaaa aatccagact     300
   tgtgattcga agtggaaatt ccgatggtta tgttgttggt gagaatgatg acttgggtcg     360
   tatagccaga agaggagaat caacgtcaaa ggttttgatt cctggtttgc ctgatgaatc     420
   aaatggtgaa attgctgctc gaatcagtca ttctcactgc gagtggaagc ccaagcttag     480
   agtacattat gagaaagccg gttgtgacaa tctcgatgct cctgcggtgt tgtttcttcc      540
   tggctttggc gttggttcat ttcactatga gaagcagctt accgatttgg gaagggatta      600
   tcgagtatgg gctattgatt ttcttggaca gggtttatct ctccctactg aagatcctac      660
   taccatgact gaagaaacca gttcctcgga agataaggaa ccattttggg gatttggtga      720
   caaaactgaa ccgtgggctg atcaacttgt attctctctg gatctctgga gggatcaagt      780
   tcagtatttt gtagaagagg ttatcggtga gcctgtgtac attgcaggga actcacttgg      840
   agggtatgta gctctctact ttgcagcaac ccatcctcac ctggttaagg gtgttacctt      900
   gcttaatgca acacctttct ggggtttctt ccctaatcca gtaagatccc caaagctagc      960
   acgtctcttt ccatggcccg gagcattccc tctgccggaa agagtgaaaa aaatcacaga     1020
   attggtgtgg caaaagataa gtgatcctga aagcatagct gagatactta aacaggtcta     1080
   cacagaccat tctatcaatg tggataaagt attctcacgt attgtggagg tcacacagca     1140
   tccggctgct gcagcatcgt ttgcttcaat catgcttgct cctggtggag agctatcttt     1200
   ctccgaagct ttatctaggt gtaaggaaaa caatgttcag atatgtctca tgtatggaag     1260
   agaagatcca tgggtgagac cgttatgggg aaagaagata aagaaggaaa tccccaacgc     1320
   tccatactac gagatcagcc cagcgggtca ctgcccacac gatgaagtcc ctgaggtggt     1380
   gaactatctg atgcgcgggt ggatcaagca cctggagtct ggtggttttg aagcgctccc     1440
   gcttttggag gacactgaag aagattggga ggagtccagg attggtagag aaattgagtt     1500
   cccgagagat ggttggaaaa aagcagtgaa tctgtggtta tatgggtcaa actatacgta     1560
   ctggagagga gttagagaat ctttcagatc cagttttata agggtgtttg gagggaagtc     1620
   tgcatagaag aagcatggaa cagtcgtcta gtgtaaatta attgtaatct atgttgcatc     1680
   cgatgctagc tatataatgt tgtctgtaga atcaagtttc taaaatgttc aaaaggaaaa     1740
   gttagaaaaa tatctacttg atagttagtc acctaaatcg aaggaactcc tttcttgcat     1800
   tgttgtatat aatccacagg ttcagattaa tataggaagg cgacattgca ggc            1853
<210> 8
   <211> 566
   <212> PRT
   <213> Populus trichocarpa
<400> 8
   Met Met Ile Leu Ala Phe Phe Leu Ile Phe Met Glu Phe Tyr Phe Gln
   1               5                   10                  15
   Leu Arg Arg Arg Tyr Ala Ser Tyr Leu Leu Ile Asn Met Ile Leu Leu
               20                  25                  30
   Ile Thr Ala Asp Gln Pro Phe Trp Gly Met Glu Ile Leu Thr Ser Ser
           35                  40                  45
   Thr Ala Ser Cys Cys Leu Val Val Asn Leu Arg Trp Lys Leu Ala Glu
       50                  55                  60
   Asn Gly Ser Asn Ser Ser Gln Leu Lys Leu Pro Thr Ser Arg Glu Arg
   65                  70                  75                  80
   Lys Ile Leu Phe Ala Arg Thr Asn Gln Arg Asn Gly Ser Leu Arg Phe
                   85                  90                  95
   Ser Ser Val Asp Lys Phe Leu Lys Lys Leu Asn His Gly Lys Gly Ser
               100                 105                 110
   Arg Ser Leu Asp Ser Phe Gly Gly Leu Lys Asn Gly Asn Ser Lys Val
           115                 120                 125
   Phe Ser Gly Asn Ser Ser Ser Tyr Val Val Gly Gly Glu Asp Asp Val
       130                 135                 140
   Gly Ser Ile Thr Glu Asn Gly Glu Ser Pro Thr Lys Val Leu Ile Pro
   145                 150                 155                 160
   Gly Leu Pro Asp Glu Ser Asn Gly Glu Tyr Ser Ala Pro Val Ser Ser
                   165                 170                 175
   Cys Phe Trp Lys Trp Lys Pro Lys Leu Asn Val His Tyr Glu Lys Ala
               180                 185                 190
   Gly Cys Glu Asn Val Asn Ser Pro Pro Val Leu Phe Leu Pro Gly Phe
           195                 200                 205
   Gly Val Gly Ser Phe His Tyr Glu Lys Gln Leu Lys Asp Leu Gly Arg
       210                 215                 220
   Asp Tyr Arg Val Trp Ala Ile Asp Phe Leu Gly Gln Gly Met Ser Leu
   225                 230                 235                 240
   Pro Val Glu Asn Pro Thr Leu Phe Ser Lys Asp Gly Ala Ala Ser Glu
                   245                 250                 255
   Gly Lys Asp Ser Ile Trp Gly Phe Gly Asp Glu Ile Glu Pro Trp Ala
               260                 265                 270
   Asn Asp Leu Val Phe Ser Met Asp Leu Trp Gln Asp Gln Val His Asn
           275                 280                 285
   Phe Ile Glu Glu Val Ile Gly Glu Pro Val Tyr Ile Val Gly Asn Ser
       290                 295                 300
   Leu Gly Gly Phe Val Ala Leu Tyr Phe Ala Ala Arg Tyr Pro His Leu
   305 310 315 320
   Val Lys Gly Val Thr Leu Leu Asn Ala Thr Pro Phe Trp Gly Phe Leu
   325 330 335
   Pro Asn Pro Ile Arg Ser Pro Arg Leu Ala Arg Ile Phe Pro Trp Ser
   340 345 350
   Gly Thr Phe Pro Leu Pro Ala Asn Val Arg Lys Leu Ile Ala Phe Phe
   355 360 365
   Trp Gln Lys Ile Ser Asp Pro Lys Ser Ile Ala Glu Ile Leu Lys Gln
   370 375 380
   Val Tyr Thr Asp His Ser Thr Asn Ile Asp Lys Val Phe Ser Arg Ile
   385 390 395 400
   Leu Glu Ile Thr Gln His Pro Ala Ala Ala Ala Ser Phe Ala Ser Ile
   405 410 415
   Met Phe Ala Pro Gln Gly Gln Leu Ser Phe Arg Glu Thr Leu Ala Arg
   420 425 430
   Cys Lys Met Ser Asp Thr Pro Ile Cys Leu Val Tyr Gly Lys Glu Asp
   435 440 445
   Pro Trp Val Lys Pro Val Trp Gly Leu Gln Val Lys Gln Gln Val Pro
   450 455 460
   Glu Ala Pro Tyr Tyr Glu Ile Ser Pro Ala Gly His Cys Pro His Asp
   465 470 475 480
   Glu Val Pro Glu Ala Val Asn Tyr Leu Leu Arg Gly Trp Ile Lys Asn
   485 490 495
   Leu Glu Ser His Gly Ser Val Ala Leu Pro Leu His Glu Asp Ala Glu
   500 505 510
   Val Val Glu Asn Ser Phe Ala Met Asp Leu Glu Phe Val Arg Glu Gly
   515 520 525
   Ser Arg Lys Ser Val Ile Val Arg Phe Phe Gly Ser Arg Phe Ser Ile
   530 535 540
   Trp Asn Ser Phe Ser Ser Tyr Ile Lys Ser Gln Phe Lys Glu Thr Thr
   545 550 555 560
   Ser Arg Ile Leu Thr Pro
   565
<210> 9
   <211> 524
   <212> PRT
   <213> Vitis vinifera
<400> 9
   Met Glu Ile Leu Ser Cys His Ser Ala Pro Cys Cys Lys Leu Val Asn
   1               5                   10                  15
   Leu Gly Gly Thr Ser Val His Lys Ser Ser Gly Ser Ser Gln Ala Lys
               20                  25                  30
   Leu Pro Gly Ser Arg Asn Asn Arg Ile Leu Cys Ala Arg Ile Gly Ser
           35                  40                  45
   Lys Leu Gly Ser Ser Gly Tyr Ser Asn Leu Asp Asp Phe Cys Thr Lys
       50                  55                  60
   Asn Phe Gly Arg His Glu Gly Ser Arg Ser Leu Thr Ala Phe Lys Gly
   65                  70                  75                  80
   Ser Ala Asn Val Asn Ser Lys Ala Leu Ser Glu Ser Tyr Asn Gly Tyr
                   85                  90                  95
   Val Ile Asp Gly Lys Glu Gly Val Gly Asp Ile Ser Glu Arg Gly Asp
               100                 105                 110
   Leu Ile Thr Gln Ile Leu Ile Pro Gly Leu Pro Asp Asp Ser Asn Asp
           115                 120                 125
   Asp Ser Gly Ala Gln Ile Ser Ser Cys Phe Trp Glu Trp Lys Pro Lys
       130                 135                 140
   Leu Thr Val His Tyr Glu Lys Ser Gly Cys Glu Asn Val Asn Ser Pro
   145                 150                 155                 160
   Pro Val Leu Phe Leu Pro Gly Phe Gly Val Gly Ser Phe His Tyr Glu
                   165                 170                 175
   Lys Gln Leu Lys Asp Leu Gly Arg Asp Phe Arg Val Trp Ala Val Asp
               180                 185                 190
   Phe Leu Gly Gln Gly Met Ser Leu Pro Phe Glu Asp Pro Ala Pro Gln
           195                 200                 205
   Ser Lys Lys Glu Leu Asp Ser Glu Arg Asn Asp Phe Ser Trp Gly Phe
       210                 215                 220
   Gly Asp Glu Thr Glu Pro Trp Ala Asn Glu Leu Val Tyr Ser Ile Asp
   225                 230                 235                 240
   Leu Trp Gln Asp Gln Val Arg Tyr Phe Ile Glu Gln Val Ile Gly Glu
                   245                 250                 255
   Pro Val Tyr Ile Val Gly Asn Ser Leu Gly Gly Phe Val Ala Leu Tyr
               260                 265                 270
   Phe Ala Ala Cys Asn Pro Gln Leu Val Lys Gly Val Thr Leu Leu Asn
           275                 280                 285
   Ala Thr Pro Phe Trp Gly Phe Leu Pro Asn Pro Ser Arg Ser Pro Ser
       290                 295                 300
   Leu Ala Arg Ile Phe Pro Trp Ala Gly Thr Phe Pro Leu Pro Ala Phe
   305                 310                 315                 320
   Val Arg Lys Leu Thr Glu Phe Val Trp Gln Lys Ile Ser Asp Pro Arg
                   325                 330                 335
   Ser Ile Gly Glu Val Leu Lys Gln Val Tyr Ala Asp His Ser Thr Lys
               340                 345                 350
   Val Asp Lys Val Phe Ser Arg Ile Leu Glu Thr Thr Gln His Pro Ala
           355                 360                 365
   Ala Ala Ala Ser Phe Ala Ser Ile Met Phe Ala Pro Gln Gly Gln Leu
       370                 375                 380
   Ser Phe Ser Glu Ala Leu Ser Arg Cys Gln Met Ser Asn Val Pro Ile
   385                 390                 395                 400
   Cys Leu Met Tyr Gly Lys Glu Asp Pro Trp Val Arg Pro Val Trp Gly
                   405                 410                 415
   Leu Gln Val Lys Arg Gln Leu Leu Glu Ala Pro Tyr Tyr Glu Ile Ser
               420                 425                 430
   Pro Ala Gly His Cys Pro His Asp Glu Val Pro Glu Val Val Asn Tyr
           435                 440                 445
   Leu Leu Arg Gly Trp Ile Gly Asn Leu Glu Ser Lys Gly Ser Val Thr
       450                 455                 460
   Leu Pro Leu Leu Asp Asp Pro Glu Asn Ile Gln Tyr Gly Thr Thr Lys
   465                 470                 475                 480
   Asp Leu Glu Phe Val Arg Glu Gly Ser Lys Lys Ser Val Arg Val His
   485 490 495
   Phe Tyr Gly Ser Arg Phe Ser Leu Trp Asn Arg Ile Arg Ser Tyr Val
   500 505 510
   Lys Ser Arg Phe Glu Ala Leu Glu Ile Asn Ser Arg
   515 520
<210> 10
   <211> 481
   <212> PRT
   <213> Ricinus communis
<400> 10
   Met Phe Ser Pro Cys Pro Leu Ile Ser Ser Gly Gln Thr Gln Trp Leu
   1               5                   10                  15
   Asp Leu Gly Met Asp Ile Leu Thr Phe Asn Val Thr Thr Ser His Arg
               20                  25                  30
   Thr Ala His Phe Gly Ser Lys Leu Val Asp Lys Thr Lys Tyr Ser Cys
           35                  40                  45
   Lys Ser Lys Val Ser Thr Ile Ile Lys Pro Gln Val Phe Cys Ala Arg
       50                  55                  60
   Ile Asp Gln Ser Cys Gly Leu Leu Arg Phe Ser Ser Ser Asn Lys Phe
   65                  70                  75                  80
   Leu Asp Tyr Pro Lys Lys Ile Glu Val Ser Lys Lys His Asn Ala Leu
                   85                  90                  95
   Lys Gly Ile Lys Val Val Asn Ser Lys Val Leu Ser Gly Asn Tyr Asn
               100                 105                 110
   Gly Tyr Val Ile Glu Ala Asp Glu Asp Met Glu Ser Val Ser Gly Ser
           115                 120                 125
   Gly Glu Ser Thr Pro Glu Ile Leu Ile Pro Gly Leu Pro Asn Glu Ser
       130                 135                 140
   Ser Gly Glu Cys Gly Ala Pro Ile Asn Ser Cys Phe Trp Glu Trp Lys
   145                 150                 155                 160
   Pro Lys Leu Tyr Val His Tyr Glu Lys Ala Gly Cys Glu Asn Val Lys
                   165                 170                 175
   Ser Pro Pro Val Leu Phe Leu Pro Gly Phe Gly Val Gly Ser Phe His
   180 185 190
   Phe Glu Asn Gln Leu Lys Asp Leu Gly Arg Asp Tyr Arg Val Trp Ala
   195 200 205
   Ile Asp Phe Leu Gly Gln Gly Met Ser Leu Pro Val Glu Asn Pro Thr
   210 215 220
   Leu Gln Leu Arg Glu Gly Asp Ile Leu Glu Gly Lys Asn Ser Phe Trp
   225 230 235 240
   Gly Phe Gly Asp Glu Thr Glu Pro Trp Ala Asn Glu Leu Val Tyr Ser
   245 250 255
   Met Asp Leu Trp Arg Asp Gln Val Arg Tyr Phe Ile Glu Glu Val Ile
   260 265 270
   Gly Glu Pro Val Tyr Val Val Gly Asn Ser Leu Gly Gly Phe Val Ala
   275 280 285
   Ile Tyr Phe Ala Ala Ser Asn Pro Gln Leu Val Lys Gly Val Thr Leu
   290 295 300
   Leu Asn Ala Thr Pro Phe Trp Gly Phe Leu Pro Asn Pro Ile Arg Ser
   305 310 315 320
   Pro Arg Leu Ala Arg Ile Ile Pro Trp Ser Gly Thr Phe Pro Leu Pro
   325 330 335
   Ala Ser Val Arg Lys Leu Thr Glu Phe Phe Trp Gln Lys Ile Ser Asp
   340 345 350
   Pro Lys Ser Ile Ala Gln Val Leu Lys Gln Val Tyr Ala Asp His Ser
   355 360 365
   Thr Asn Val Asp Gln Val Phe Ser Arg Ile Leu Lys Ile Thr Gln His
   370 375 380
   Pro Ala Ala Ala Ala Ser Phe Ala Ser Ile Met Phe Ala Pro Gln Gly
   385 390 395 400
   Gln Leu Ser Phe Arg Glu Cys Leu Met Arg Cys Lys Met Asn Asn Leu
   405 410 415
   Pro Ile Cys Leu Leu Tyr Gly Arg Glu Asp Pro Trp Val Lys Pro Ile
   420 425 430
   Trp Gly Leu Gln Val Lys Arg Gln Val Pro Glu Ala Ser Tyr Tyr Glu
   435 440 445
   Ile Ser Pro Ala Gly His Cys Pro His Asp Glu Val Pro Glu Val Cys
       450                 455                 460
   Ser Leu Ser Leu Phe Leu Val Gly Ile Pro Leu Leu Phe Leu Val Ile
   465                 470                 475                 480
   Leu
<210> 11
   <211> 486
   <212> PRT
   <213> Oryza sativa
<400> 11
   Met Glu Val Val Ser Ser Ser His Ser Cys Leu Ala Phe Asn Arg Thr
   1               5                   10                  15
   Pro Ser Ser Ala Trp Arg Phe Pro Gly Asn Gly Leu Gly Pro Gly His
               20                  25                  30
   Ala Lys Leu Thr Arg Pro Arg Ser Ala Ile Leu Cys Val Arg Ser Gly
           35                  40                  45
   Thr Ala Ser Asn Pro Ala Asp Ser Gly Lys Val His Ala Ser His Gly
       50                  55                  60
   Phe Tyr Val Ser Asp Val Asp Ala Ala Leu Gln Gly Ile Pro Lys Lys
   65                  70                  75                  80
   Val Gly Glu Ile Glu Lys Met Ile Ile Pro Ser Leu Pro Glu Gly Pro
                   85                  90                  95
   Glu Ser Ser Leu Ile Ser Thr Gly Phe Trp Glu Trp Lys Pro Lys Leu
               100                 105                 110
   Ser Val Tyr Tyr Glu Lys Ser Gly Ile Asp Asn Ser Lys Ala Pro Ser
           115                 120                 125
   Val Leu Phe Leu Pro Gly Phe Gly Val Gly Thr Phe His Phe Glu Lys
       130                 135                 140
   Gln Leu Lys Asp Leu Gly Arg Asp Tyr Lys Val Trp Thr Met Asp Phe
   145                 150                 155                 160
   Leu Gly Gln Gly Met Ser Leu Pro Cys Glu Asp Pro Ala Pro Lys Ser
                   165                 170                 175
   Thr Ser Gly Glu Leu Asp Glu Asp Thr Tyr Trp Gly Phe Gly Gln Glu
               180                 185                 190
   Leu Gln Pro Trp Ala Glu Glu Leu Val Tyr Ser Ile Asp Leu Trp Arg
           195                 200                 205
   Asp Gln Val Gln His Phe Ile Glu Glu Val Ile Gly Glu Pro Val Tyr
       210                 215                 220
   Ile Val Gly Asn Ser Leu Gly Gly Phe Val Ser Leu Tyr Leu Ala Ala
   225                 230                 235                 240
   Ser Cys Pro His Leu Val Lys Gly Val Thr Leu Leu Asn Ala Thr Pro
                   245                 250                 255
   Phe Trp Gly Phe Leu Pro Asn Pro Ala Thr Ser Pro Arg Leu Ser Lys
               260                 265                 270
   Ile Phe Pro Trp Ala Gly Thr Phe Pro Leu Pro Ser Phe Val Arg Lys
           275                 280                 285
   Leu Thr Glu Thr Val Trp Gln Lys Ile Ser Asp Pro Arg Ser Ile Gln
       290                 295                 300
   Gly Ile Leu Lys Gln Val Tyr Ala Asp His Ser Thr Asn Val Asp Met
   305                 310                 315                 320
   Val Phe Ser Arg Ile Ile Glu Thr Thr Gln His Pro Ala Ala Ala Ala
                   325                 330                 335
   Ser Phe Ala Ser Ile Met Cys Ala Pro Lys Gly Gln Ile Ser Phe Glu
               340                 345                 350
   Glu Ala Leu Ser Arg Cys Gln Arg Gln Gly Ile Pro Ile Ser Leu Met
           355                 360                 365
   Tyr Gly Arg Glu Asp Pro Trp Val Arg Pro Ile Trp Gly Ile Lys Val
       370                 375                 380
   Lys Gln Gln Val Pro Glu Ser Pro Tyr Tyr Glu Ile Ser Pro Ala Gly
   385                 390                 395                 400
   His Cys Pro His Asp Glu Val Pro Glu Val Ile Asn Tyr Leu Leu Arg
                   405                 410                 415
   Gly Trp Leu Lys Asn Val Glu Ser Glu Gly Ser Val Ala Val Pro Phe
               420                 425                 430
   Leu Glu Glu Pro Ser Tyr Ala Glu Asn Gly Val Ser Arg Glu Leu Glu
                   435                 440                 445
   Phe Val Arg Gly Gly Ser Lys Lys Ser Val His Val Arg Leu Phe Gly
       450                 455                 460
   Ser Lys Ile Ser Leu Trp Ser Gln Leu Arg Ser Leu Leu Lys Ser Asn
   465                 470                 475                 480
   Thr Trp Val Ile Ser Arg
                   485
<210> 12
   <211> 491
   <212> PRT
   <213> Zea mays
<400> 12
   Met Glu Val Val Ser Cys Ser His Ser Cys Ser Ala Leu His Gln Thr
   1               5                   10                  15
   Pro Ala Ser Thr Trp Arg Leu Arg Gly Ser Ala Leu Gly Leu Gly Leu
               20                  25                  30
   Gly His Ala Arg Pro Ser Arg Thr Arg Arg Tyr Thr Val Ala Cys Val
           35                  40                  45
   Gly Thr Thr Ser Gly Ala Ser Asn Pro Gly Gly Ser Gly Lys Val His
       50                  55                  60
   Ala Ala Gln Gly Phe His Val Ser Asp Val Asp Ala Ala Leu Gln Gly
   65                  70                  75                  80
   Ile Pro Ser Met Lys Ala Gly Glu Ala Glu Arg Val Met Ile Gln Gly
                   85                  90                  95
   Leu Pro Glu Gly Pro Asp Ser Ser Pro Ile Ser Thr Gly Phe Trp Glu
               100                 105                 110
   Trp Lys Pro Lys Leu Thr Val His Tyr Glu Arg Ser Gly Met Lys Asn
           115                 120                 125
   Ser Lys Ala Pro Ala Val Leu Phe Leu Pro Gly Phe Gly Val Gly Thr
       130                 135                 140
   Phe His Phe Glu Lys Gln Leu Arg Asp Leu Gly Arg Asp His Arg Val
   145                 150                 155                 160
   Trp Thr Met Asp Phe Leu Gly Gln Gly Met Ser Leu Pro Gly Glu Asp
                   165                 170                 175
   Pro Ala Pro Ser Ser Ile Ala Ser Glu Asp Ala Phe Trp Gly Phe Gly
               180                 185                 190
   Gln Asp Ser Gln Pro Trp Ala Glu Glu Leu Val Tyr Ser Val Asp Leu
           195                 200                 205
   Trp Gln Asn Gln Val Gln His Phe Ile Glu Glu Val Ile Arg Glu Pro
       210                 215                 220
   Val Tyr Ile Val Gly Asn Ser Leu Gly Gly Phe Val Ala Leu Tyr Phe
   225                 230                 235                 240
   Ala Ala Ser Ser Pro His Leu Val Lys Gly Val Thr Leu Leu Asn Ala
                   245                 250                 255
   Thr Pro Phe Trp Gly Phe Phe Pro Asn Pro Ala Thr Ser Pro Arg Leu
               260                 265                 270
   Ser Lys Ile Phe Pro Trp Ala Gly Thr Phe Pro Leu Pro Ser Phe Val
           275                 280                 285
   Arg Lys Ile Thr Glu Ala Val Trp Gln Lys Ile Ser Asp Pro Lys Ser
       290                 295                 300
   Ile Gln Asp Ile Leu Lys Gln Val Tyr Ala Asp His Ser Thr Asn Val
   305                 310                 315                 320
   Asp Lys Val Phe Ser Arg Ile Val Glu Ile Thr Gln His Pro Ala Ala
                   325                 330                 335
   Ala Ala Ser Phe Ala Ser Ile Met Phe Ala Pro Arg Gly Gln Ile Ser
               340                 345                 350
   Phe Gln Glu Ala Ile Ser Arg Cys Gln Asp Gln Gly Ile Pro Ile Ser
           355                 360                 365
   Leu Met Tyr Gly Arg Glu Asp Pro Trp Ile Arg Pro Ile Trp Gly Leu
       370                 375                 380
   Lys Val Lys Gln Gln Val Pro Glu Ala Pro Tyr Tyr Glu Ile Ser Pro
   385                 390                 395                 400
   Ala Gly His Cys Pro His Asp Glu Val Pro Glu Val Ile Asn Tyr Leu
                   405                 410                 415
   Leu Arg Gly Trp Leu Lys Asn Leu Glu Ser Glu Gly Ser Val Asp Leu
               420                 425                 430
   Pro Phe Leu Glu Glu Arg Ser Tyr Ala Glu Arg Gly Val Ser Arg Glu
           435                 440                 445
   Leu Glu Phe Val Arg Glu Gly Ser Arg Lys Ser Val Ser Val Arg Leu
       450                 455                 460
   Tyr Gly Thr Lys Ile Ser Leu Trp Ser Gln Leu Ser Ser Phe Leu Asn
   465                 470                 475                 480
   Thr Arg Val Pro Lys Ser Arg Ile Val Leu Arg
                   485                 490
<210> 13
   <211> 505
   <212> PRT
   <213> Nicotiana tabacum
<400> 13
   Met Glu Val His Ser Cys Tyr Ser Thr Thr Tyr Tyr Cys Ile Val Asn
   1               5                   10                  15
   Val Ser Lys Cys Leu Ile Ser Asn Gln Ala Lys Phe Pro Ile Val Lys
               20                  25                  30
   Glu Arg Arg Leu Tyr Ser Gly Leu Asp Val Tyr Ser Ile Lys Lys Lys
           35                  40                  45
   Arg Thr Gln Arg Leu Thr Ile Thr Ala Leu Lys Gly Phe Asp Ser Val
       50                  55                  60
   Asp Ser Ser Leu Leu Ser Glu Ser Tyr Asn Ser Asp Ile Ile Asp Gly
   65                  70                  75                  80
   Lys Val Gly Thr Gln Asp Val Ile Gly Ser Ala Lys Ser Val Pro Lys
                   85                  90                  95
   Val Ile Val Pro Ser Leu Pro Asp Glu Thr Lys Ala Asp Ser Val Ala
               100                 105                 110
   Val Val Asp Ser Cys Leu Trp Glu Trp Lys Pro Lys Leu Lys Val His
           115                 120                 125
   Tyr Glu Lys Ser Gly Cys Gln Asn Val Asn Ser Ala Pro Ile Leu Phe
       130                 135                 140
   Leu Pro Gly Phe Gly Val Gly Ser Phe His Tyr Glu Lys Gln Leu Lys
   145                 150                 155                 160
   Asp Leu Gly Cys Asp His Arg Ile Trp Ala Leu Asp Phe Leu Gly Gln
                   165                 170                 175
   Gly Lys Ser Leu Pro Cys Glu Asp Pro Thr Leu Gln Ser Lys Arg Leu
               180                 185                 190
   Asp Glu Ser Glu Arg Asp Gly Asn Asn Ala Val Trp Gly Phe Gly Asp
           195                 200                 205
   Glu Ala Glu Pro Trp Ala Lys Glu Leu Val Tyr Ser Val Asp Leu Trp
       210                 215                 220
   Arg Asp Gln Val Arg Tyr Phe Ile Glu Glu Val Ile Lys Glu Pro Val
   225                 230                 235                 240
   Tyr Ile Val Gly Asn Ser Leu Gly Gly Tyr Val Ala Leu Tyr Leu Ala
                   245                 250                 255
   Ala Tyr Tyr Pro Gln Leu Val Lys Gly Val Thr Leu Leu Asn Ala Thr
               260                 265                 270
   Pro Phe Trp Gly Phe Leu Pro Asn Pro Val Arg Ser Pro Arg Leu Ser
           275                 280                 285
   Arg Leu Phe Pro Trp Ala Gly Thr Phe Pro Leu Pro Asp Thr Ile Arg
       290                 295                 300
   Lys Leu Thr Glu Leu Val Trp Gln Lys Ile Ser Ala Pro Glu Ser Ile
   305                 310                 315                 320
   Ala Glu Val Leu Lys Gln Val Tyr Ala Asp His Thr Thr Lys Val Asp
                   325                 330                 335
   Lys Val Phe Ser Ser Ile Leu Glu Val Thr Glu His Pro Ala Ala Ala
               340                 345                 350
   Ala Ser Leu Ala Ser Ile Leu Phe Ala Pro Arg Gly Gln Leu Ser Phe
           355                 360                 365
   Lys Glu Ala Leu Thr Gly Cys Arg Met Asn Asn Val Pro Val Cys Leu
       370                 375                 380
   Met Tyr Gly Lys Glu Asp Pro Trp Val Met Pro Phe Trp Ala Leu Gln
   385                 390                 395                 400
   Val Lys Arg Gln Leu Pro Glu Ala Pro Tyr Tyr Gln Ile Ser Pro Ala
                   405                 410                 415
   Gly His Cys Pro His Asp Glu Val Pro Glu Ile Val Asn Phe Leu Leu
               420                 425                 430
   Arg Gly Trp Ile Lys Asn Ile Glu Ser His Ser Ser Val Ala Leu Pro
           435                 440                 445
   Leu Leu Asp Ser Pro Glu Ser Ile Glu Tyr Asp Ile Val Arg Asp Leu
       450                 455                 460
   Glu Phe Val Arg Gln Gly Met Lys Lys Ser Val Arg Val Gln Phe Tyr
   465                 470                 475                 480
   Gly Ser Met Thr Ser Gln Trp Glu Lys Leu Gly Met Phe Leu Lys Ser
                   485                 490                 495
   Arg Phe Gln Tyr Gly Val Tyr Ser Pro
               500                 505
<210> 14
   <211> 426
   <212> PRT
   <213> Oryza sativa
<400> 14
   Met Glu Val Val Ser Ser Ser His Ser Cys Leu Ala Phe Asn Arg Thr
   1               5                   10                  15
   Pro Ser Ser Ala Trp Arg Phe Pro Gly Asn Gly Leu Gly Pro Gly His
               20                  25                  30
   Ala Lys Leu Thr Arg Pro Arg Ser Ala Ile Leu Cys Val Arg Ser Gly
           35                  40                  45
   Thr Ala Ser Asn Pro Ala Asp Ser Gly Lys Val His Ala Ser His Gly
       50                  55                  60
   Phe Tyr Val Ser Asp Val Asp Ala Ala Leu Gln Gly Ile Pro Lys Lys
   65                  70                  75                  80
   Val Gly Glu Ile Glu Lys Met Ile Ile Pro Ser Leu Pro Glu Gly Pro
                   85                  90                  95
   Glu Ser Ser Leu Ile Ser Thr Gly Phe Trp Glu Trp Lys Pro Lys Leu
               100                 105                 110
   Ser Val Tyr Tyr Glu Lys Ser Gly Ile Asp Asn Ser Lys Ala Pro Ser
           115                 120                 125
   Val Leu Phe Leu Pro Gly Phe Gly Val Gly Thr Phe His Phe Glu Lys
       130                 135                 140
   Gln Leu Lys Asp Leu Gly Arg Asp Tyr Lys Val Trp Thr Met Asp Phe
   145 150 155 160
   Leu Gly Gln Gly Met Ser Leu Pro Cys Glu Asp Pro Ala Pro Lys Ser
   165 170 175
   Thr Ser Gly Glu Leu Asp Glu Asp Thr Tyr Trp Gly Phe Gly Gln Glu
   180 185 190
   Leu Gln Pro Trp Ala Glu Glu Leu Val Tyr Ser Ile Asp Leu Trp Arg
   195 200 205
   Asp Gln Val Gln His Phe Ile Glu Glu Val Ile Gly Glu Pro Val Tyr
   210 215 220
   Ile Val Gly Asn Ser Leu Gly Gly Phe Val Ser Leu Tyr Leu Ala Ala
   225 230 235 240
   Ser Cys Pro His Leu Val Lys Gly Val Thr Leu Leu Asn Ala Thr Pro
   245 250 255
   Phe Trp Gly Phe Leu Pro Asn Pro Ala Thr Ser Pro Arg Leu Ser Lys
   260 265 270
   Ile Phe Pro Trp Ala Gly Thr Phe Pro Leu Pro Ser Phe Val Arg Lys
   275 280 285
   Leu Thr Glu Thr Val Trp Gln Lys Ile Ser Asp Pro Arg Ser Ile Gln
   290 295 300
   Gly Ile Leu Lys Gln Val Tyr Ala Asp His Ser Thr Asn Val Asp Met
   305 310 315 320
   Val Phe Ser Arg Ile Ile Glu Thr Thr Gln His Pro Ala Ala Ala Ala
   325 330 335
   Ser Phe Ala Ser Ile Met Cys Ala Pro Lys Gly Gln Ile Ser Phe Glu
   340 345 350
   Glu Ala Leu Ser Arg Cys Gln Arg Gln Gly Ile Pro Ile Ser Leu Met
   355 360 365
   Tyr Gly Arg Glu Asp Pro Trp Val Arg Pro Ile Trp Gly Ile Lys Val
   370 375 380
   Lys Gln Gln Val Pro Glu Ser Pro Tyr Tyr Glu Ile Ser Pro Ala Gly
   385 390 395 400
   His Cys Pro His Asp Glu Val Pro Glu Val Pro Gly Lys Ser Leu Ala
   405 410 415
   Trp Trp Ile Thr Gly Arg Leu Gln Ala Ser
   420 425
<210> 15
   <211> 337
   <212> PRT
   <213> Physcomitrella patens subsp. patens
<400> 15
   Ile Ala Ser His Ile Trp Glu Trp Arg His Arg Trp Asn Ile His Tyr
   1 5 10 15
   Glu Cys Ala Gly Thr Ser Leu Asn Thr Asn Ala Pro Ala Met Leu Leu
   20 25 30
   Leu Pro Gly Phe Gly Val Gly Ser Phe His Tyr His Gln Gln Leu Arg
   35 40 45
   Asp Leu Gly Gln Glu Tyr Arg Val Trp Ala Ile Asp Phe Leu Gly Gln
   50 55 60
   Gly Lys Ser Trp Pro Ser His Asp Pro Ala Pro Glu Glu Ala Glu Glu
   65 70 75 80
   Val Val Glu Glu Ile Arg His Trp Ser Leu Gly Lys Asn Pro Glu Pro
   85 90 95
   Trp Ala Glu Gly Leu Val Tyr Ser Val Asp Thr Trp Arg Asp Gln Val
   100 105 110
   His Ala Phe Ile Glu Lys Val Ile Gly Gly Pro Val Tyr Ile Val Gly
   115 120 125
   Asn Ser Leu Gly Gly Tyr Val Gly Ser Tyr Phe Ala Ala Thr Asn Pro
   130 135 140
   Glu Leu Val Lys Gly Val Thr Leu Leu Asn Ala Thr Pro Phe Trp Ala
   145 150 155 160
   Phe Thr Pro Asn Ser Arg Arg Tyr Pro Leu Leu Ser Lys Leu Thr Pro
   165 170 175
   Trp Gly Gly Leu Leu Pro Val Pro Ile Phe Ala Lys Ala Ile Ile Arg
   180 185 190
   Phe Trp Trp Asp Leu Leu Arg Asn Pro Ser Thr Ile Arg Asn Met Leu
   195 200 205
   Gly Ala Val Tyr Ala Asn Arg Ser Ala Ile Asn Lys Lys Leu Ile Thr
   210 215 220
   Gln Ile Ile Glu Ala Thr Asp His Pro Ala Ala Phe Ala Ala Phe Ala
   225 230 235 240
   Ser Ile Val Phe Ala Pro Arg Ala His Thr Asp Phe Gly Glu Asn Leu
   245 250 255
   Ile Ser Leu Lys Glu Arg Arg Met Pro Met Cys Met Ile Tyr Gly Lys
   260 265 270
   Glu Asp Pro Trp Val Val Pro Phe Trp Gly Gln Arg Ala Lys Gln Arg
   275 280 285
   Asn Pro Asp Ala Ile Tyr Tyr Glu Leu Ser Pro Ala Gly His Cys Pro
   290 295 300
   His His Glu Ala Pro Glu Val Leu Phe Pro Ala Gln Ile Val Leu Leu
   305 310 315 320
   Ala Cys Met Val Gln Asn Ile Ile Gly Lys Ala Arg Pro Leu Phe Lys
   325 330 335
   Gly
<210> 16
   <211> 7
   <212> PRT
   <213> Artificial Sequence
<220>
   <223> Conserved sequence motif
<400> 16
   Leu Pro Gly Phe Gly Val Gly
   1 5
<210> 17
   <211> 6
   <212> PRT
   <213> Artificial Sequence
<220>
   <223> Conserved sequence motif
<400> 17
   Asp Phe Leu Gly Gln Gly
   1 5
<210> 18
   <211> 6
   <212> PRT
   <213> Artificial Sequence
<220>
   <223> Conserved sequence motif
<400> 18
   Gly Asn Ser Leu Gly Gly
   1               5
<210> 19
   <211> 14
   <212> PRT
   <213> Artificial Sequence
<220>
   <223> Conserved sequence motif
<400> 19
   Leu Val Lys Gly Val Thr Leu Leu Asn Ala Thr Pro Phe Trp
   1               5                   10
<210> 20
   <211> 4
   <212> PRT
   <213> Artificial Sequence
<220>
   <223> Conserved sequence motif
<400> 20
   His Pro Ala Ala
   1
<210> 21
   <211> 4
   <212> PRT
   <213> Artificial Sequence
<220>
   <223> Conserved sequence motif
<400> 21
   Glu Asp Pro Trp
   1
<210> 22
   <211> 8
   <212> PRT
   <213> Artificial Sequence
<220>
   <223> Conserved sequence motif
<400> 22
   Ser Pro Ala Gly His Cys Pro His
   1 5
<210> 23
   <211> 285
   <212> PRT
   <213> Aeromonas salmonicida
<400> 23
   Ala Asp Thr Arg Pro Ala Phe Ser Arg Ile Val Met Phe Gly Asp Ser
   1               5                   10                  15
   Leu Ser Asp Thr Gly Lys Met Tyr Ser Lys Met Arg Gly Tyr Leu Pro
               20                  25                  30
   Ser Ser Pro Pro Tyr Tyr Glu Gly Arg Phe Ser Asn Gly Pro Val Trp
           35                  40                  45
   Leu Glu Gln Leu Thr Lys Gln Phe Pro Gly Leu Thr Ile Ala Asn Glu
       50                  55                  60
   Ala Glu Gly Gly Ala Thr Ala Val Ala Tyr Asn Lys Ile Ser Trp Asp
   65                  70                  75                  80
   Pro Lys Tyr Gln Val Ile Asn Asn Leu Asp Tyr Glu Val Thr Gln Phe
                   85                  90                  95
   Leu Gln Lys Asp Ser Phe Lys Pro Asp Asp Leu Val Ile Leu Trp Val
               100                 105                 110
   Gly Ala Asn Asp Tyr Leu Ala Tyr Gly Trp Asn Thr Glu Gln Asp Ala
           115                 120                 125
   Lys Arg Val Arg Asp Ala Ile Ser Asp Ala Ala Asn Arg Met Val Leu
       130                 135                 140
   Asn Gly Ala Lys Gln Ile Leu Leu Phe Asn Leu Pro Asp Leu Gly Gln
   145                 150                 155                 160
   Asn Pro Ser Ala Arg Ser Gln Lys Val Val Glu Ala Val Ser His Val
                   165                 170                 175
   Ser Ala Tyr His Asn Lys Leu Leu Leu Asn Leu Ala Arg Gln Leu Ala
               180                 185                 190
   Pro Thr Gly Met Val Lys Leu Phe Glu Ile Asp Lys Gln Phe Ala Glu
           195                 200                 205
   Met Leu Arg Asp Pro Gln Asn Phe Gly Leu Ser Asp Val Glu Asn Pro
       210                 215                 220
   Cys Tyr Asp Gly Gly Tyr Val Trp Lys Pro Phe Arg Ser Ala Ser Pro
   225                 230                 235                 240
   Arg Ser Ala Ser Pro Leu Asn Cys Glu Gly Lys Met Phe Trp Asp Gln
                   245                 250                 255
   Val His Pro Thr Thr Val Val His Ala Ala Leu Ser Glu Arg Ala Ala
               260                 265                 270
   Thr Phe Ile Glu Thr Gln Tyr Glu Phe Leu Ala His Gly
           275                 280                 285
<210> 24
   <211> 4
   <212> PRT
   <213> Artificial Sequence
<220>
   <223> Conserved sequence motif
<220>
   <221> MISC_FEATURE
   <222> (4)..(4)
   <223> Xaa may be Leu, Ala, Val, Ile, Phe, Tyr, His, Gln, Thr, Asn, Met or Ser
<400> 24
   Gly Asp Ser Xaa
   1